# EUROPEAN PATENT APPLICATION

(11) **EP 1 995 311 A1**
(43) Date of publication of application: **26.11.2008**
(21) Application number: 07012992.9
(22) Date of filing: 08.12.2000
(51) Int. Cl.: C12N 9/08

(54) **Rice peroxidases with various characteristics**

(30) Priority: 10.12.1999 JP 35247299
(62) Divisional of application: 00980026.9
(71) Applicant: NATIONAL INSTITUTE OF AGROBIOLOGICAL SCIENCES, Tsukuba-shi, Ibaraki 305-8602 (JP)
(72) Inventor: Ohashi, Yuko, Tsukuba-shi Ibaraki 305-8602 (JP); Mitsuhara, Ichiro, Tsukuba-shi Ibaraki 305-8602 (JP); Saskai, Takuji, Tsukuba-shi Ibaraki 305-8602 (JP); Nagamura, Yoshiaki, Tsukuba-shi Ibaraki 305-8602 (JP); Ito, Hiroyuki, Sapporo-shi Hokkaido 060-8589 (JP); Iwai, Takayoshi, Tsukuba-shi Ibaraki 305-8602 (JP); Hiraga, Susumu, Sapporo-shi Hokkaido 060-8589 (JP)
(74) Representative: Harding, Charles Thomas

(57) **Abstract**

The present invention relates to a plurality of peroxidases. The present invention provides clarification of the expression specificities of various peroxidases, which was conventionally difficult. The present invention further provides a means for modifying plants using the peroxidase expression specificities. The present invention also relates to peroxidases having various expression specificities and promoters derived these peroxidases . The present invention further relates to gene expression analysis using the peroxidase genes of the present invention.

## Description

### TECHNICAL FIELD

The present invention relates to a peroxidase gene of a plant . More particularly, the present invention relates to a novel peroxidase gene derived from rice. The present invention also relates to a gene analyzing method with a microarray using a group of novel peroxidase genes derived from rice, and a system and apparatus for performing the gene analyzing method.

### BACKGROUND ART

Peroxidases (EC.1.11.1.7) (also herein referred to as "POX") are generally enzymes which catalyze oxidation of various substrates by hydrogen peroxide, and which are widely present in from microorganisms to animals and plants. Peroxidases constitute a superfamily consisting of various isozymes and isoforms, and are currently divided into class I, class II and class III, depending on the reaction specificity and structure (Welinder, Reference 1). Class I is also called prokaryote peroxidase, including yeast mitochondria cytochrome c POX, chloroplast ascorbic acid POX, cytosol ascorbic acid POX, gene bacterial POX, and the like. Class II is also called secretory fungus peroxidase, and which representatively include P. chrysosporium manganese-dependent POX (PCM), ligninase , and the like. Class III is also called classical secretory plant peroxidase, and representatively includes horseradish POX and the like. Class III plant POX is universally found in plants, and a plurality of isoforms have been found in the same plant (Reference 1).

It is suggested that class III plant peroxidase (POXs) contribute to various physiological processes in plants(e.g., lignification (Whetten et al. (Reference 2)), suberization (Espelie et al. (Reference 3)), crosslinking of cell wall proteins (Fry et al . (Reference 4)), auxin degradation and oxidization of the plant hormone indoleacetic acid (IAA) (Hinman et al. (Reference 5)), defense against pathogens (Chittoor et al . (Reference 6)), salt tolerance (Amaya et al . (Reference 7)), senescence (Abeles et al. (Reference 8)), the development, differentiation and growth of plants (Horton et al. (Reference 9)), etc.). It is also considered that class III plant peroxidase (POX) plays an important role in the growth and response to disease and wound stresses of plants, and the like. Since a plurality of peroxidases are present in a single plant and the substrate specificity thereof is low, it is also difficult to define the specific physiological functions of individual peroxidases.

As for plant POX genes, for example, at least seven POX genes have been isolated and identified from each of alfalfa, tomato and wheat (Chittoor et al. (1999), Reference 6). Chittoor et al. isolated three cDNAs and a genomic DNA fragment, which are highly homologous, from rice, and indicated that these three POXs had different induction patterns when rice is infected with Xanthomonas oryzae pv. oryzae (Chittoor et al. (1997). Reference 10). Ito et al. indicated that 25 POXs should be present in aerial parts in terms of proteins. Ito et al. purified four out of the 25, and studied the N-terminal amino acid sequences each of the four and the reactivity of each of the four with antibodies. As a result, Ito et al. inferred that the four POXs are two sets of isoforms (Ito et al., Reference 11). Ito et al. isolated cDNAs (prxRPA and prxRPN) encoding two structurally-related POXs . These two genes are not the same but exhibited similar expression patterns (Ito et al., Reference 12). It was indicated that 12 POX isozymes were detected in tobacco by isoelectric focusing followed by activity staining, and these isozymes had different organ specificities and responsiveness to wounding or TMV infection. According to the observation of these tobacco POXs, it is suggested that the expression of individual POX genes are differently regulated (Langrimini et al., Reference 13).

Thus, the number of POX isozymes which have been conventionally studied as to their expression specificities is limited. The expression tendency of POXs, the number of which is expected to be several tens, has not been analyzed in a generalized manner and it was difficult to perform such an analysis based on conventional findings.

It is known that peroxidases (POXs) have a role in removal of active oxygen species such as representatively hydrogen peroxide. In general, the state of plants or the like in which the concentration of active oxygen species (superoxide, hydrogen peroxide, hydroxyl radical, singlet oxygen, and the like) is increased in cells thereof is called oxidative stress. This is caused by a loss of balance between the oxidation state caused by a peroxidization state; ultraviolet light and radiation; abnormal conditions in the electron transfer system of cytochrome; an increase in peroxisome abnormality; non-biological causalities such as high temperature, low temperature, chemical substances, and the like; air pollutants such as ozone, sulfur dioxide, and the like; and the intracellular antioxidant protection mechanism due to the actions of superoxide dismutase (SOD), catalase (CAT), POX, vitamins E, C and A, and the like.

It has been conventionally known that if eukaryotes are exposed to an oxidative stress, the activity of SOD and the like are increased. An example has been observed, in which by inducing SOD in advance, a cell exhibited a slight level of resistance.

Further, in plants, regarding generation of oxidative stress, the following is known: (1) under various conditions for inhibiting photosynthesis (low temperature, herbicide treatment, or the like under light irradiation), active oxygen species due to a photosynthesis reaction pathway and the like are generated to an extraordinary extent; (2) also in the dark, if plants are exposed to low temperature, the concentration of active oxygen species, such as hydrogen peroxide and the like, maybe increased; and (3) active oxygen species are involved in the action mechanism of herbicide (paraquat (brand name) (1,1-dimethyl-4,4-dipyridinium dichloride) and the like) and ultraviolet light. It is believed that (4) drought. stress, heavy metal, or the like cause oxidative stress.

Recently, attention has been given to the influence of environmental stresses, including oxidative stress, on organisms including plants. Modern industrialization has degraded the global environment, causing social problems. Improvement of the resistance of plants to environmental stresses has been researched. Anti-oxidization enzymes, such as POX, are believed to be a factor in resistance to environmental stresses (protection of an organism) which are useful for removal of active oxygen species excessively generated by various environmental stresses. However, the role of POX in resistance to environmental stresses has not been sufficiently clarified due to the diversity and broad substrate specificity of isozymes and the like. Therefore, at the present time, it is difficult to use POX as a material to produce plants resistant to environmental stresses.

It has been suggested that class III POX has a role in action against biological stimuli such as infection by pathogenic bacteria. In Ohashi et al. (Reference 14), the relationship between tobacco POX and generation of local lesion spots by infection of tobacco mosaic virus (TMV) was studied, and it is suggested that POX functions to oxidize polyphenol in the process of generation of lesion spots caused by necrosis due to TMV. Therefore, it is suggested that POX plays a role in conferring to plants a protection function against infection of pathogens.

Therefore, there is a demand for accumulation of detailed findings on the expression characteristics of POX genes in terms of production of useful plants including stress-resistant plants using genetic engineering methods.

Recently, a large-scale program for sequencing of expressed sequence tags (EST) is proceeding for a number of plants including rice. The presence of 42 and 41 different POX genes has been confirmed in rice (Yamamoto et al., (Reference 15)) and Arabidopsis (0stergaard et al., (Reference 16)), respectively. However, these EST sequences only provide information on the partial sequences of the genes. There has been no report on analysis of the functions of these genes.

### (Problems to be Solved by the Invention)

An objective of the present invention is to provide a novel peroxidase gene group, in which the expression characteristics of each gene is clarified, and the members thereof. Another object of the present invention is to provide a plant expression promoter having identified expression specificity. The present invention is useful for clarification of a picture of the whole group of peroxidases having different expression specificities, and production of modified plants having desired traits by utilizing information obtained by such clarification. The present invention is also useful for analysis of gene expression using a DNA microarray and the like.

### DISCLOSURE OF THE INVENTION

### (Summary of the Invention)

The present invention relates to peroxidase genes characterized by at least two expression specificities defined herein, and a set of such genes. Examples of such expression specificities include period specificity, site specificity, responsiveness to stresses, and the like. The present invention further relates to a promoter for peroxidase genes having identified expression specificity.

The present invention is based on analysis of various aspects of data on the expression specificities of 21 representative rice peroxidase genes. The present inventors clarified that individual rice peroxidase genes have separate expression patterns for various parameters (e.g., the periods of growth, tissues, and the like). Further, it was clarified that there are a plurality of rice peroxidase genes having different induced expression pattern under stresses, such as an oxygen stress, infection of pathogens, and the like.

In one aspect, the present invention relates to a set of peroxidase genes useful for evaluation of a characteristic of plants, comprising:
(A1) a subset of root-expression constitutive genes including at least one type of gene selected from the gene group consisting of:
   (1) DNA having a sequence of SEQ ID NO: 1, a homolog thereof, or a fragment thereof;
   (2) DNA having a sequence of SEQ ID NO: 3, a homolog thereof, or a fragment thereof ;
   (3) DNA having a sequence of SEQ ID NO: 5, a homolog thereof, or a fragment thereof ;
   (4) DNA having a sequence of SEQ ID NO: 7, a homolog thereof, or a fragment thereof ;
   (5) DNA having a sequence of SEQ ID NO: 9, a homolog thereof, or a fragment thereof;
   (6) DNA having a sequence of SEQ ID NO: 11, a homolog thereof, or a fragment thereof;
   (7) DNA having a sequence of SEQ ID NO: 13, a homolog thereof, or a fragment thereof;
   (8) DNA having a sequence of SEQ ID NO: 15, a homolog thereof, or a fragment thereof;
   (9) DNA having a sequence of SEQ ID NO: 17, a homolog thereof, or a fragment thereof:
   (10) DNA having a sequence of SEQ ID NO: 19, a homolog thereof, or a fragment thereof ; and
   (11) DNA having a sequence of SEQ ID NO: 21, a homolog thereof, or a fragment thereof:
(A2) a subset of aerial-expression constitutive genes including at least one type of gene selected from the gene group consisting of:
   (12) DNA having a sequence of SEQ ID NO: 23, a homolog thereof, or a fragment thereof; and
   (13) DNA having a sequence of SEQ ID NO: 25, a homolog thereof, or a fragment thereof;
(B1) a subset of root-expression stress-inducible genes including at least one type of gene selected from the gene group consisting of:
   (14) DNA having a sequence of SEQ ID NO: 27, a homolog thereof, or a fragment thereof;
   (15) DNA having a sequence of SEQ ID NO: 29, a homolog thereof, or a fragment thereof;
   (16) DNA having a sequence of SEQ ID NO: 31, a homolog thereof, or a fragment thereof;
   (17) DNA having a sequence of SEQ ID NO: 33, a homolog thereof, or a fragment thereof; and
   (18)- DNA having a sequence of SEQ ID NO: 35, a homolog thereof, or a fragment thereof;
(B2) a subset of aerial-expression stress-inducible genes including at least one type of gene selected from the gene group consisting of:
   (19) DNA having a sequence of SEQ ID NO: 37, a homolog thereof, or a fragment thereof; and
   (20) DNA having a sequence of SEQ ID NO: 39, a homolog thereof, or a fragment thereof;
(C) a gene below:
   (21) DNA having a sequence of SEQ ID NO: 41, a homolog thereof, or a fragment thereof.

In another aspect, the present invention relates to a peroxidase gene, wherein the peroxidase gene is any of:
(a) peroxidase DNA having a sequence of positions 50 to 1021 in SEQ ID NO: 3;
(b) peroxidase DNA having a sequence of positions 108 to 1109 in SEQ ID NO: 5;
(c) peroxidase DNA having a sequence of positions 66 to 1046 in SEQ ID NO: 7:
(d) peroxidase DNA having a sequence of positions 71 to 1078 in SEQ ID NO: 9;
(e) peroxidase DNA having a sequence of positions 134 to 1108 in SEQ ID NO: 11;
(f) peroxidase DNA having a sequence of positions 75 to 1058 in SEQ ID NO: 13;
(g) peroxidase DNA having a sequence of positions 136 to 1147 in SEQ ID NO: 15;
(i) peroxidase DNA having a sequence of positions 29 to 997 in SEQ ID NO: 17;
(j) peroxidase DNA having a sequence of positions 14 to 997 in SEQ ID NO: 19;
(k) peroxidase DNA having a sequence of positions 110 to 1090 in SEQ ID NO: 21;
(l) peroxidase DNA having a sequence of positions 53 to 1033 in SEQ ID NO: 23;
(m) peroxidase DNA having a sequence of positions 20 to 982 in SEQ ID NO: 25;
(n) peroxidase DNA having a sequence of positions 81 to 1025 in SEQ ID NO: 29;
(o) peroxidase DNA having a sequence of positions 44 to 1084 in SEQ ID NO: 31;
(p) peroxidase DNA having a sequence of positions 68 to 1114 in SEQ ID NO: 33;
(q) peroxidase DNA having a sequence of positions 31 to 1101 in SEQ ID NO: 35;
(r) peroxidase DNA having a sequence of positions 34 to 1089 in SEQ ID NO: 37;
(s) peroxidase DNA having a sequence of positions 52 to 1062 in SEQ ID NO: 39: and
(t) a gene having a sequence which hybridizes to any one sequence of (a) to (s) under stringent conditions and encoding a peroxidase having the same expression specificity as that of a peroxidase encoded by said one sequence.

In another aspect, the present invention relates to a peroxidase gene promoter, wherein the promoter is present on an upstream side of a coding region of a peroxidase gene having a sequence selected from the group consisting of SEQ ID NOs: 1, 3, 5, 7, 9, 11, 13, 15, 17, 19, 21, 23, 25, 27, 29, 31, 33, 35, 37and39, oraperoxidase gene which hybridizes to said peroxidase gene under stringent conditions and has the same specific expression activity as that of said peroxidase gene.

In one embodiment, the present invention relates to a peroxidase gene promoter, wherein the promoter is present on an upstream side of a coding region of a peroxidase gene having a sequence selected from the group consisting of SEQ ID NOs: 1, 3, 5, 7, 9, 11, 13, 15, 17, 19, 21, 27, 29, 31, 33 and 35, or a peroxidase gene which hybridizes to said peroxidase gene under stringent conditions and has root-specific expression activity.

In another embodiment, the present invention relates to a peroxidase gene promoter, wherein the promoter is present on an upstream side of a coding region of a peroxidase gene having a sequence selected from the group consisting of SEQ ID NOs : 15 , 17 , 19 and 21, or a peroxidase gene which hybridizes to said peroxidase gene under stringent conditions and has expression activity in root and aerial parts.

In still another embodiment, the present invention relates to a peroxidase gene promoter, wherein the promoter is present on an upstream side of a coding region of a peroxidase gene having a sequence selected from the group consisting of SEQ ID NOs: 23, 25, 37 and 39, or a peroxidase gene which hybridizes to said peroxidase gene under stringent conditions and has aerial-specific expression activity.

In still another embodiment, the present invention relates to a peroxidase gene promoter, wherein the promoter is present on an upstream side of a coding region of a peroxidase gene having a sequence selected from the group consisting of SEQ ID NOs: 1, 3, 5, 7, 9, 13, 15, 17, 21, 23 and 25, or a peroxidase gene which hybridizes to said peroxidase gene under stringent conditions and has constitutive expression activity.

In still another embodiment, the present invention relates to a peroxidase gene promoter, wherein the promoter is present on an upstream side of a coding region of a peroxidase gene having a sequence selected from the group consisting of SEQ ID NOs: 11 and 19, or a peroxidase gene which hybridizes to said peroxidase gene under stringent conditions and has stress reducible expression activity.

In still another embodiment, the present invention relates to a peroxidase gene promoter, wherein the promoter is present on an upstream side of a coding region of a peroxidase gene having a sequence selected from the group consisting of SEQ ID NOs: 27, 29, 31, 33, 35, 37 and 39, or a peroxidase gene which hybridizes to said peroxidase gene under stringent conditions and has stress-inducible expression activity.

In another aspect, the present invention relates to a method for producing an expression cassette, comprising the steps of:
(1) providing:
   (a) a peroxidase gene containing a sequence selected from the group consisting of SEQ ID NO: 1, 3, 5, 7, 9, 11, 13, 15, 17, 19, 21, 23, 25, 27, 29, 31, 33, 35, 37 and 39; and
   (b) a peroxidase gene which hybridizes to said peroxidase gene under stringent conditions and has the same specific expression activity as that of said peroxidase gene;
(2) specifying a region having a promoter activity on an upstream side of a coding region of peroxidase gene (a) or (b); and
(3) operatively linking the specified region having the promoter activity to a heterologous gene.

The peroxidase gene of the present invention may be useful for a method of producing a plant variety having a modified characteristic. For example, such a plant variety production method comprises the steps of:
preparing an expression cassette in which a promoter is operatively linked to at least one gene selected from the gene group consisting of the peroxidase genes of the present invention or homologs thereof;
introducing the expression cassette into a cell of a plant variety; and
regenerating the cell to a plant body.

In this case, the expression amount of the gene in the plant variety may be evaluated to be different from a standard expression amount of the species to which the plant variety belongs, so that the plant variety is selected (note that, in the above-described selecting step, when DNA having a sequence of SEQ ID NO: 1 or a homolog thereof, or DNA having a sequence of SEQ ID NO: 27 or a homolog thereof is selected, at least one of other genes are simultaneously selected).

Here, a "standard expression amount" of a gene refers to an average expression amount under normal growth conditions for the species to which a plant variety to be modified belongs.

The above-described production method may comprise the steps of:
introducing an expression cassette containing a promoter for a peroxidase gene of the present invention into a cell of a plant variety; and
regenerating the cell to a plant.

Examples of modification of a characteristic of a plant variety include modification of resistance to a stress caused by a factor selected from the group consisting of air pollutants, wounds, hydrogen peroxide, UV, pathogens, environmental stresses and ethylene, and further, modification of a growth characteristic or metabolism characteristic of a plant.

In another aspect, the present invention relates to a method for analyzing a characteristic of a plant using a set of peroxidase genes according to the present invention, comprising the steps of:
extracting RNA from a sample;
binding the RNA to a membrane;
labeling the set of peroxidase genes according to the present invention;
incubating the membrane along with the set of the labeled peroxidase genes; and
detecting signals derived from the labeled peroxidase genes.

The present invention also provides a method for analyzing a characteristic of a plant using a gene according to the present invention in a sample. The method comprises the steps of:
extracting RNA from a sample;
binding the RNA to a membrane;
labeling the peroxidase gene of the present invention;
incubating the membrane along with the labeled peroxidase gene; and
detecting signals derived from the labeled peroxidase gene.

In one embodiment, the characteristic is response to rice blast fungus.

In another embodiment, the gene is at least one gene selected from the group consisting of SEQ ID NOs: 29, 31, 33 and 37.

Samples in the present invention may be derived from any plants. In one embodiment, the samples may be derived from plants of the family rice.

In another embodiment, a method for analyzing a characteristic of a plant using a sequence derived from a promoter according to the present invention is provided. The method comprises the steps of:
extracting RNA from a sample;
binding the RNA to a membrane;
labeling an oligonucleotide having the sequence derived from the promoter according to the present invention;
incubating the membrane along with the labeled oligonucleotide; and
detecting a signal derived from the labeled oligonucleotide.

In another aspect, the present invention provides a method for analyzing gene expression using a DNAmicroarray. The method comprises the steps of :
(a) immobilizing a set of peroxidase genes according to the present invention on the DNA microarray;
(b) preparing at least two samples from a plant;
(c) labeling the samples;
(d) mixing and hybridizing the labeled samples to the DNA microarray; and
(e) washing the hybridized DNA micro array and detecting a signal derived from the label.

In another embodiment, the present invention provides a method for analyzing gene expression using a DNA microarray, comprising the steps of:
(a) immobilizing a peroxidase gene of the present invention and/or a probe of the present invention on the DNA microarray;
(b) preparing at least two samples from a plant;
(c) labeling the samples;
(d) mixing and hybridizing the labeled samples to the DNA microarray; and
(e) washing the hybridized DNA microarray and detecting a signal derived from the label.
   In one embodiment, the method of the present invention further comprises the step of:
(f) correcting the detected signal.
   In another embodiment, the method of the present invention further comprises the step of:
(g) analyzing the detected signal or the corrected signal by an analysis software.

In one embodiment, changes in gene expression over time may be monitored in the analysis method of the present invention using a DNA microarray. In another embodiment, a change in gene expression may be compared between plant samples given different stimuli or given no stimuli in the method of the present invention. By such comparison, global changes in gene expression over time may be monitored, or the metabolism and the like of plants may be predicted by the pattern of gene expression due to a certain stimulus.

### BRIEF DESCRIPTION OF THE DRAWINGS

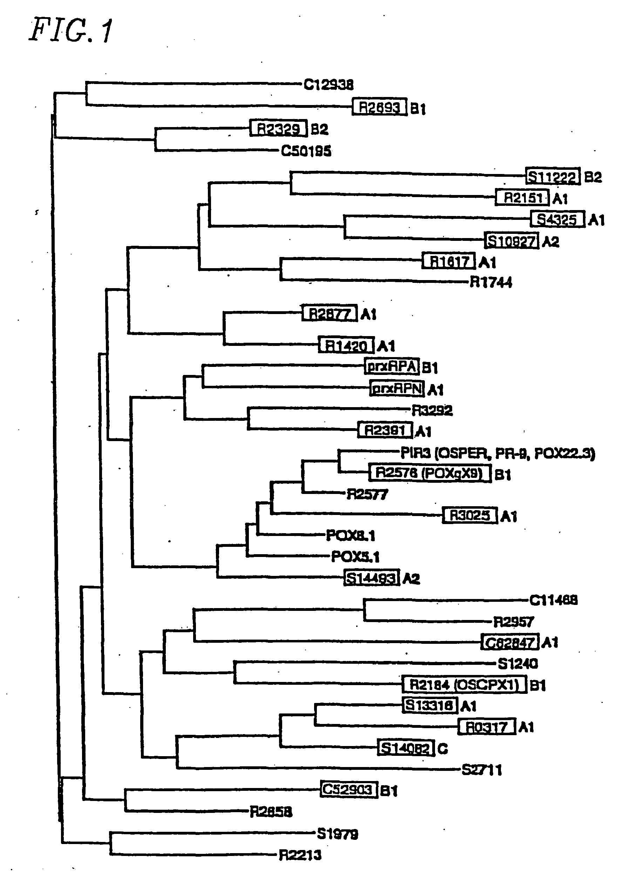

Figure 1 shows the results of phylogenetic analysis in which rice peroxidases are divided into clusters according to their putative amino acid sequences.

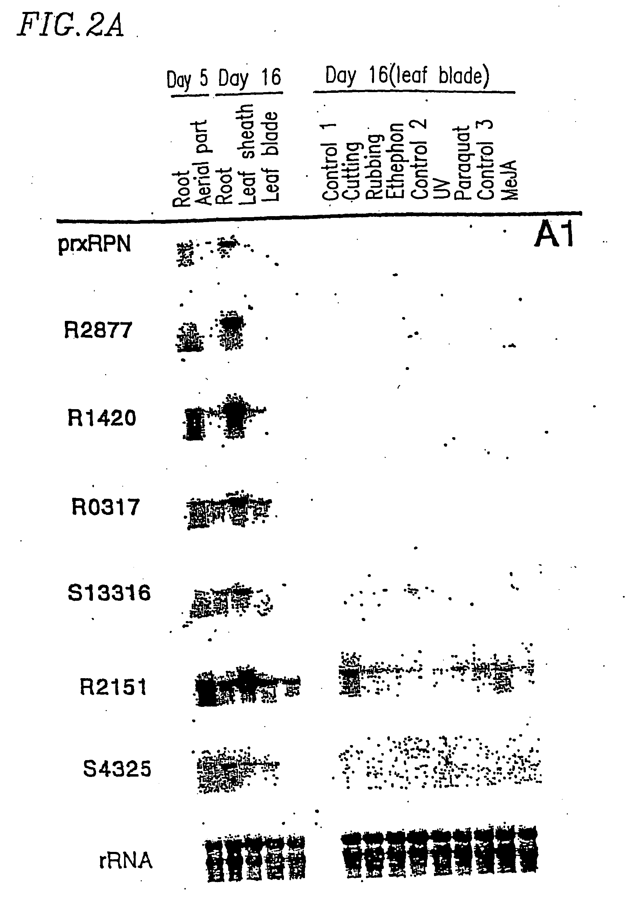

Figure 2A is an electrophoresis photograph showing analysis of gene expression of rice peroxidases in growth stages and sites and due to various stimuli. Among 21 peroxidases used herein, the expression specificities of prxRPN, R2877, R1420, R0317, S13316, R2151 and S4325 are shown.

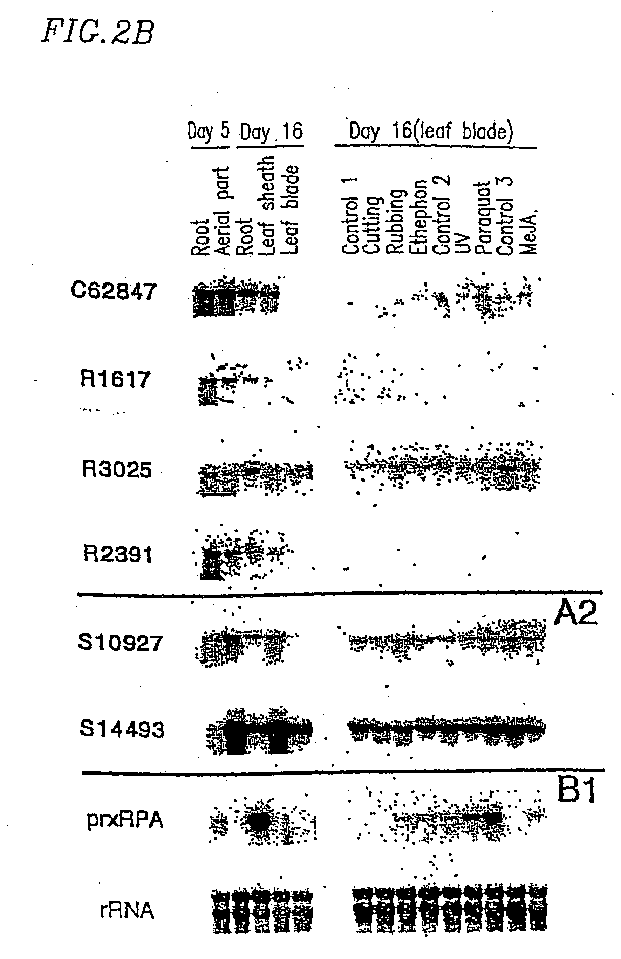

Figure 2B is an electrophoresis photograph showing analysis of gene expression of rice peroxidases in growth stages and sites and due to various stimuli. Among 21 peroxidases used herein, the expression specificities of C62847, R1617, R3025, R2391, S10927, S14493 and prxRPA are shown.

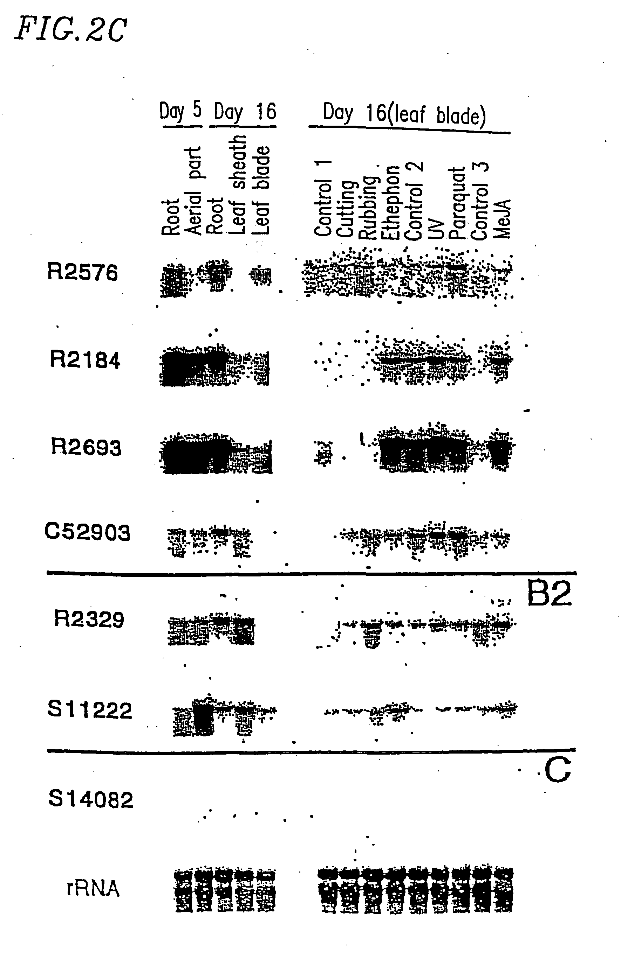

Figure 2C is an electrophoresis photograph showing analysis of gene expression of rice peroxidases in growth stages and sites and due to various stimuli. Among 21 peroxidases used herein, the expression specificities of R2576, R2184, R2693, C52903, R2329, S11222 and S14082 are shown.

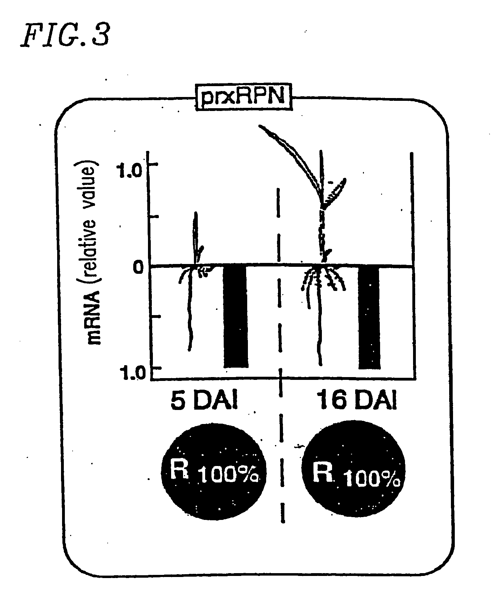

Figure 3 is a diagram showing the results of analysis of the expression specificity of prxRPN peroxidase. Figure 3(a) is a graph showing the relative values of the expression amounts of mRNA in roots and aerial parts on days 5 and 16. Figures 3(b) and (o) shows the expression amount ratio between roots (R) and aerial parts (A) on days 5 and 16, respectively.

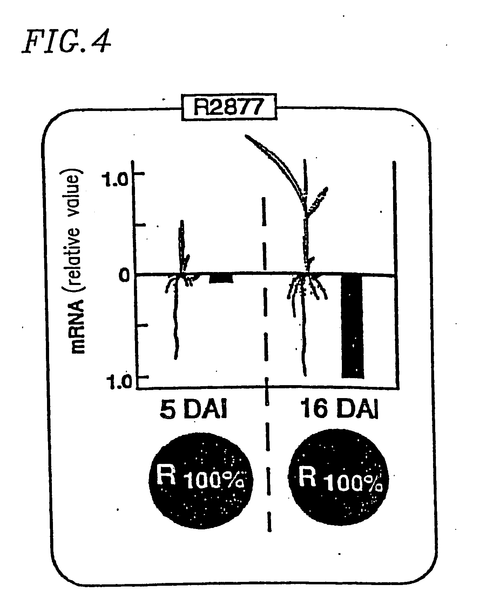

Figure 4 is a diagram showing the results of analysis of the expression specificity of R2877 peroxidase. Figure 4(a) is a graph showing the relative values of the expression amounts of mRNA in roots and aerial parts on days 5 and 16. Figures 4(b) and (c) shows the expression amount ratio between roots (R) and aerial parts (A) on days 5 and 16, respectively.

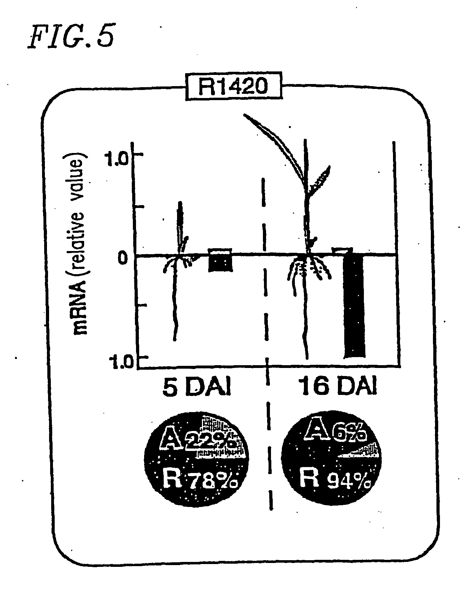

Figure 5 is a diagram showing the results of analysis of the expression specificity of R1420 peroxidase. Figure 5(a) is a graph showing the relative values of the expression amounts of mRNA in roots and aerial parts on days 5 and 16. Figures 5(b) and (o) shows the expression amount ratio between roots (R) and aerial parts (A) on days 5 and 16, respectively.

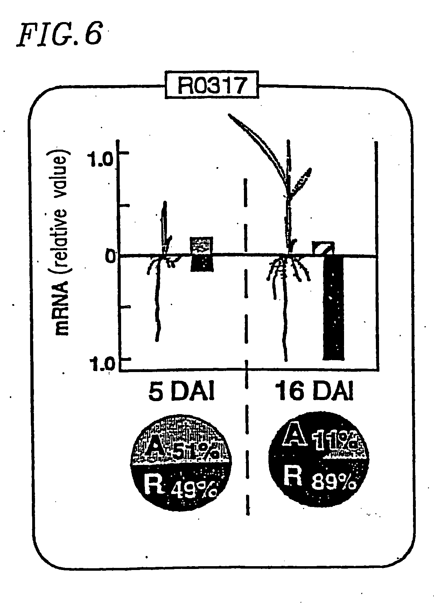

Figure 6 is a diagram showing the results of analysis of the expression specificity of R0317 peroxidase. Figure 6(a) is a graph showing the relative values of the expression amounts of mRNA in roots and aerial parts on days 5 and 16. Figures 6(b) and (c) shows the expression amount ratio between roots (R) and aerial parts (A) on days 5 and 16, respectively.

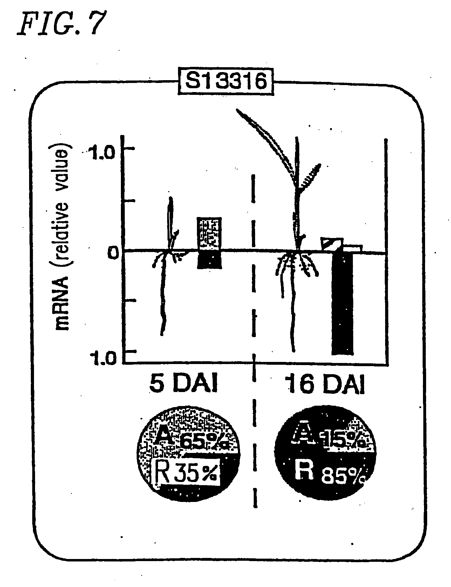

Figure 7 is a diagram showing the results of analysis of the expression specificity of S13316 peroxidase. Figure 7(a) is a graph showing the relative values of the expression amounts of mRNA in roots and aerial parts on days 5 and 16. Figures 7(b) and (c) shows the expression amount ratio between roots (R) and aerial parts (A) on days 5 and 16, respectively.

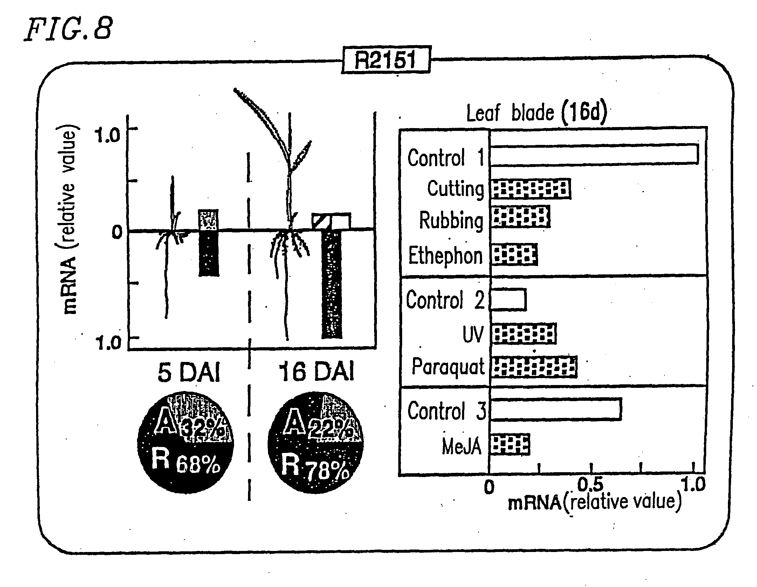

Figure 8 is a diagram showing the results of analysis of the expression specificity of R2151 peroxidase. Figure 8(a) is a graph showing the relative values of the expression amounts of mRNA in roots and aerial parts on days 5 and 16. Figures 8(b) and (c) shows the expression amount ratio between roots (R) and aerial parts (A) on days 5 and 16, respectively. Figure 8(d) is a graph showing comparison of the response levels of gene expression to various stresses on day 16 with those of a control.

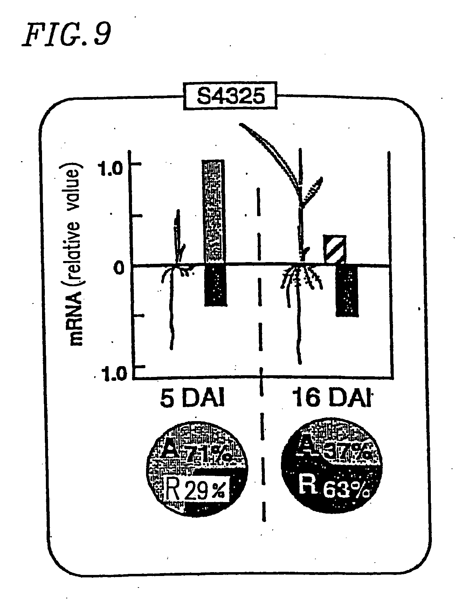

Figure 9 is a diagram showing the results of analysis of the expression specificity of S4325 peroxidase. Figure 9(a) is a graph showing the relative values of the expression amounts of mRNA in roots and aerial parts on days 5 and 16. Figures 9(b) and (c) shows the expression amount ratio between roots (R) and aerial parts (A) on days 5 and 16, respectively.

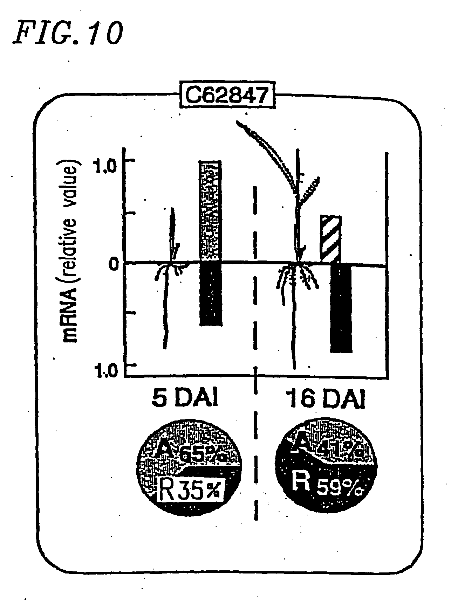

Figure 10 is a diagram showing the results of analysis of the expression specificity of C62847 peroxidase. Figure 10(a) is a graph showing the relative values of the expression amounts of mRNA in roots and aerial parts on days 5 and 16. Figures 10(b) and (a) shows the expression amount ratio between roots (R) and aerial parts (A) on days 5 and 16, respectively.

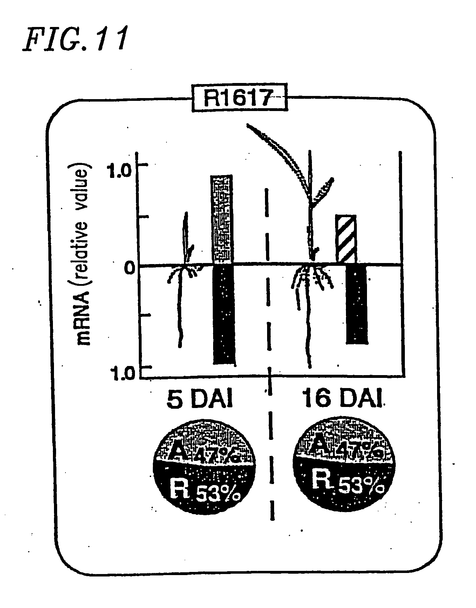

Figure 11 is a diagram showing the results of analysis of the expression specificity of R1617 peroxidase. Figure 11(a) is a graph showing the relative values of the expression amounts of mRNA in roots and aerial parts on days 5 and 16. Figures 11(b) and (c) shows the expression amount ratio between roots (R) and aerial parts (A) on days 5 and 16, respectively.

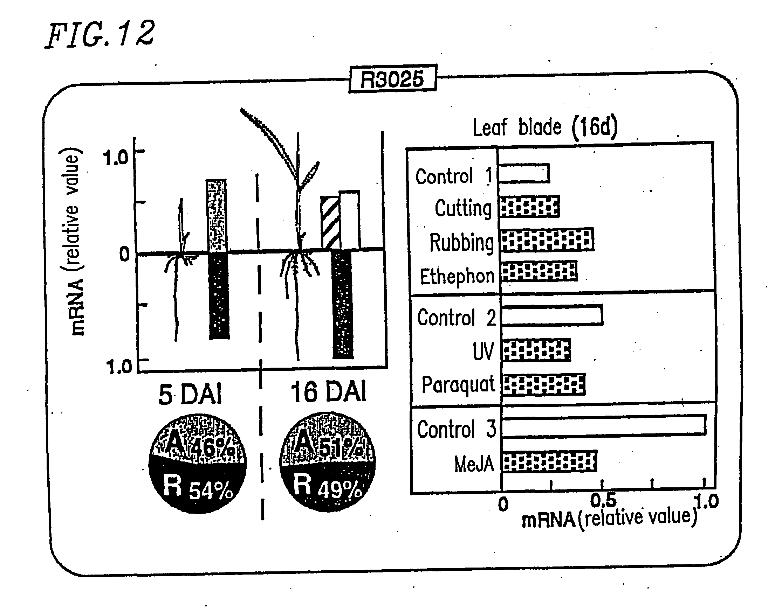

Figure 12 is a diagram showing the results of analysis of the expression specificity of R3025 peroxidase. Figure 12(a) is a graph showing the relative values of the expression amounts of mRNA in roots and aerial parts on days 5 and 16. Figures 12(b) and (c) shows the expression amount ratio between roots (R) and aerial parts (A) on days 5 and 16, respectively. Figure 12(d) is a graph showing comparison of the response levels of gene expression to various stresses on day 16 with those of a control.

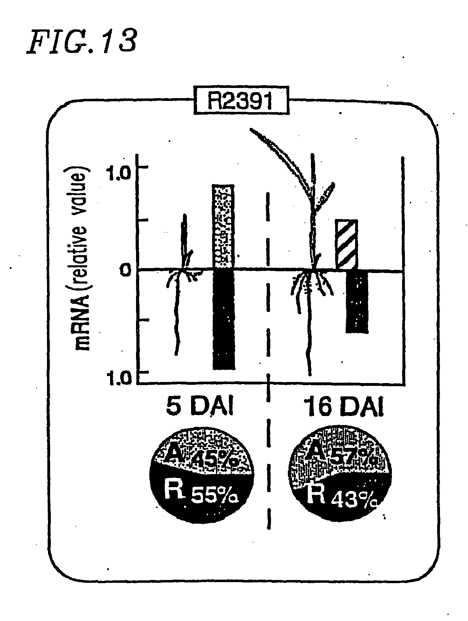

Figure 13 is a diagram showing the results of analysis of the expression specificity of R2391 peroxidase. Figure 13(a) is a graph showing the relative values of the expression amounts of mRNA in roots and aerial parts on days 5 and 16. Figures 13(b) and (c) shows the expression amount ratio between roots (R) and aerial parts (A) on days 5 and 16, respectively.

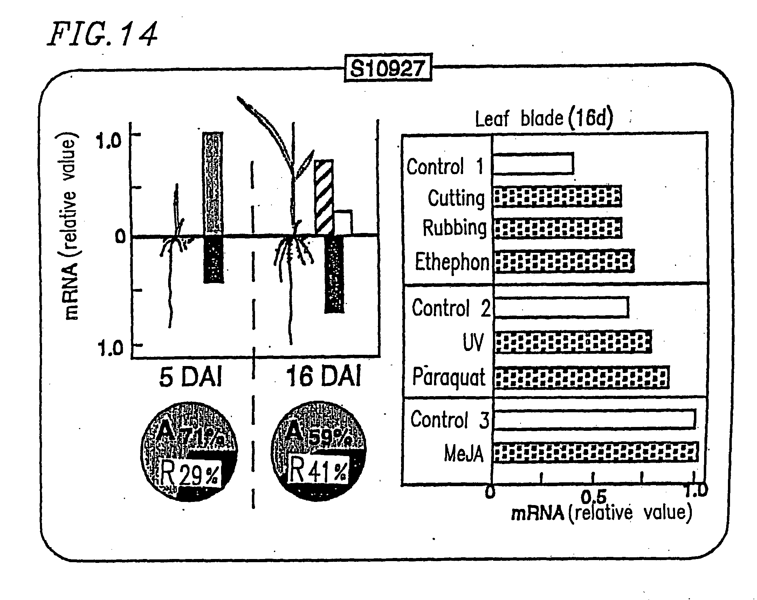

Figure 14 is a diagram showing the results of analysis of the expression specificity of S10927 peroxidase. Figure 14 (a) is a graph showing the relative values of the expression amounts of mRNA in roots and aerial parts on days 5 and 16. Figures 14(b) and (o) shows the expression amount ratio between roots (R) and aerial parts (A) on days 5 and 16, respectively. Figure 14(d) is a graph showing comparison of the response levels of gene expression to various stresses on day 16 with those of a control.

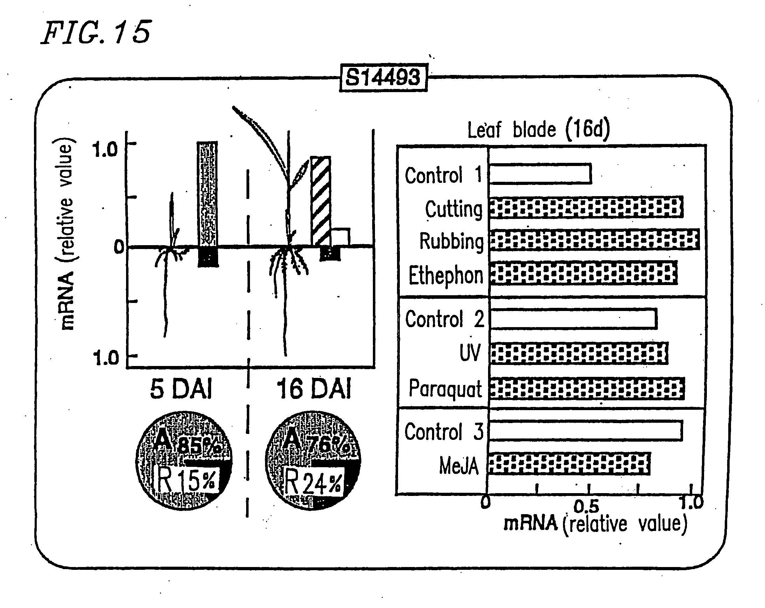

Figure 15 is a diagram showing the results of analysis of the expression specificity of S14493 peroxidase. Figure 15 (a) is a graph showing the relative values of the expression amounts of mRNA in roots and aerial parts on days 5 and 16. Figures 15(b) and (c) shows the expression amount ratio between roots (R) and aerial parts (A) on days 5 and 16, respectively. Figure 15(d) is a graph showing comparison of the response levels of gene expression to various stresses on day 16 with those of a control.

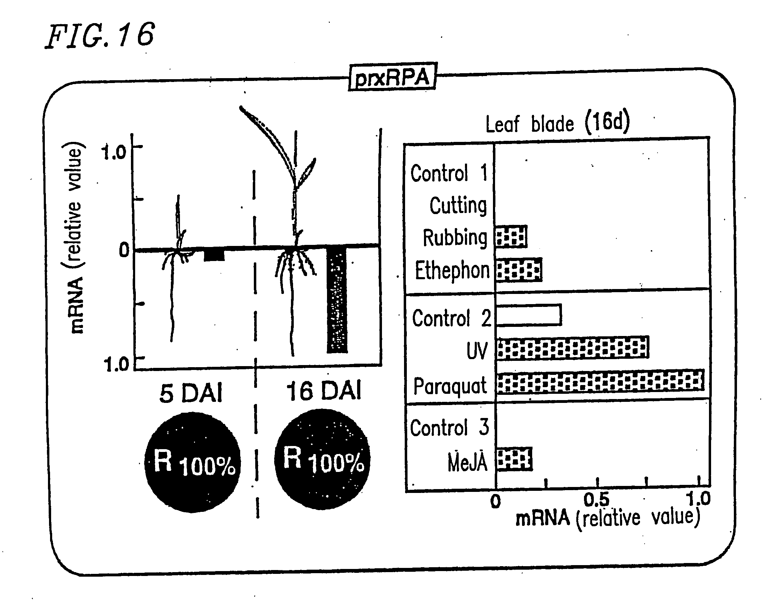

Figure 16 is a diagram showing the results of analysis of the expression specificity of prxRPA peroxidase. Figure 16(a) is a graph showing the relative values of the expression amounts of mRNA in roots and aerial parts on days 5 and 16. Figures 16(b) and (c) shows the expression amount ratio between roots (R) and aerial parts (A) on days 5 and 16, respectively. Figure 16(d) is a graph showing comparison of the response levels of gene expression to various stresses on day 16 with those of a control.

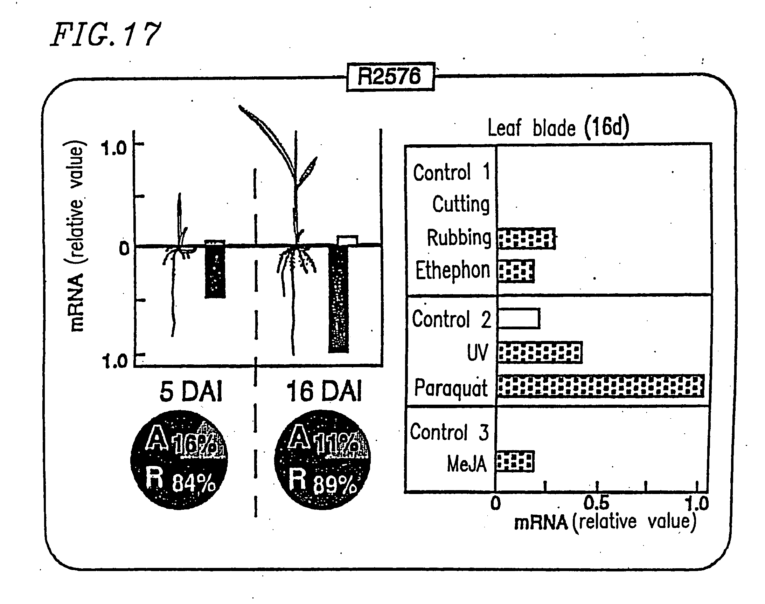

Figure 17 is a diagram showing the results of analysis of the expression specificity of R2576 peroxidase. Figure 17 (a) is a graph showing the relative values of the expression amounts of mRNA in roots and aerial parts on days 5 and 16. Figures 17(b) and (a) shows the expression amount ratio between roots (R) and aerial parts (A) on days 5 and 16, respectively. Figure 17(d) is a graph showing comparison of the response levels of gene expression to various stresses on day 16 with those of a control.

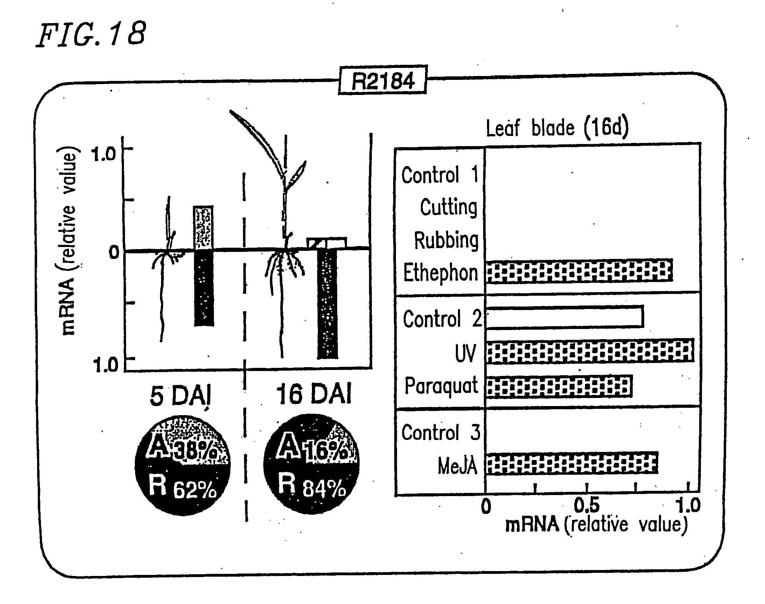

Figure 18 is a diagram showing the results of analysis of the expression specificity of R2184 peroxidase. Figure 18 (a) is a graph showing the relative values of the expression amounts of mRNA in roots and aerial parts on days 5 and 16. Figures 18(b) and (c) shows the expression amount ratio between roots (R) and aerial parts (A) on days 5 and 16, respectively. Figure 18(d) is a graph showing comparison of the response levels of gene expression to various stresses on day 16 with those of a control.

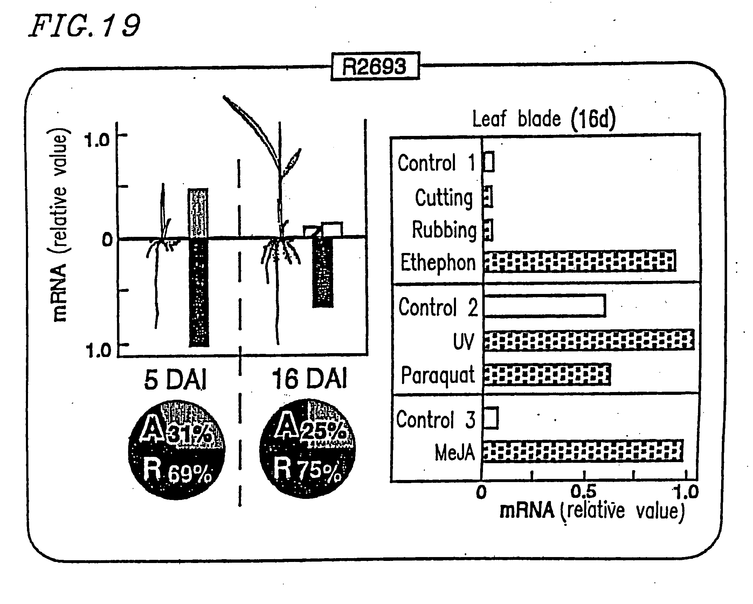

Figure 19 is a diagram showing the results of analysis of the expression specificity of R2693 peroxidase. Figure 19(a) is a graph showing the relative values of the expression amounts of mRNA in roots and aerial parts on days 5 and 16. Figures 19(b) and (c) shows the expression amount ratio between roots (R) and aerial parts (A) on days 5 and 16, respectively. Figure 19(d) is a graph showing comparison of the response levels of gene expression to various stresses on day 16 with those of a control.

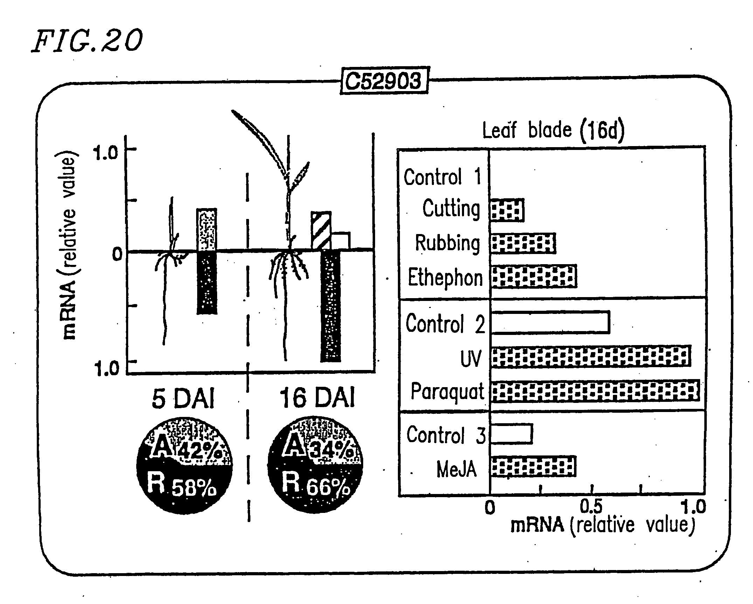

Figure 20 is a diagram showing the results of analysis of the expression specificity of C52903 peroxidase. Figure 20(a) is a graph showing the relative values of the expression amounts of mRNA in roots and aerial parts on days 5 and 16. Figures 20(b) and (c) shows the expression amount ratio between roots (R) and aerial parts (A) on days 5 and 16, respectively. Figure 20(d) is a graph showing comparison of the response levels of gene expression to various stresses on day 16 with those of a control.

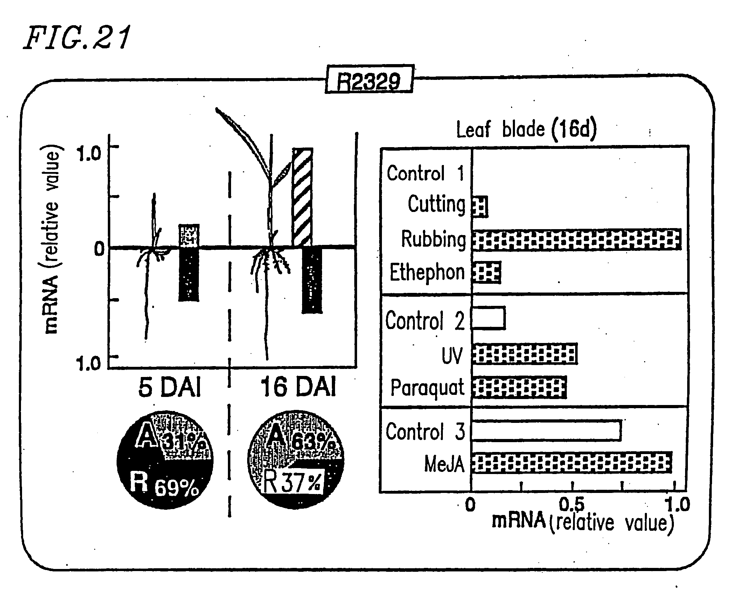

Figure 21 is a diagram showing the results of analysis of the expression specificity of R2329 peroxidase. Figure 21(a) is a graph showing the relative values of the expression amounts of mRNA in roots and aerial parts on days 5 and 16. Figures 21(b) and (c) shows the expression amount ratio between roots (R) and aerial parts (A) on days 5 and 16, respectively. Figure 21(d) is a graph showing comparison of the response levels of gene expression to various stresses on day 16 with those of a control.

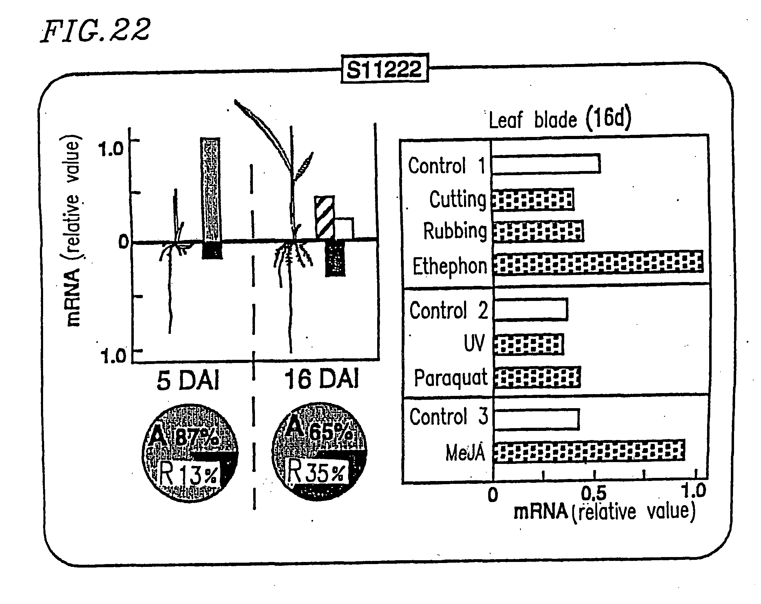

Figure 22 is a diagram showing the results of analysis of the expression specificity of S11222 peroxidase. Figure 22(a) is a graph showing the relative values of the expression amounts of mRNA in roots and aerial parts on days 5 and 16. Figures 22(b) and (o) shows the expression amount ratio between roots (R) and aerial parts (A) on days 5 and 16, respectively. Figure 22(d) is a graph showing comparison of the response levels of gene expression to various stresses on day 16 with those of a control.

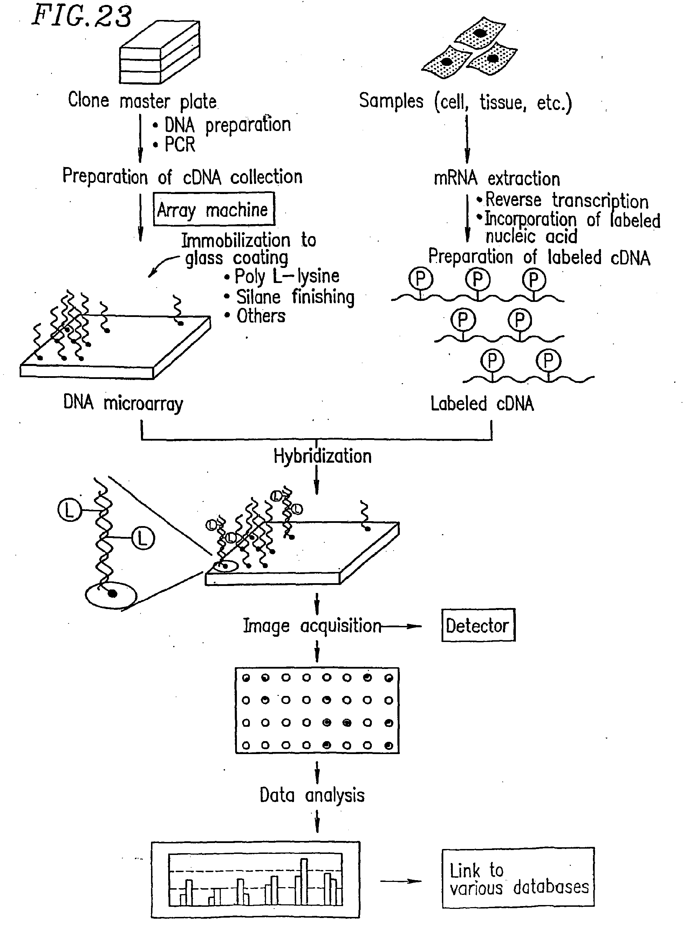

Figure 23 schematically shows a flow of a DNA microarray experiment.

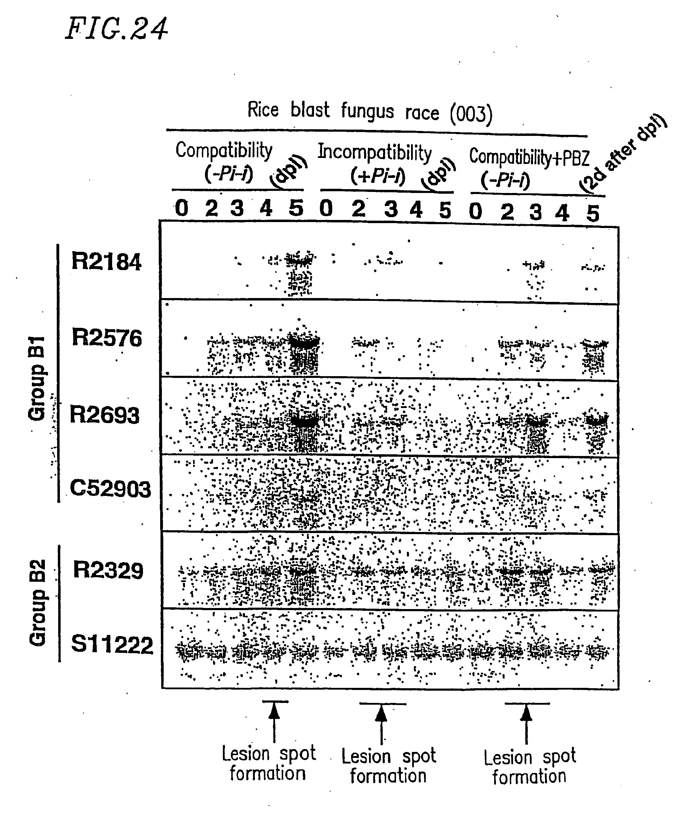

Figure 24 is a diagram showing changes in the expression patterns of the genes (R2184, R2576, R2693, C52903, R2329 and S11222) of the present invention given a stimulus of infection with rice blast fungus race (003) using three rice samples.

### BEST MODE FOR CARRAYING OUT THE INVENTION

Hereinafter, the present invention will be described in detail.

### (Definitions)

A part of the major terms used herein will be defined below.

"Plant" as used herein includes any of the monocotyledons and dicotyledons. Examples of preferable plants include monocotyledons belonging to the family rice, such as wheat, maize, rice, barley, Sorghum, and the like. Other examples of preferable plants include tobacco, pimento, eggplant, melon, tomato, sweet potato, cabbage, onion, broccoli, carrot, cucumber, citrus, Chinese cabbage, lettuce, peach, potato, and apple. Preferable plants are not limited to crops, but include flowers, trees, grasses, weeds, and the like. Plant means any of a plant itself, plant organs, plant tissues, plant cells, and seeds unless otherwise specified. Examples of plant organs include root, leaf, stem, flower, and the like. Examples of plant cells include callus and suspension cultured cells.

"Fragment" of DNA as used herein refers to a polynucleotide having a length which is shorter than the full length of the reference DNA but sufficient for use at least as a probe or a primer. A certain DNA fragment has to be capable of specifically hybridizing in order to be used as a selective probe or a selective primer for DNA from which the fragment originated. "A certain DNA hybridizes specifically to" as used herein indicates that when peroxidases (POXs) are used, at least 21 POX DNAs of the present invention can be separately detected and amplified. The selective probe may have a length of representatively at least 10 nucleotides, preferably at least 15 nucleotides, more preferably at least 20 nucleotides, and even more preferably at least 30, 40 or 50 nucleotides, and may further have a length of more than 50 nucleotides. The selective probe may be available as a product of PCR amplification using a selective primer. When a selective primer is used as at least one of a pair of primers in PCR, the selective primer has a length of representatively at least 9 nucleotides, preferably at least 10 nucleotides, more preferably at least 15 nucleotides, even more preferably at least 17, 18, 19, 20, 21, 22, 23, 24, 25, 30 or 50 nucleotides, or more than 50.

Herein, in order for a fragment of each POX to be specific, the fragment has to be selected from regions excluding a POX conserved region. "POX conserved region" as used herein refers to a region in which a DNA sequence or an amino acid sequence is conserved between the POXs of the present invention. "POX non-conserved region" refers to other than such a region. "Conserved" indicates that the sequence of a certain nucleic acid sequence region is the same as or similar to the original nucleic acid sequence to an extent that the functions of a polypeptide encoded by the sequence are retained. The POX conserved regions are representatively portions of a sequence alignment indicated by boxes in the sequence below. These portions are characterized as regions including particularly two invariable histidine (represented by h) residues and 8 cysteine residues (represented by c1 to c8). The invariable histidine residues in the POX conserved regions correspond to amino acids 67 and 193, respectively, in prxRPA (SEQ ID NO: 28), for example. The invariable cysteine residues in the POX conserved regions correspond to amino acids 38, 71, 76, 115, 121, 200, 230 and 322, respectively, in prxRPA (SEQ ID NO: 28), for example.

Rice RPA
Rice RPN
Tobacco
Horseradish C1a
Turnip TP7
Potato
Arabidopsis Ca
Peanut PNC1
Wheat POX1
Barley BP1

c1
QLSDDFYDYICPDVYTVVQQHVYAA
--T--Y---C--Q--RI-RSR-A--
---AT---TT--N-TSI-RGVMDQR
--TPT---NS--N-SNI-RDTIVNE
--TTN--STT--NLLST-KSG-KS-
--TP EA- -PSA-RGV-DS-
--TPT---TS--T-TNI-RDTIVNE
---SN--ATK--NALSTIKSA-NSC
---ST---TS--RALVAIKSG-A--
AEPPVAPG--F---RRT--RAESI-REF-QE-

I
h c2 c3
MRTEMRMGASLL | RLHFHDCFVNGCDGSILLDG | DDG EKFALP
-KA---------- | --------------A------- | TNS -----A-
Q--DA-A--KII | ---------------------T | -GTQT --D- -
L-SDP-IA--I- | --------------A------N | TT SFRT--D-FG
VSSQP------I- | --F------------------D | TS SFTG-QN-G-
IDA-T------I- | ----------D----G-----D | IN-TFTG-QNSP-
L-SDP-IAG-I- | --------------A------N | TT SFRT--D--G
VAK-A-------- | ----------Q---A-V----D | TS NFTG--T-G-
VSSDP-------- | ---------- ---A-V--T- | M-QN-G-
V-KDIGLA-G-- | ----------Q---A-V----- | SA TGPG-QQ-P-

c4 c5 II
NKN SVRGFEVIDAIKEDLENICPEV | VSCAD IVALAAGYGVLFS
-N- ----Y--------A---GA--G- | ------ ---------K-----L-
ANV GAG--DIV-D--TA---V--G- | ------ -L---SEI--VLA
-A- -A---P---RM-AAV-SA--RT | ------ LLTI--QQS-TLA
-R- -A---T--ND--SAV-KA--G- | ------ -L-I--RDS-VQL
-A- -A--Y---AQA-QSVIDT--NIS | ------ -L-I--RDS-AKL
-A- -A---P---RM-AAV-RA--RT | ------ MLTI--QQS-TLA
-A- -I--------T--SQV-SL--G- | ------ -L-V--RDS-VAL
-VG -L---G---N--TQ--SV-KQT | ------ -LTV--RDS-VAL
-LTLRPSA-KAVND-RDP--RE-RGAV | ---S- -L----RDS-VV-

GGPYYDVLLGRRD GL VANQSGADNGLPSPFEPIKSIIQKFNDVGLD
---D--------- -- -----T--NSN------DS-SV-TAR-K----N
K--SWQ--F--K- S- T--R---NSDI------TLAVM-PQ-TNK-M-
---SWR-P----- S- Q-FLDL-NAN--A--FTLPQLKDS-RN---N
---NWN-KV---- AK T-S-AA-NSNI-A-SMSLSQL-SS-SA---S
--QT-N-A---S- AR T--FT--LTQ--A--DNLTVQ-------KNFT
---SWK-P----- S- Q-FLDL-NAN--A--FTLPQLKAN-KN----
--ASWN------- ST T-SL-S-NSD--A--FNLSGL-SA-SNK-FT
---SWT-P----- ST T-SA-L-NSD--G-SSSRSQLEAA-LKKN-N
---D-R-P----- SRSF-STQDVLSD--G-SSNVQ-LLALLGRL---

III
h c6
TTDV VVLSGGHTIGRARC TLFSNRL STTSSSADPTLDATMA
A--- -----A-----S-- L-------ANF-A-N-V ------SSL-
L--L -A---A--F------ GT-EQ---FNFNG-GNP -L-V---FL
RSS-L -A-------F-KNQ- RFIMD---YNF-N-GLP -----NT-YL
-R-M -A---A-----QS-- VN-RA-VYNETN IN-AF-
LREM -A-A-A--V-F--- ---VCT-GNVNP -
RPS-L -A---A--F-KNQ- RFIMD---YNF-N-GLP -----NT-YL
-KEL -T---A-----Q-Q- -A-RT-I YNESNI-P-Y-
-V-M -A---A-----K-Q- SN-RT-I YGG-TNINTAF-
A--L -TI--------C-H- SS-ED-- FPRP---ISP-FL

c7
ANLQSLC AGGDGNETTVLDITSAYVFDNRYYQNLLNQKGLLSSD
SS--QV- R-- ADQLAA--VN--DA---H-------AN----A--
QT--GI-FQ G-NN--TF-N----STPND---D-FT--QSNQ---QT-
QT-RG--PL N-N LSALVDF-LRTPTI---K--V--EE----IQ--
TLR-RS-PRA--AG-ANLAP---N--TS---S-FK--MA-R---H--
-Q--CN- S -TLTDSDLQQ--T- PTM--KV--D--N-NQ-IMF--
QT-RGQ-PR N-N QSVLVDF-LRTPL-----K--V--KE----IQ--
KS--AN-PS V---T-L SPF-V-TPNK---A--I--R-K-----H--
TS-KAN-PQ S--NT-L AN--TMTPNA---A--T---S-----H--
SR-KRT- PVKGTDRR----VRTPN-----K--ID-V-RE--FV--

QGLFSS DDGI ANTKELVETYSADAHKFPHDFGRS | MVKHGNISPL
---V--SG-PAV-A--A--QA---NGQR-SC---N- | ----------
-E---TSG SA-IAI-NR-AGSQTQ--D--VS- | -I-L------
-E---- PNATD-IP--RSFANSTQT--NA-VEA | -DR----T--
-V-- NGG S-DSI-RG--NSPSS-NS--AAA | -I---D----
-V- TG- -T-AGF-TD--N-VSV-LG--AAA | -I---DLP-S
-E---- PNATD-IP--RA-ADGTQT--NA-VEA | -NR----T-T
-Q-- N-VSTDSQ -TA--NN-AT-NT---NA | -I----L---
-V--NNETTDN TVRN FASN-AA-SSA-TTA | -I-----A--
-D--TN -I-RPI--RFARSQQD--EQ--V- | IG---QMRVR

IV
c8
TGDDGQIRKNCRVVN | (299)
--SA---------A-- | (302)
--TN----TD-KR-- | (302)
--TQ----L------- | SNSLLHDMVEVVDFVSSM (323)
--SS-E---V-GKT- | (296)
A-AQLE--DV-SR-- | PTSVASM (290)
--TQ----L------- | SNSLLHDVVDIVDFVSSM (323)
--TS----T---KT- | (292)
--TQ----LS-SK-- | S (289)
-S-Q-EV-R--S-R- | PGPGADALQWPSLVQTIVDEAAGSIG (331)

"Homolog" of DNA as used herein refers to DNA having a nucleotide sequence which is homologous to the nucleotide sequence of a reference DNA. Representatively, homolog refers to a polynucleotide which hybridizes to a reference DNA under stringent conditions. In the case of peroxidases (POXs), a "homolog" of a POX gene is DNA which has a DNA sequence sharing homology with the DNA sequence of the POX gene, and has the same or similar expression characteristics (e.g., site specificity, period specificity, responsiveness to stresses, and the like).

Herein, a homolog of a certain POX gene generally has homology to the POX non-conserved region of a POX polypeptide to be referenced, but not to the POX non-conserved region of the other POX polypeptide. "Homology" of a gene refers to the magnitude of identity between two or more gene sequences. Therefore, the greater the homology between two genes, the greater the identity or similarity between their sequences. Whether or not two genes have homology is determined by comparing their sequences directly or by a hybridization method under stringent conditions. When two gene sequences are directly compared with each other, the genes have homology if representatively at least 50%, preferably at least 70%, more preferably at least 80%, 90%, 95%, 96%, 97%, 98%, or 99% of the DNA sequence of the genes are identical.

Comparison in identity between base sequences may be calculated using a sequence analysis tool, FASTA (Pearson et al., Reference 17), for example.

"A POX gene has 'the same or similar' expression characteristics" indicates that at least one of the site specificity, period specificity and responsiveness to stresses of expression, preferably any two of the characteristics, more preferably all of the characteristics are the same as or similar to each other. When site specificity is herein mentioned, the proportions of the expression amount of a POX gene in roots and aerial parts are evaluated. POX genes are divided into three groups: expression is predominant in the root; expression is predominant in the aerial parts; and expression is substantially of the same level between the roots and the aerial parts. In this case, when genes are categorized into the same group, the genes are said to be "the same as or similar to" each other. When period specificity is mentioned, the ratio of the expression amounts of a POX gene on day 5 and day 16 of germination is evaluated. POX genes are divided into three groups: expression is predominant on day 5 (juvenile period); expression is predominant on day 16 (mature period); and expression is substantially of the same level in both periods. In this case, when genes are categorized into the same group, the genes are said to be "the same as or similar to" each other. When responsiveness to stresses is mentioned, a change in the expression amount of a POX gene is evaluated when a plant is subjected to any of stresses due to chemicals (e.g., paraquat, ethephon, methyl jasmonate (MeJA), and the like) and physical stimuli (e.g., ultraviolet light (UV), cutting, rubbing, and the like) . POX genes are divided into three groups, depending on responsiveness to a particular stress: the expression amount is increased; the expression amount is decreased; and the expression amount is not changed. In this case, when genes are categorized into the same group, the genes are said to be "the same as or similar to" each other. To investigate each of the above-described expression characteristics, the expression amounts of POX genes may be confirmed by northern blot analysis under conditions similar to those in the examples below.

"Stringent conditions" for hybridization as used herein refer to conditions under which the complementary strand of a nucleotide strand having homology to a target sequence predominantly hybridizes the target sequence, and the complementary strand of a nucleotide strand having no homology substantially does not hybridize. "Complementary strand" of a certain nucleic acid sequence refers to a nucleic acid sequence paired with the certain nucleic acid sequence by hydrogen bonds between nucleic acid bases (.e.g., T for A and C for G). The stringent conditions are sequence-dependent, and vary depending on various circumstances. The higher the sequence, the higher temperature the sequence specifically hybridizes at. In general, as for the stringent conditions, the temperature is selected about 5°C lower than a heat melting point (Tm) of a particular sequence at a predetermined ionic strength and pH. Tm is the temperature at which 50% of nucleotides complementary to a target sequence hybridize to the target sequence in an equilibrium state under a predetermined ionic strength, pH, and nucleic acid concentration. "Stringent conditions" are sequence-dependent and vary depending on various environmental parameters. General guidelines for hybridization of nucleic acids can be found in Tijssen (Reference 18).

Representatively, as to the stringent conditions, the salt concentration is less than about 1.0 M Na⁺, representatively about 0.01 to 1.0 M Na⁺ concentration (or other salts) at pH 7.0 to 8.3, and the temperature is at least about 30°C for short nucleotides (e.g., 10 to 50 nucleotides) and at least about 60°C for long nucleotides (e.g., 50 nucleotides). The stringent conditions may be achieved by addition of a destabilizer, such as formamide. Herein, examples of the stringent conditions include hybridization in buffered solution containing 50% formamide, 1 M NaCl, and 1% SDS (37°C), and washing with 0.1 x SSC at 60°C .

Herein, when used regarding the expression of peroxidase (POX) in plants, "site specificity" generally refers to the expression specificity of a POX gene to a site of a plant (e.g., root, stem, trunk, leaves, flower, seed, germ, embryo, fruit, and the like). "Period specificity" refers to the expression specificity of a POX gene to a growth stage of a plant (e.g., the number of days after germination of a seedling). "Responsiveness to stresses" refers to a change in the expression of a POX gene caused by at least one stress given to a plant.

Here, "stress" may be a factor which is physically, chemically or biologically applied to plants which are in turn inhibited from growing normally. Examples of stresses include physical stresses (light, heat, cooling, freezing, ultraviolet light, X-ray, cutting, rubbing, and the like), chemical stresses (oxygen stress, chemicals, biologically active substances, and the like), biological stresses (viruses, pathogens (e.g., rice blast fungus infection)), and the like. When herein used, "environmental stresses" refer to stresses to plants caused by changes in the global environment. For example, the stresses are caused mainly by an increase in the amount of ultraviolet light due to the destruction of the ozone layer, and active oxygen species and chemicals due to air pollution, or the like.

"Oxygen stress" or "oxidative stress" refers to a stress caused by oxygen and derivatives of oxygen, representatively, active oxygen species (superoxide, hydrogen peroxide, hydroxyl radical, singlet oxygen, and the like), ozone, air pollutants (e.g., SOₓ, NOₓ, and the like), and the like. The oxidative stress is caused by loss of a balance between an "oxidation state" caused by a peroxidization state; ultraviolet light or radiation; abnormal conditions in the electron transfer system of cytochrome; an increase in peroxisome abnormality; non-biological causalities such as high temperature, low temperature, chemical substances, and the like; air pollutants such as ozone, sulfur dioxide, and the like; and the intracellular "antioxidant protection mechanism" due to the actions of superoxide dismutase (SOD), catalase (CAT), POX, vitamins E, C and A, and the like.

"Root expression type" as used herein refers to any of a trait in which a POX gene or a promoter therefor is expressed predominantly in the root of a plant, and a trait in which a POX gene or a promoter therefor is similarly expressed in the roots or aerial parts of a plant. Particularly, the trait in which a POX gene or a promoter therefor is similarly expressed in the roots or aerial parts of a plant is called a "root and aerial part expression type". "Aerial part expression type" refers to a trait in which a POX gene or a promoter therefor is expressed in at least a portion of the aerial parts of a plant more predominantly than the roots. These traits can be determined by extracting RNA from each portion and subjecting the RNA to northern blot analysis to analyze expression amounts.

"Structural" expression of a POX gene or a promoter therefor as used herein refers to a trait in which expression is similarly carried out in a plant tissue during the juvenile period and the mature period in the course of the growth of a plant. Specifically, when northern blot analysis is carried out under conditions similar to those in the examples described herein, if expression is observed in the same or corresponding site of a seedling on both day 5 and day 16, the expression is regarded as being constitutive by the definition in the present invention. Structural peroxidases are believed to play a role in the homeostasis of plants in a normal growth environment. "Responsiveness to stresses" expression of a POX gene or a promoter therefor refers to a trait in which when at least one stress is applied to a plant, the expression amount is changed. Particularly, a trait in which the expression amount is increased is called stress inductivity", and a trait in which the expression amount is decreased is called "stress reducibility" "Stress reducible" expression is based on the assumption that expression can be observed in normal cases, and therefore overlaps the idea of "constitutive" expression. These traits can be determined by extracting RNA from an arbitrary portion and subjecting the RNA to northern blot analysis to analyze expression amounts.

### (Various rice peroxidases)

The present inventors indicated that a number of rice peroxidase genes have different expression specificities. Herein, rice peroxidases may be divided representatively into at least 5 classes, A1, A2, B1, B2 and C, depending on the expression specificity. Categories A and B are based on the responsiveness (inducibility) to stimuli. POXs having no inducibility to stresses are categorized as class A and POXs having inducibility to stresses are categorized as class B. POXs having an expression level which is no more than the limit of detection are categorized as class C.

POXs of the "root expression type" or the "root and aerial part expression type", which are expressed predominantly in belowground parts or similarly in below-and aboveground parts, are categorized as A1 and B1. POXs which are expressed mainly in aerial parts are categorized as A₂ and B2. (e.g., see Figures 2A to 2C and 3 to 22; the summary of the categorization is shown in Table 1.)

The present inventors analyzed expression of the 21 POX genes. As a result, 16 enzymes were categorized as A1 and B1, 4 enzymes were categorized as A2 and B2, and one enzyme was categorized as C. It was clarified that the POX genes of the present invention exhibit various, different and separate responsivenesses to stresses, and are expressed mainly in roots rather than aerial parts, as described below in detail. This was difficult to predict from conventional preliminary findings (Reference 11 and the like).

**Table 1**

| Group | Induction by stimuli^{a} | Spatial distribution^{b,c} | Number^{d} |
|---|---|---|---|
| A1 | no | root≥aerial part | 11 |
| A2 | no | root<aerial part | 2 |
| B1 | yes | root≥aerial part | 5 |
| B2 | yes | root<aerial part | 2 |
| C | no detection | no detection | 1 |

| | | | |
|---|---|---|---|
| a: leaf blade of 16-day-old plants b: 16-day-old plants c: root≥aerial part where the ratio of the root to the aerial part exceeds 4/6 d: the number of POX genes in the group | | | |

Hereinafter, representative rice POX genes within the scope of the present invention will be described.

prxRPN. as well as prxRPA described below are POXs which have been isolated by the present inventors (Ito et al. (1994), Reference 12). These sequences have been isolated from rice based on a sequence conserved in plant peroxidases. The prxRPN gene has a sequence indicated by SEQ ID NO: 1. The genetic products of this gene are categorized as A1 because of the expression specificity thereof. The genetic products are expressed predominantly. in roots. This suggests that the prxRPN gene has a function contributing to characteristics of plants, such as an ability to grow under anaerobic conditions , such as under water and the like. Examples of a primer having a sequence specific to this gene include prxRPNFP1 and prxRPNRP1 (SEQ ID NO: 58 and 59). These primers are useful for obtaining a gene of interest or genes similar thereto. A promoter derived from the prxRPN gene is considered to reflect the expression specificity of prxRPN. Use of the prxRPN gene and homologs thereof and promoters derived therefrom is useful for modification of plant characteristics.

R2877 is a novel POX obtained from rice based on the EST sequences, and belongs to A1 in accordance with the categorization described herein. This gene has a sequence indicated by SEQ ID NO: 3. R2877 may be expressed predominantly in roots. R2877 is expressed in a seedling during the mature period more significantly than during the juvenile period. Dominant expression of genetic products. in roots suggests that R2877 has a function contributing to characteristics of plants, such as an ability to grow under anaerobic conditions, such as under water and the like. The significant expression in a seedling during the mature period suggests that R2877 is required for the metabolism of a plant during the mature period, such as regulation of the amount of indoleacetic acid (IAA) which is a plant hormone, and the like. A promoter derived from the R2877 gene is considered to reflect the expression specificity of R2877. Use of the R2877 gene and homologs thereof and promoters derived therefrom is useful for modification of plant characteristics.

R1420 is also a sequence obtained from rice based on the EST sequences, and belongs to A1 in accordance with the categorization described herein. This gene has a sequence indicated by SEQ ID NO: 5. R1420 may also be expressed predominantly in roots. R1420 is also expressed in a seedling during the mature period more significantly than during the juvenile period. The predominant expression of genetic products in roots suggests that R1420 has a function contributing to characteristics of plants, such as an ability to grow under anaerobic conditions, such as under water and the like. The significant expression in a seedling during the mature period suggests that R1420 is required for the metabolism of a plant during the mature period, such as regulation of the amount of IAA which is a plant hormone, and the like. An exemplary primer having a sequence specific to this gene is R1420FP1 (SEQ ID NO: 48). A promoter derived from the R1420 gene is considered to reflect the expression specificity of R1420. Use of the R1420 gene and homologs thereof and promoters derived therefrom is useful for modification of plant characteristics.

R0317 is also a sequence obtained from rice based on the EST sequences, and belongs to A1 in accordance with the categorization described herein. This gene has a sequence indicated by SEQ ID NO: 7. R0317 may also be expressed predominantly in roots. R0317 is also expressed in a seedling during the mature period more significantly than during the juvenile period. The predominant expression of genetic products in roots suggests that R0317 has a function contributing to characteristics of plants, such as an ability to grow under anaerobic conditions, such as under water and the like. The significant expression in a seedling during the mature period suggests that R0317 is required for the metabolism of a plant during the mature period, such as regulation of the amount of IAA which is a plant hormone, and the like. An exemplary primer having a sequence specific to this gene is R0317F1 (SEQ ID NO: 47). A promoter derived from the R0317 gene is considered to reflect the expression specificity of R0317. Use of the R0317 gene and homologs thereof and promoters derived therefrom is useful for modification of plant characteristics.

S13316 is also a sequence obtained from rice based on the EST sequences, and belongs to A1 in accordance with the categorization described herein. This gene has a sequence indicated by SEQ ID NO:9. S13316 may also be expressed predominantly in roots. S13316 is also expressed in a seedling during the mature period more significantly than during the juvenile period. The predominant expression of genetic products in roots suggests that S13316 has a function contributing to characteristics of plants, such as an ability to grow under anaerobic conditions, such as under water and the like. The significant expression in a seedling during the mature period suggests that S13316 is required for the metabolism of a plant during the mature period, such as regulation of the amount of IAA which is a plant hormone, and the like. An exemplary primer having a sequence specific to this gene is S13316FP1 (SEQ ID NO: 54). A promoter derived from the S13316 gene is considered to reflect the expression specificity of S13316. Use of the S13316 gene and homologs thereof and promoters derived therefrom is useful for modification of plant characteristics.

R2151 is also a sequence obtained from rice based on the EST sequences, and belongs to A1 in accordance with the categorization described herein. This gene has a sequence indicated by SEQ ID NO: 11. R2151 may also be expressed predominantly in roots. R2151 is also expressed in a seedling during the mature period more significantly than during the juvenile period. The significant expression in a seedling during the mature period suggests that R2151 is required for the metabolism of a plant during the mature period, such as regulation of the amount of IAA which is a plant hormone, and the like. This gene may exhibit reducible responses to cutting and rubbing stresses, and stimuli of wound information transfer substances (e.g., MeJA) and stimuli of ethylene release factors (e.g., ethephon). A promoter derived from the R2151 gene is considered to reflect the expression specificity of R2151. Use of the R2151 gene and homologs thereof and promoters derived therefrom is useful for modification of plant characteristics.

S4325 is also a sequence obtained from rice based on the EST sequences, and belongs to A1 in accordance with the categorization described herein. This gene has a sequence indicated by SEQ ID NO: 13. S4325 may also be expressed predominantly in roots. S4325 may be similarly expressed during the juvenile period and the mature period. The similar expression in a seedling during the juvenile period and the mature period suggests that S4325 is involved generally in the growth, elongation, and metabolism of plants. An exemplary primer having a sequence specific to this gene is S4325F1 (SEQ ID NO: 57). A promoter derived from the S4325 gene is considered to reflect the expression specificity of S4325. Use of the S4325 gene and homologs thereof and promoters derived therefrom is useful for modification of plant characteristics.

C62847 is also a sequence obtained from rice based on the EST sequences, and belongs to A1 in accordance with the categorization described herein. This gene has a sequence indicated by SEQ ID NO: 15. C62847 may also be similarly expressed in roots and aerial parts. C62847 may be similarly expressed during the juvenile period and the mature period. The similar expression in a seedling during the juvenile period and the mature period suggests that C62847 is involved generally in the growth, elongation, and metabolism of plants. The similar expression of genetic products in roots and aerial parts suggests that C62847 has a function contributing to characteristics of plants, such as an ability to grow under anaerobic conditions, such as under water and the like, and a function contributing to promotion and maintenance of the growth of aerial parts (e.g., the elongation of a stem and the like). An exemplary primer having a sequence specific to this gene is C62847FP1 (SEQ ID NO: 44). A promoter derived from the C62847 gene is considered to reflect the expression specificity of C62847. Use of the C62847 gene and homologs thereof and promoters derived therefrom is useful for modification of plant characteristics.

R1617 is also a sequence obtained from rice based on the EST sequences, and belongs to A1 in accordance with the categorization described herein. This gene has a sequence indicated by SEQ ID NO: 17. R1617 may also be similarly expressed in roots and aerial parts. R1617 may be similarly expressed during the juvenile period and the mature period. The similar expression in a seedling during the juvenile period and the mature period suggests that R1617 is involved generally in the growth, elongation, and metabolism of plants. The similar expression of genetic products in roots and aerial parts suggests that R1617 has a function contributing to characteristics of plants, such as an ability to grow under anaerobic conditions, such as under water and the like, and a function contributing to promotion and maintenance of the growth of aerial parts (e.g., the elongation of a stem and the like). A promoter derived from the R1617 gene is considered to reflect the expression specificity of R1617. Use of the R1617 gene and homologs thereof and promoters derived therefrom is useful for modification of plant characteristics.

R3025 is also a sequence obtained from rice based on the EST sequences, and belongs to A1 in accordance with the categorization described herein. This gene has a sequence indicated by SEQ ID NO: 19. Analysis of the putative amino acid sequence suggests that the protein product is of an extracellular secretory type. R3025 may also be similarly expressed in roots and aerial parts. R3025 may be similarly expressed during the juvenile period and the mature period. The similar expression in a seedling during the juvenile period and the mature period suggests that R3025 is involved generally in the growth, elongation, and metabolism of plants. The similar expression of genetic products in roots and aerial parts suggests that R3025 has a function contributing to characteristics of plants, such as an ability to grow under anaerobic conditions, such as under water and the like, and a function contributing to promotion and maintenance of the growth of aerial parts (e.g., the elongation of a stem and the like). A promoter derived from the R3025 gene is considered to reflect the expression specificity of R3025. Use of the R3025 gene and homologs thereof and promoters derived therefrom is useful for modification of plant characteristics.

R2391 is also a sequence obtained from rice based on the EST sequences, and belongs to A1 in accordance with the categorization described herein. This gene has a sequence indicated by SEQ ID NO: 21. R2391 may also be similarly expressed in roots and aerial parts. The similar expression of genetic products in roots and aerial parts suggests that R2391 has a function contributing to characteristics of plants, such as an ability to grow under anaerobic conditions, such as under water and the like, and a function contributing to promotion and maintenance of the growth of aerial parts (e.g., the elongation of a stem and the like) . R2391 may be expressed during the juvenile period more significantly than during the mature period. The significant expression in a seedling during the juvenile period suggests that R2391 is involved in the growth and elongation of plants; such as synthesis of cell walls, and the like. An exemplary primer having a sequence specific to this gene is R2391FP2 (SEQ ID NO: 50). A promoter derived from the R2391 gene is considered to reflect the expression specificity of R2391. Use of the R2391 gene and homologs thereof and promoters derived therefrom is useful for modification of plant characteristics.

S10927 is also a sequence obtained from rice based on the EST sequences, and belongs to A2 in accordance with the categorization described herein. This gene has a sequence indicated by SEQ ID NO: 23. S10927 is expressed predominantly in aerial parts. The predominant expression of genetic products in aerial parts suggests that S10927 has a function contributing predominantly to promotion and maintenance of the growth of aerial parts (e.g., elongation of a stem, and the like). S10927 may also be similarly expressed during the juvenile period and the mature period. The similar expression in a seedling during the juvenile period and the mature period suggests that S10927 is required for the growth, elongation and metabolism of plants, such as involvement in synthesis of cell walls and regulation of the amount of a plant hormone IAA, and the like. An exemplary primer having a sequence specific to this gene is S10927FP1 (SEQ ID NO: 52). A promoter derived from the S10927 gene is considered to reflect the expression specificity of S10927. Use of the S10927 gene and homologs thereof and promoters derived therefrom is useful for modification of plant characteristics.

S14493 is also a sequence obtained from rice based on the EST sequences, and belongs to A2 in accordance with the categorization described herein. This gene has a_{.} sequence indicated by SEQ ID NO: 25. S14493 is expressed predominantly in aerial parts. The predominant expression of genetic products in aerial parts suggests that S14493 has a function contributing predominantly to promotion and maintenance of the growth of aerial parts (e.g., elongation of a stem, and the like). S14493 may also be similarly expressed during the juvenile period and the mature period. The similar expression in a seedling during the juvenile period and the mature period suggests that S14493 is involved generally in the growth, elongation and metabolism of plants. An exemplary primer having a sequence specific to this gene is S14493FP1 (SEQ ID NO: 56). A promoter derived from the S14493 gene is considered to reflect the expression specificity of S14493. Use of the S14493 gene and homologs thereof and promoters derived therefrom is useful for modification of plant characteristics.

prxRPA as well as the above-described prxPRN are POXs which have been isolated by the present inventors (Ito et al., (1994), Reference 12). The prxRPA gene has a gene sequence indicated by SEQ ID NO: 27 and is categorized as B1 in accordance with the categorization of the present invention. The genetic products of this gene are induced by various stresses. This suggests that prxRPA is involved in the defense mechanism of plants against stresses. Specifically, prxRPA may be induced by oxygen stresses (e.g., UV and paraquat), stimuli of wound information transfer substances (e.g., MeJA), stimuli of ethylene release factors (e.g., ethephon), and a rubbing stress (see Examples 3 to 5). Thus, it is suggested that this gene plays a role in defense against pathogens and removal of active oxygen species. prxRPA may also be significantly expressed in a seedling during the mature period. The significant expression during the mature period suggests that prxRPA is required for the metabolism of plants during the mature period, such as regulation of the amount of the plant hormone IAA, and the like. Analysis of the putative amino acid sequence suggests that the protein product is of an extracellular secretory type. Examples of a primer having a sequence specific to this gene include prxRPAFP1 (SEQ ID NO: 45) and prxRPARP1 (SEQ ID NO: 46). A promoter derived from the prxRPA gene is considered to reflect the expression specificity of prxRPA. Use of prxRPA gene and homologs thereof and promoters derived therefrom is useful for modification of plant characteristics.

R2576 is a sequence obtained from rice based on the EST sequences, and belongs to B1 in accordance with the categorization described herein. The genetic products of this gene are induced by various stresses. This suggests that R2576 is involved in the defense mechanism of plants against stresses. Specifically, the genetic products of R25 7 6 may be induced by oxygen stresses (e.g., UV and paraquat), stimuli of wound information transfer substances (e.g., MeJA), stimuli of ethylene release factors (e.g., ethephon), and a rubbing stress (see Examples 3 to 5). Thus, it is suggested that this gene plays a role in defense against pathogens and removal of active oxygen species. R2576 may also be significantly expressed in a seedling during the mature period. The significant expression during the mature period suggests that R2576 is required for the metabolism of plants during the mature period, such as regulation of the amount of the plant hormone IAA, and the like. This gene has a gene sequence indicated by SEQ ID NO: 29. Analysis of the putative amino acid sequence suggests that the protein product is of an extracellular secretory type. An example of a primer having a sequence specific to this gene is R2576F1 (SEQ ID NO: 51). A promoter derived from the R2576 gene is considered to reflect the expression specificity of R2576. Use of R2576 gene and homologs thereof and promoters derived therefrom is useful for modification of plant characteristics.

R2184 is also a sequence obtained from rice based on the EST sequences, and belongs to B1 in accordance with the categorization described herein. The genetic products of this gene are induced by various stresses. This suggests that R2184 is involved in the defense mechanism of plants against stresses. Specifically, the genetic products of R2184 may be induced by oxygen stresses (e.g., UV), stimuli of wound information transfer substances (e.g., MeJA), and stimuli of ethylene release factors (e.g., ethephon) (see Examples 4 and 5). Also, such induction may be caused by stresses due to cutting into pieces (see Example 3). Therefore, it is considered that this POX is involved in defense, particularly against significant wounds occurring in plants. Thus, it is suggested that this gene plays a role in defense against pathogens and removal of active oxygen species. R2184 may also be significantly expressed in a seedling during the juvenile period. The significant expression during the juvenile period suggests that R2184 is required for the growth and elongation of plants, such as synthesis of cell walls, and the like. This gene has a gene sequence indicated by SEQ ID NO: 31. Analysis of the putative amino acid sequence suggests that the protein product is of a vacuole localization type. An example of a primer having a sequence specific to this gene is R2184FP1 (SEQ ID NO: 49). A promoter derived from the R2184 gene is considered to reflect the expression specificity of R2184. Use of R2184 gene and homologs thereof and promoters derived therefrom is useful for modification of plant characteristics.

R2693 is also a sequence obtained from rice based on the EST sequences, and belongs to B1 in accordance with the categorization described herein. The genetic products of this gene are induced by various stresses. This suggests that R2693 is involved in the defense mechanism of plants against stresses. Specifically, the genetic products of R2693 may be induced by oxygen stresses (e.g., UV), stimuli of wound information transfer substances (e.g., MeJA), and stimuli of ethylene release factors (e.g., ethephon) (see Examples 4 and 6) Also, such induction may be caused by stresses due to cutting into pieces (see Example 3). Therefore, it is considered that this POX is involved in defense particularly against significant wounds occurring in plants. Thus, it is suggested that this gene plays a role in defense against pathogens and removal of active oxygen species. R2693 may also be significantly expressed in a seedling during the juvenile period. The significant expression during the juvenile period suggests that R2693 is required for the growth and elongation of plants, such as synthesis of cell walls, and the like. This gene has a gene sequence indicated by SEQ ID NO: 33. Analysis of the putative amino acid sequence suggests that the protein product is of an extracellular secretory type. A promoter derived from the R2693 gene is considered to reflect the expression specificity of R2693. Use of R2693 gene and homologs thereof and promoters derived therefrom is useful for modification of plant characteristics.

C52903 is also a sequence obtained from rice based on the EST sequences, and belongs to B1 in accordance with the categorization described herein. The genetic products of this gene are induced by various stresses. This suggests that C52903 is involved in the defense mechanism of plants against stresses. Specifically, the genetic products of C52903 may be induced by oxygen stresses (e.g., UV and paraquat), stimuli of wound information transfer substances (e.g., MeJA), stimuli of ethylene release factors (e.g., ethephon), and rubbing and cutting stresses (see Examples 3 to 5). The inducibility of this POX by a wide range of stresses suggests that this gene plays a role in defense against various stresses. C52903 may also be significantly expressed in a seedling during the mature period. The significant expression during the mature period suggests that C52903 is required for the metabolism of plants during the mature period, such as regulation of the amount of the plant hormone IAA, and the like. This gene has a gene sequence indicated by SEQ ID NO: 35. Analysis of the putative amino acid sequence suggests that the protein product is of a vacuole localization type. An example of a primer having a sequence specific to this gene is C52903FP1 (SEQ ID NO: 43). A promoter derived from the C52903 gene is considered to reflect the expression specificity of C52903. Use of C52903 gene and homologs thereof and promoters derived therefrom is useful for modification of plant characteristics.

R2329 is also a sequence obtained from rice based on the EST sequences, and belongs to B2 in accordance with the categorization described herein. The genetic products of this gene are induced by various stresses. This suggests that R2329 is involved in the defense mechanism of plants against stresses. Specifically, the genetic products of R2329 maybe induced by oxygen stresses (e.g. , UV and paraquat), stimuli of wound information transfer substances (e.g., MeJA), stimuli of ethylene release factors (eg. , ethephon), and rubbing and cutting stresses (see Examples 3 to 5). The inducibility of this POX by a wide range of stresses suggests that this geneplays a role in defense against various stresses. R2329 may also be significantly expressed in a seedling during the mature period. The significant expression during the mature period suggests that R2329 is required for the metabolism of plants during the mature period, such as regulation of the amount of the plant hormone IAA, and the like. This gene has a gene sequence indicated by SEQ ID NO : 37. Analysis of the putative amino acid sequence suggests that the protein product is of an extracellular secretory type. A promoter derived from the R23 29 gene is considered to reflect the expression specificity of R2329. Use of R2329 gene and homologs thereof and promoters derived therefrom is useful for modification of plant characteristics.

S11222 is also a sequence obtained from rice based on the EST sequences, and belongs to B2 in accordance with the categorization described herein. The genetic products of this gene are induced by various stresses. This suggests that S11222 is involved in the defense mechanism of plants against stresses. Specifically, the genetic products of S11222.may be induced by stimuli of wound information transfer substances (e.g., MeJA), and stimuli of ethylene release factors (e.g., ethephon) (see Example 4). Thus, it is suggested that this gene plays a role in defense against pathogens. S11222 may also be significantly expressed in a seedling during the juvenile period. The significant expression during the juvenile period suggests that S11222 is required for the growth and elongation of plants, such as synthesis of cell walls, and the like. This gene has a gene sequence indicated by SEQ ID NO: 39. An example of a primer having a sequence specific to this gene is Sl1222FPl (SEQ ID NO: 53). A promoter derived from the S11222 gene is considered to reflect the expression specificity of S11222. Use of S11222 gene and homologs thereof and promoters derived therefrom is useful for modification of plant characteristics.

S14082 is also a sequence obtained from rice based on the EST sequences. Herein, no expression of this gene was detected in experiments. Therefore, S14082 belongs to C in accordance with the categorization described herein. This gene has a gene sequence indicated by SEQ ID NO: 41. An example of a primer having a sequence specific to this gene is S14082FP1 (SEQ ID NO: 55). A promoter derived from the S14082 gene is considered to reflect the expression specificity of S14082. Use of S14082 gene and homologs thereof and promoters derived therefrom is useful for modification of plant characteristics.

### (Method for obtaining each gene and its homologs and method for confirming the expression specificity)

The peroxidase (POX) genes of the present invention and homologs of the POX genes which hybridize to the POX genes under stringent conditions may be isolated using a degenerate primer pair corresponding to the non-conserved regions of the amino acid sequence encoded by the POX genes. This primer pair may be used and a cDNA or genomic DNA of any subject plant may be used as a template to carry out PCR. Thereafter, the resultant amplified DNA fragments may be used as a probe to screen a cDNA library or genomic library of the same subject plant. Next, positive clones are selected and subjected to sequencing, thereby characterizing the POX genes of the present invention or homologs thereof.

The thus-obtained POX genes of the present invention or homologs thereof may be confirmed to have a desired expression specificity by analyzing the expression characteristics of the original plant using the genes or fragments thereof as a selective probe. Alternatively, such a desired expression specificity may be confirmed by introducing the genes into any plant to produce a transformed plant in accordance with a method disclosed herein. RNA samples may be prepared from an appropriate plant material based on a desired expression characteristic, and subjected to northern blot analysis, thereby making it possible to confirm and compare expression amounts.

### (Specification of promoter)

It is well known that a promoter for each of the above-described genes can be obtained from the upstream sequence of the coding region. Such a promoter is representatively defined as, but is not limited to, a sequence present in the range of about 2 kb upstream of a translation initiation point.

A promoter region may be specif ied in accordance with a well-known method in the art . Briefly, a candidate sequence for a promoter region is operatively linked to a reporter gene (e.g., GUS gene) to construct an expression cassette. The constructed expression cassette is used to transform an appropriate plant cell. The transformed cell is regenerated to a plant. The expression of the reporter gene in the transformed plant is detected by utilizing an appropriate detection system (e.g., dye staining). Based on the results of the detection, the promoter region and its expression characteristics may be confirmed.

### (Method for modifying plants by utilizing expression-specific POX genes)

As described above, the POX genes (structural gene) of the present invention and promoters therefor may be each useful as a material for modifying the characteristics of plants in a desired manner. Characteristics to be modified include, but are not limited to, resistance of plant to stresses, and characteristics relating to the growth or metabolism of plants (e.g., the rate or period of growth).

The POX gene of the present invention may be introduced into plant cells as an expression cassette in which each gene is operatively linked to an appropriate promoter. Further, the promoters of the present invention may be introduced into plant cells as an expression cassette in which each promoter is operatively linked to an appropriate heterologous gene, using a well-known method in the art. "Expression cassette" as used herein refers to a nucleic acid sequence containing DNA encoding a POX of the present invention and a plant gene promoter operatively linked thereto (i.e., the promoter can control the expression of the DNA), and a nucleic acid sequence containing a promoter of the present invention and a heterologous gene operatively linked thereto (i.e., linked in-frame thereto). Use of a naturally-occurring expression cassette containing a peroxidase gene optionally in combination with other regulatory elements falls within the scope of the present invention. A preferable expression cassette may be cut by a particular restriction enzyme and is easy to recover.

"Heterologous gene" which may be linked to the promoters of the present invention refers to any of the POX genes of the present invention other than POX genes from which the promoters are derived, plant endogenous genes other than the POX genes, or genes foreign to plants (e.g., genes derived from animals, insects , bacteria and fungi), provided that an expression cassette containing such a gene is introduced into a plant and the genetic products of the gene can be expressed in the plant.

"Plant gene promoter" which may be linked to the POX genes of the present invention means any promoters which can be expressed in plants. Examples of such a plant gene promoter include, but are not limited to, promoters, such as a tobacco PR1a promoter and the like, of which the expression is induced by a certain stress, a CaMV35S promoter, a promoter (Pnos) for nopaline synthetase, and the like.

The above-described., expression cassette is preferably utilized in the form of a plant expression vector. "Plant expression vector" refers to a nucleic acid sequence in which various regulatory elements as well as a structural gene and a promoter for regulating the expression are operatively linked in a host plant cell. Examples of the regulatory elements include, preferably, terminators, drug-resistant genes, and enhancers. It is well known to those skilled in the art that the types of plant expression vectors and the types of regulatory elements used may vary according to a host cell. The plant expression vectors used in the present invention may further have a T-DNA region. The T-DNA region can, improve the efficiency of gene introduction when plants are transformed by, particularly, Agrobacterium.

"Terminator" is a sequence which is located downstream of a region encoding a protein of a gene and which is involved in the termination of transcription when DNA is transcribed into mRNA, and the addition of a polyA sequence. It is known that a terminator contributes to the stability of mRNA; and has an influence on the amount of gene expression. Examples of such a terminator include, but are not limited to, a CaMV35S terminator, a terminator for the nopaline synthetase gene (Tnos), and a terminator for the tobacco PR1a gene.

"Drug-resistant gene" refers to a gene which confers drug resistance to a host when the genetic product thereof is expressed in the host. The drug-resistant gene is desirably one that facilitates the selection of transformed plants. The neomycin phosphotransferase II (NPTII) gene for conferring kanamycin resistance, the hygromycin phosphotransferase gene for conferring hygromycin resistance, and the like may be preferably used.

"Enhancer" maybe used so as to enhance the expression efficiency of a gene of interest. As such an enhancer, an enhancer region containing an upstream sequence within the CaMV35S promoter is preferable. A plurality of enhancers may be used.

As a vector for use in construction of a plant expression vector, pBI vectors, pUC vectors, or pTRA vectors are preferably used. The pBI and pTRA vectors may be used to introduce a gene of interest into plants via Agrobacterium. pBI binary vectors or intermediate vectors may be preferably used. Examples of such vectors include pBI121, pHI101, pBI101.2, pHI101.3, and the like. These vectors contain a gene of a region (T-region) to be introduced into a plant, and the NPT2 gene (conferring kanamycin resistance) as a marker gene which is expressed under the control of a plant promoter. With pUC vectors, a gene may be introduced directly into plants. Examples of pUC vectors include pUC18, pUC19, pUC9, and the like. Plant expression vectors may be produced using gene recombinant techniques well known to those skilled in the art.

For the purpose of introduction of a plant expression vector into a plant cell, a method well known to those skilled in the art, such as an indirect method using Agrobacterium, and a method for directly introducing into cells, can be used. As such an indirect method using Agrobacterium, for example, a method of Nagel et al. (Reference 19) may be used. In this method, initially Agrobacterium is transformed with a plant expression vector by electroporation, and then the transformed Agrobacterium is introduced into a plant cell with a method described in Gelvin et al. (Reference 20). As a method for directly introducing a plant expression vector into a cell, an electroporation method (see Shimamoto et al. ; Reference 21; and Rhodes et al., Reference 22), a particle gun method (see Reference 23), and a polyethylene glycol (PEG) method (see Reference 24) are illustrated. These methods are well known in the art. A method suitable for a plant to be transformed can be appropriately selected by those skilled in the art.

A cell into which a plant expression vector has been introduced is first selected according to drug resistance, such as kanamycin resistance, and the like. Thereafter, the cell may be regenerated to a plant tissue, a plant organ, and/or a plant using a well-known method in the art. Further, seeds may be obtained from the plant. The expression of introduced genes may be detected by a northern method or a PCR method. The expression of proteins which are genetic products may be confirmed by, for example, a western blot method.

The POX genes and promoters of the present invention may be utilized for modification of not only monocotyledons but also dicotyledons, this is because both have a similar genomic structure (Moore et al., Reference 25, and Nagamura et al., Reference 26). Particularly preferable examples of subject plants include wheat, maize, rice, barley, Sorghum, citrus, Chinese cabbage, lettuce, tobacco, peach, potato, tomato, apple, and the like. It has been demonstrated that the POX genes are capable of being introduced into plants, such as, Arabidopsis (Reference 27), Japanese aspen (Reference 28), sweet potato (Reference 29), rice (Reference 30), and the like.

A regenerated plant from a transformed cell may be confirmed to have a desired modified characteristic by carrying out an assay appropriate to the type of characteristic. For example, when it is intended to confer resistance to a pathogenic bacterium as stress resistance, a model bacterium (e.g., Pseudomonas syringae pv. tabaci) is inoculated into a regenerated plant which is in turn compared with a control plant so as to observe the presence or absence of a change due to the inoculation. As a result, a change in the characteristic can be evaluated.

Alternatively, the stress resistance of transformed plants may be evaluated as resistance to an UV treatment, resistance to a treatment with a superoxide generation type herbicide (e.g., paraquat), and/or resistance to salt stress, and the like.

### (Gene expression assay)

The present invention also provides the peroxidase genes of the present invention or a set thereof, or a method for analyzing the characteristics of plants using an oligonucleotide containing a sequence derived from a promoter. Examples of such a method include northern blot analysis and the like. Such a method is reviewed in, for example, Sambrook J. et al. (Reference 60), Reference 36, and the like.

### (DNA array)

The nucleotides of the present invention may be used in a gene analysis method using a DNA array. A DNA array is widely reviewed (Shujunsha Ed., Saibo-kogaku (Cellular Engineering), Special issue, "DNA-maikuro-arei-to-saisin-PCR-ho [DNA microarray and Up-to-date PCR Method"). Further, plant analysis using a DNA array has been recently used (Reference 58). Hereinafter, a DNA array and a gene analysis method using the same will be briefly described.

"DNA array" refers to a device in which DNAs are arrayed and immobilized on a plate. DNA arrays are divided into DNA macroarrays, DNA microarrays, and the like according to the size of a plate or the density of DNA placed on the plate.

The border between macro and micro is not strictly determined. However, generally, "DNA macroarray" refers to a high density filter in which DNA is spotted on a membrane, while "DNA microarray" refers to a plate of glass, silicon, and the like which carries DNA on a surface thereof . There are a cDNA array, an oligoDNA array, and the like according to the type of DNA placed.

A certain high density oligoDNA array, in which a photolithography technique for production of semiconductor integrated circuits is applied and a plurality of oligoDNAs are simultaneously synthesized on a plate, is particularly called "DNA chip" , an adaptation of the term "semiconductor chip". Examples of the DNA chip prepared by this method include GeneChip® (Affymetrix, CA), and the like (References 50 and 51). Preferably, GeneChip® may be used in gene analysis using a microarray according to the present invention. The DNA chip is defined as described above in narrow sense, but may refer to all types of DNA arrays or DNA microarrays.

Thus, DNA microarrays are a device in which several thousands to several ten thousands or more of gene DNAs are arrayed on a glass plate in high density. Therefore, it is made possible to analyze gene expression profiles or gene polymorphism at a genomic scale by hybridization of cDNA, cRNA or genomic DNA. With this technique, it has been made possible to analyze a signal transfer system and/or a transcription control pathway (Reference 45) ; the mechanism of tissue repair (Reference 46); the action mechanism of medicaments (Reference 47): fluctuations in gene expression during development and differentiation processes in a wide scale, and the like; identify a gene group whose expression is fluctuated according to pathologic conditions; find a novel gene involved in a signal transfer system or a transcription control; and the like. Further, as to gene polymorphism, it has been made possible to analyze a number of SNP with a single DNA microarray (Reference 48).

### (Principle of assay using DNA microarray)

The principle of assay using a DNA microarray will be described. DNA microarrays are prepared by immobilizing a number of different DNA probes in high density on a solid-phase plate, such as a slide glass, whose surface is appropriately processed. Thereafter, labeled nucleic acids (targets) are subjected to hybridization under appropriate hybridization conditions, and a signal from each probe is detected by an automated detector. The resultant data is subjected to massive analysis by a computer. For example, in the case of gene monitoring, target cDNAs integrated with fluorescent labels by reverse transcription from mRNA are allowed to hybridize to oligoDNAs or cDNAs as a probe on a microarray, and are detected with a fluorescence image analyzer. In this case, T7 polymerase may be used to carry out other various signal amplification reactions, such as cRNA synthesis reactions or via enzymatic reactions. A flow of a DNA microarray experiment is shown in Figure 23.

### (Synthesized DNA chip)

Fodor et al. has developed a technique for synthesizing polymers on a plate using a combination of combinatorial chemistry and photolithography for semiconductor production (Reference 53). This is called the synthesized DNA chip. Photolithography allows extremely minute surface processing, thereby making it possible to produce a DNA microarray having a packing density of as high as 10 µm²/DNA sample. In this method, generally, about 25 to about 30 DNAs are synthesized on a glass plate.

Gene expression using a synthesized DNA chip was reported by Lockart et al. (Reference 54). This method overcomes a drawback of the chip of this type in that the specificity is low since the length of synthesized DNA is short. This problem was solved by preparing perfect match (PM) oligonucleotide probes corresponding to from about 10 to about 20 regions and mismatch (MM) oligonucleotide probes having one base mutagenesis in the middle of the PM probes for the purpose of monitoring the expression of one gene. Here, the MM probes are used as an indicator f or the specificity of hybridization. Based on the signal ratio between the PM probe and the MM probe, the level of gene expression may be determined. When the signal ratio between the PM probe and the MM probe is substantially 1:1, the result is called cross hybridization, which is not interpreted as a significant signal.

### (Attached DNA microarray)

A so-called attached DNA microarray is prepared by attaching DNAs onto a slide glass, and fluorescence is detected (54) (see also http://cmgm.stanford.edu/pbrown). In this method, no gigantic semiconductor production machine is required, and only a DNA array machine and a detector can be used to perform the assay in a laboratory. This method has the advantage that it is possible to select DNAs to be attached. A high density array can be obtained by spotting spots having a diameter of 100 µm at intervals of 100 µm*,* for example. It is mathematically possible to spot 2500 DNAs per cm². Therefore, a usual slide glass (the effective area is about 4 cm²) can carry about 10,000 DNAs.

As a labeling method for synthesized DNA arrays, for example, double fluorescence labeling is used. In this method, two different mRNA samples are labeled by respective different fluorescent dyes. The two samples are subjected to competitive hybridization on the same microarray, and both fluorescences are measured. By comparing the fluorescences, a difference in gene expression is detected. Examples of the fluorescent dye include, but are not limited to, Cy5 and Cy3, which are most often used, and the like. The advantage of Cy3 and Cy5 is that the wavelengths of fluorescences do not overlap substantially. Double fluorescence labeling may be used to detect mutations or morphorisms in addition to differences in gene expression.

An array machine may be used for assay using a DNA array. In the array machine, basically, a pin tip or a slide holder is moved in directions along X, Y and Z axes in combination with a high-performance servo motor under the control of a computer so that DNA samples are transferred from a microtiter plate to the surface of a slide glass. The pin tip is processed into various shapes. For example, a DNA solution is retained in a cloven pen tip like a crow's bill and spotted onto a plurality of slide glasses. After washing and drying cycles, a DNA sample is then placed on the slide glasses. The above-described steps are repeated. In this case, in order to prevent contamination of the pin tip by a different sample, the pin tip has to be perfectly washed and dried. Examples of such an array machine include SPBIO2000 (Hitachi Software Engineering Co., Ltd.; single strike type), GMS417 Arrayer (Takara Shuzo Co., Ltd. ; pin ring type), Gene Tip Stamping (Nippon Laser&Electronics Lab.; fountain pen type), and the like.

There are various DNA immobilizing methods for use in assays using a DNA array. Glass as a material for a plate has a small effective area for immobilization and electrical charge amount as compared to membranes, and therefore is given various coatings. In practice, Poly L-lysine coating (Reference 55), silane finishing (Reference 56), or the like. Further, a commercially available precoated slide glass exclusive to DNA microarrays (e.g., polycarboimide glass (Nissin Spinning Co., Ltd.) and the like) may also be used. In the case of oligoDNA, a method of aminating a terminal of the DNA and crosslinking the DNA to silane-finished glass is available.

### (Method for preparing cDNA collection)

DNA microarrays may carry mainly cDNA fragments amplified by PCR. When the concentration of cDNA is insufficient, signals cannot be sufficiently detected in some cases. In such a case when a sufficient amount of cDNA fragments is not obtained by one PCR operation, PCR is repeated some times. The resultant overall PCR products may be purified and condensed at one time. A probe cDNA may generally carry a number of random cDNAs, but may carry a group of selected genes (e.g., the gene or promoter groups of the present invention) or candidate genes for gene expression changes obtained by RDA (representational differential analysis) according to the purpose of an experiment. It is preferable to avoid overlapping clones. Clones may be prepared from a stock cDNA library, or cDNA clones may be purchased.

In assays using a DNA array, a fluorescent signal indicating hybridization on the DNA microarray is detected by a fluorescence detector or the like. As such a detector, there are conventionally various available detectors. For example, a research group at the Stanford University has developed an original scanner which is a combination of a fluorescence microscope and a movable stage (see http://cmgm.stanford.edu/pbrown). A conventional fluorescence image analyzer for gel, such as FMBIO (Hitachi Software Engineering), Storm (Molecular Dynamics), and the like, can read a DNA microarray if the spots are not arrayed in very high density. Examples of other available detectors include ScanArray 4000 and 5000 (GeneralScanning; scan type (confocal type)), GMS418 Array Scanner (Takara Shuzo; scan type (confocal type)), Gene Tip Scanner (Nippon Laser & Electronics Lab.; scan type (non-confocal type)), Gene Tac 2000 (Genomic Solutions; CCD camera type)), and the like.

### (Data analysis software)

The amount of data obtained from DNA microarrays is huge. Software for managing correspondences between clones and spots, analyzing data, and the like is important. As such software, software attached to each detection system is available (57). Further, an example of a database format is GATC (genetic analysis technology consortium) proposed by Affymetrix.

### (Differential display technique)

The present invention may also be used in gene analysis using a differential display technique.

The differential display technique is a method for detecting or identifying a gene whose expression fluctuates . In this method, cDNA is prepared from each of at least two samples, and amplified by PCR using a set of any primers. Thereafter, a plurality of generated PCR products are separated by gel electrophoresis. After the electrophoresis pattern is produced, expression fluctuating genes are cloned based on a relative signal strength change between each band.

The genes, gene groups, methods for using the same, and methods for analyzing the same using DNA arrays of the present invention are described above in detail. Thereinafter, examples of the present invention will be described.

### Examples

Examples described below illustrate the present invention, but not limit the present invention in any manner. Therefore, those skilled in the art may modify the present invention based on the matters described in Examples without departing from the scope of the claims.

It should be noted that reagents used in the following examples were obtained from Wako Pure Chemical Industries, Ltd. unless otherwise specified.

### (Example 1) Selection of POX genes, Sequencing and phylogenetic analysis

Materials shown in Table 2 below were each used to extract mRNA by a commonly used method. cDNA was synthesized from the mRNA using reverse transcriptase. The cDNA was inserted into a plasmid vector pBluescript SK (+) (Stratagene) in a predetermined direction. This plasmid vector was used to transform a host E. coli strain NM522. The resultant transformed clones were picked up at random, and stored at -80°C. In order to estimate the genetic products of the obtained cDNA clones, a partial base sequence of each clone was determined from the 5 ' end using ABI 373A DNA sequencing machine (PE Hiosystems). The determined sequences had an average length of about 300 bp. From the resultant sequences, amino acid sequences were estimated using three reading frames. The resultant amino acid sequences were subjected to a similarity search in the NBRF-PIR database using the FASTA algorithm. As a result of the search, when the similarity score relative to a peroxidase protein, which indicated the highest similarity, was greater than or equal to 200, the clone having the score was regarded as having an amino acid sequence derived from rice which has a significant level of homology to peroxidase.

**Table 2**

| Number | library Name | Organ | Vector |
|---|---|---|---|
| C1 | BA Callus | Callus from scutellum (BA treatment) | pBluescriptSK(+) |
| C2 | C(-) Callus | Callus from scutellum(C deletion) | pBluescriptsk(-)(λUni-ZAP XR) |
| C3 | NAA Callus | Callus from scutellum(NAA and BA treatments) | pBluescriptSK(-)(λUni-ZAP XR) |
| C4 | Root Callus | Callus from root tip | pBluescriptSK(-)(λUni-ZAP XR) |
| C5 | GA3 Callus | treatment Callus from scutellum (GA treatment) | pBluescriptSK(+) |
| C6 | Heat shock Callus | Callus from scutellum(heat shock treatment) | pBluescriptSK(+) |
| C | Growth Phase | Callus from scutellum (growth phase) | pBluescriptSK(+) |
| (V) | Developing Seed | Developing seed (5DAF to 15DAF) | pBluescriptSK(+) |
| E | Flowering Panicle | Flowering panicle | pBluescriptSX(+) |
| E1 | Ripening Panicle | Ripening panicle | pBluescriptSK(+) |
| E2 | Young panicle3 | Panicle (longer than 10 cm) | pBluescriptSK(+) |
| E3 | Young panicle1 | Panicle (less than 3 cm) | pBluescriptSK(+) |
| E4 | Young panicle2 | Panicle (3-10 cm) | pBluescriptSX(+) |
| E5 | Apical meristem(long) | Immature leaf containing apical meristem(long day condition) | pBluescriptSK (+) |
| E6 | Apical meristem(short) | Immature leaf containing apical meristem(short day condition) | pBluescriptSK(+) |
| F1 | Early heading | lines Leaf light period insensitive, one day treatment of short day) | λZAP II |
| F | Late heading | lines Leaf (light period sensitive, one day treatment of short day) | λZAP II |
| R | Root | Root of shoot | pBluescriptSK(+) |
| R1 | Chilled Root | Root of shoot | pBluescriptSK(+) |
| S2 | Adult leaf (SD) | Mature leaf (short day condition) | pBluescriptSK(+) |
| S1 | Shoot | Green shoot | 1-9 6; pBluescriptSK(-) (λUni-ZAP XR), 101-; pBluescriptSK(+) |
| S | Etiolated Shoot | Etiolated shoot | 1-9 6; pBluescriptSK(-)(λUni-ZAP XR), 101-; pBluescriptSK(+) |

A part of the gene sequences having homology to peroxidase are registered as EST sequences and available from a public databank (DDBJ (DNA Data Bank of Japan)). Its URL is http://www.ddbj.nig.ac.jp/).

In an example below, cDNAs isolated from cDNA libraries derived from gibberellin (GA₃) callus, heat shock callus, roots, green shoots, and etiolated shoots were used. Out of the clones of the present invention, the clones whose initial letter is R are derived from library R (root), the clones having 4 digits following S are derived from library S (etiolated shoots), the clones having 4 digits following S1 are derived from library S1 (shoot), the clones having 4 digits following C5 are derived from library C5 (GA₃ treatment callus), and the clones having 4 digits following C6 are derived from library C6 (heat shock callus).

34 of the thus-obtained cDNAs having a putative open reading frame of peroxidase were selected and sequenced from both sides.

31 of the sequenced cDNAs were selected, and 5 cDNAs isolated in Ito et al. (1994) (Reference 12) and Chittoor et al. (1997) (Reference 10) were selected. For a total of 36 cDNAs, their putative amino acid sequences were analyzed by Kimura's protein distance prediction method (Kimura et al., Reference 31) and a neighbor joining method (Saitou et al . , Reference 32, and Studier et al. , Reference 33) to construct a phylogenetic tree. Analysis of amino acid sequences was limited to a region between upstream of the proximal heme binding domain and the vicinity of the 5^{tn} cysteine invariable residue (this portion corresponds to a region of glycine 168 to proline 210 of horseradish POX C isozymes (Welinder et al . , Reference 34)). This is because this region has been proposed to be important for catalytic activity determining the physiological function of each P_{OX} (Chittoor et al. (1997), Reference 10). EST clones (the remaining 3 out of 34) lacking histidine residue 2 and cysteine residue 8 which characterize POX were excluded from the phylogenetic analysis.

Thus, the 36 rice POXs were subjected to the phylogenetic analysis based on their amino acid sequences. The result is shown in Figure 1.

The above-described 36 rice POXs are divided into a plurality of clusters. Seven POXs (PIR3, R2576, R2577, R3025, POX8.1, POX5.1 and S14493) are grouped into the same cluster, and exhibit a high level of homology to pathogen-induced POXs which have been found in a plurality of plants (Chittoor (1999), Reference 6). In fact, it has been observed that POX22.3 (identical to PIR3) and POX8.1 are induced in rice leaves infected with Xanthomonas oryzae pv. oryzae (Chittoor et al., (1997), Reference 10). Similarly, the POX gene of the other clusters shown in Figure 1 are considered to have a common or related role according to their proximity on the phylogenetic tree. Representative POX genes were selected evenly from these clusters, and used. in the examples below. The names of the selected genes are indicated by boxes in Figure 1.

### (Example 2) Changes in expression of rice peroxidases derived from growth stages and plant portions

### (Growth of rice)

Rice (Oryza sativa cv. Nipponbare) was cultivated at a green house (20°C to 32°C). Roots and aerial parts of the 5-day-old young seedlings were obtained, and roots, leaf sheaths and leaf blades of the 16-day-old mature seedlings were obtained. These materials were used in the experiments below.

### (Analysis of gene expression)

Gene expression was analyzed by subjecting total RNAs isolated by the ATA method (Nagy et al., Reference 35) to RNA gel blot analysis (Ausubel et al., Reference 36).

cDNA fragments containing 3' untranslated regions of 21 rice POX genes indicated by boxes in Figure 1 were amplified by the PCR method. The amplified fragments were used as probes specific to the respective POX genes for the RNA gel blot analysis. Major sequencesr(SEQ ID NO: 43 to 59) used in the production of each clone-specific probe are shown in Table 3.

**Table 3**

| PCR probe specific to each POX | | | | |
|---|---|---|---|---|
| Primer | SEQ ID NO. | Sequence (5' - 3') | Length (bp) | Tm (°C) |
| C52903FP1 | 43 | TGTGCCCGTCGAACGCGTCG | 20 | 63 |
| C62847FP1 | 44 | TGCCCGCTCAGCTACAGC | 18 | 60 |
| prxRPAFP1 | 45 | AACCTCCAGAGCCTCTGTGC | 20 | 59 |
| prxRPARP1 | 46 | AAGGCACATACATTCAGTTC | 20 | 51 |
| R0317F1 | 47 | TCAAGACGTTCGACCTGG | 18 | 55 |
| R1420FP1 | 48 | TTCACCTCTGACGCGGCG | 18 | 60 |
| R2184FP1 | 49 | CGACAACAAGTACTACTTCG | 20 | 58 |
| R2391FP2 | 50 | GCCTCTACAACGAGACGG | 18 | 58 |
| R2576F1 | 51 | TCAAGGCCAACTGCCCA | 17 | 55 |
| S10927FP1 | 52 | CGACCTCGCCGCGCTGTCCG | 20 | 67 |
| S11222FP1 | 53 | GACGACGGCGCCCATCGTCG | 20 | 65 |
| S13316FP1 | 54 | GCGACAACACGACGCTGGCG | 20 | 63 |
| S14082FP1 | 55 | TCTTCCACTCCGACTCCGCG | 20 | 61 |
| S14493FP1 | 56 | CGGCGGCGACACCAACCTGG | 20 | 65 |
| S4325F1 | 57 | ATGTTCAGCGCCAAGGGC | 18 | 57 |
| prxRPNFP1 | 58 | CCTCGTCTCCAGCTCCGGCG | 20 | 65 |
| prxRPNRP1 | 59 | TTAAACCATATGGCAGTTGC | 20 | 51 |

The above-described RNAs were subjected to electrophoresis, followed by transcription to a membrane (HyBond N, Amersham). The specific probes were allowed to hybridize, followed by washing for 5 min once) and for 10 min (twice) in 2xSSC containing 0.1% SDS at room temperature, and then three times in 1xSSC containing 0.1% SDS for 15 min each at 65°C. Subsequently, the membrane was subjected to autoradiography at -80°C using a film for autoradiography (XA OMT, Kodak) overnight or more. The recovered film was analyzed by the BAS2000 Bioimaging analyzer (Fuji Photo Film Co., Ltd.) or PhosphorImager SI (Molecular Dynamics) in accordance with the manufacturer' s instruction. The amount of RNA loadedwas confirmed by monitoring the level of ribosome RNA(rRNA)stained by methylene blue. The expression amounts of the POXs were compared with each other with reference to the rRNA levels.

### (Changes in expression amount according to growth stage and plant site)

5-day-old and 16-day-old rice plant seedlings were produced in a manner as described above. Total RNAs from each sample were analyzed in a manner as described above. The expression amounts of the POXs were compared between different growth stages and between the roots and the aerial parts. The comparison results are shown in Figures 2A to 2C. The expression patterns of the POX genes are arranged in Figures 3 to 22. As can be seen from the figures, the 21 POX genes analyzed exhibited various expression patterns according to the growth stage and the plant site. Except for S14802, all POX genes analyzed expressed mRNA in the roots of both 5- and 16-day-old seedlings. Transcripts for 17 POX genes were detected in aerial parts (including leaf sheaths and leaf blades).

The number of types of POXs expressed on day 16 was reduced as compared to that of the 5-day-old seedlings. The reason is considered to be that POXs particularly required in the growth stage are expressed only in the juvenile period and are no longer used after the growth.

According to the results of this example, the 21 POX genes analyzed are divided into 5 classes, A1, A2, B1, B2 and C. These classes are described next to the boxes in Figure i. Site specificity where the expression ratio of root/aerial part is at least about 4/6 is categorized as "root≥aerial part", while site specificity where the ratio is less than that value is categorized as "root<aerial part".

Nine POX genes belonging to group A1 other than R2151 and R3025 were not detected in the leaf blades of 16-day-old plants. For R2151 and R3025, considerable amounts of transcript were detected in the leaf blades of the 16-day-old plants. This suggests involvement of group A1 POX genes in the basic metabolism of plant growth (e.g., crosslinking of cell wall proteins and feruloylated polysaccharides, lignification, suberization and auxin degradation). R2151, S4325, R2877, R1420, prxPRN, S13316 and R3017 were predominantly expressed in roots, while R1617, R2391, R3025 andC62847 were similarly expressed in roots and aerial parts. Therefore, these genes are categorized as group A1. The site specificity indicates that each POX gene has a role at a site at which the gene is expressed. For S10927 and S14493, their expression levels were higher in aerial parts than in roots. Therefore, these genes are categorized as group A2. It is suggested that these POX genes play a basic role in aerial parts. Further, as described in examples below, POX genes belonging to groups A1 and A2 did not respond to stressestimuli, indicating that the genes play a basic role which is not inhibited by environmental stresses.

R2693, prxPRA, R2576, R2184 and C52903 genes were predominantly expressed in roots. Therefore, these genes are categorized as group B1. R2329 and S11222 were predominantly expressed in aerial parts. Therefore, these genes are categorized as group B2. These group B genes responded to stresses as indicated in examples below.

### (Example 3) Inducibility of rice peroxidases to physical stressestimuli

16-day-old seedlings were produced under the conditions as described in Example 2. The seedlings were used to analyze the inducibility of rice peroxidase to a cutting stress and a rubbing stress as a physical stress. A cutting stress was given by cutting the tips of the leaf blades by commercially available pruning shears. A rubbing stress was given by rubbing the whole blades by hands using caborundum #600 (Nacalai Tesque).

RNAs were extracted from the leaf sheaths of a rice plant given stimuli in a manner as described in Example 2. For each POX, expression specificity was analyzed. As a control, leaf blades which were not given a stress were used. The analysis results are shown in Figures 2A to 2C, and the results from the respective genes are shown in Figure 3 to 22. Plants or leaf blade sections treated were incubated under continuous irradiation (200 µE/m²/s) at 25°C for 48 hours, followed by sampling.

For C52903 belonging to B1 and R2329 belonging to B2, their gene expressions were responsive to both a cutting stress and a rubbing stress. For prxRPA and R2576 genes belonging to B1, their expressions were induced only by a rubbing stress. For R2693 and R2184 belonging to B1, although expression was induced when leaf blades were cut into small pieces (no data shown), expression was not induced by the cutting stress or rubbing stress which was carried out in this example. This suggests that like cutting stresses lead to different expression-induction specificities according to the treatment methods . Both cutting stresses and rubbing stresses cause wound stresses. It is known that these wound stresses inhibit the normal growth and regeneration of plants, so that pathogens can easily invade plant tissues. Therefore, it is suggested that expression of peroxidase genes are controlled specifically in various stages such as suberization, lignification, and crosslinking of cell wall proteins, and the like in the course of restoration from wound stresses.

POX genes inducible to a certain wound stress may be induced by pathogens (Chittoor (1997), Reference 10, and Mohan et al. , Reference 37). Therefore, it is suggested that wound stress-inducible POXs shown in this example are also involved in a defense system against pathogen infection.

### (Example 4) Inducibility of rice peroxidases to ethephon and MeJA

16-day-old seedlings were produced under the conditions described in Example 2. With these seedlings, the inducibility of rice peroxidases to ethephon (an ethylene release factor) and MeJA (a wound information transfer substance) was analyzed. For ethephon stimulus, 1 mM ethephon solution containing 0.05% ethanol was used. For MeJA stimulus, 25 µM MeJA solution containing 0.125% Triton X-100 was used. These solutions were sprayed onto whole plants, and maintained for 48 hours.

RNAs were extracted from the leaf blades of stimulated rice plants in a manner as described in Example 2. For each POX, expression specificitywas analyzed. As a control, leaf blade without a stress were used. The analysis results are shown in Figures 2A to 2C, and the results from the respective genes are shown in Figure 3 to 22.

Ethephon is known as an ethylene release factor. MeJA is known to function as a wound information transfer substance and the like. These factors induced expression of R2693, R2329, S11222, prxRPA, R2576, R2184 and C52903 POX genes. These genes were induced in a manner similar to wound stresses, drug stresses and a UV stress in Examples 3 and 5. Therefore, it is suggested that jasmine acid (JA) and ethylene are signal compounds for the stress-inducible expression of rice POX genes. In fact, JA accumulates locally or systemically in wounded rice plants (Schweizer et al., Reference 38, and Schweizer et al., Reference 39). Therefore, these 7 MeJA-inducible POX genes are suggested to be pathogen-inducible.

### (Example 5) Inducibility of rice peroxidases to oxidative stresses

16-day-old seedlings were produced under the conditions described in Example 2. These samples were used to analyze the inducibility of rice peroxidases to ultraviolet light stimuli and paraquat as oxidative stresses. For paraquat, 1 µM paraquat solution was used. To give paraquat stimuli, leaf blade sections were suspended in the 1 µM solution for 48 hours. Ultraviolet light stimuli were given by subjecting leaf blade sections suspended in sterilized water to ultraviolet light at 175 µW/cm² for 7 minutes. As a light source for ultraviolet light, a sterilization lamp (GL-15, NEC) was used.

RNAs were extracted from the leaf blades of stimulated rice plants in a manner as described in Example 2. For each POX, expression specificity was analyzed. As a control, leaf blade without a stress were used. The analysis results are shown in Figures 2A to 2C, and the results from the respective genes are shown in Figure 3 to 22.

As can be seen from Figures 2A to 2C, expression was induced by paraquat for R2329, prxRPA, R2576 and C52903. Further, for R2693, R2329, prxRPA, R2576, R2184 and C52903, expression was induced by ultraviolet light irradiation. These are peroxidases in the same group as those whose expression was induced by wound stresses. Moreover, the expression pattern caused by paraquat treatments was similar to that caused by wound stress.

Paraquat is a nonselective contact herbicide. Paraquat inhibits proton translocation through a thylakoid membrane, leading to generation of active oxygen species and energy depletion (Babbs et al., Reference 40). It has been reported that ultraviolet light causes H₂O₂ accumulation (Murphy et al., Reference 41). Among various types of peroxidases, it had been believed that ascorbic acid peroxidase is the only H₂O₂ scavenging enzyme in plants (Asada, Reference 42). Recent progresses of biochemical research suggests that class III peroxidases including the peroxidases of the present invention are also involved in scavenging of H₂O₂ (Mehlhorn et al . , Reference 43 , and Kvaratskhelia et al., Reference 44). It is suggested that the peroxidases which are induced by paraquat as indicated in this example are involved in detoxification of H₂O₂ and the like generated by active oxygen species.

### (Example 6) Analysis of RNA expression pattern in plants inoculated with rice blast fungus (Magnaporthe grisea) and POX genes as marker gene

Next, the relationship between the POX gene groups of the present invention and rice blast was investigated. As experimental strains, three rices, i.e., rice blast-susceptible rice (Oryza sativa cv. Nipponbare) (hereinafter referred to as compatible or -Pi-i in this example), rice blast-resistant rice (Oryza sativa cv. Nipponbare) (referred to as incompatible or +Pi-i in this example ; available from the Nat. Agr. Res. Cent. of Japan) ; see Reference 61), and a compatible rice treated with probenazole (available as Oryzemate granule from Meiji Seika Kaisha Ltd.) (100 mg/ml) which is a rice blast control agricultural chemical, at the 8 leaf stage (6 weeks old, the height was almost 40 cm) were used. Each plant was cultivated in a green house (20°C to 32°C).

Each rice plant strain was treated with rice blast fungus (M. grisea race(003)) (1×10⁵ spores/ml). The time point of the treatment is regarded as day 0 (a probenazole treated group was treated with rice blast fungus two days after the probenazole treatment). Total RNAs immediately before the treatment and 2 , 3 , 4 and 5 days after the treatment were prepared in a manner as described above, and were subjected to northern analysis using the POX genes of the present invention. In this experiment, R2184, R2576, R2693 and C52903 (group B1), R2329 and S11222 (group B2), R3025 (group A1), and prxRPA (group B1) were used. The results are shown in Figure 24.

Next, formation of lesion spots caused by rice blast fungus was observed. For the compatible rice, lesion spots were found on days 4 and 5. Thereafter, formation of rice blast fungus spores was observed. For the incompatible rice, formation of lesion spots was found on days 2 to 3. These lesion spots have a particular form for enclosing rice blast fungus. Thereafter, formation of rice blast fungus spores was not found. For the probenazole-treated rice, lesion spot formation was found on days 2 to 3, as for the incompatible rice, and the lesion spots were similar to that of the compatible rice.

For R3025 andprxRPA, no significant signal was found (no data shown). Further, as can be seen from Figure 24, it is found that expression patterns of the compatible rice and the probanazole-treated rice when E2184, R2576, R2693 and R2329 were used as probes were significantly similar to the transition pattern of formation of lesion spots. These patterns match the transition pattern of lesion spot formation when another marker gene was used (no data shown).

Therefore, it was demonstrated that the POX genes of the present invention or gene groups thereof can be utilized as a marker for responses to pathogenic bacteria, such as rice blast fungus and the like, as an example of stress responses.

### (Example 7) Gene expression analysis on DNA microarray

Next, gene expression was analyzed using DNA microarrays where the POX gene groups were used as markers.

As experimental strains, the three strains used in Example 6 were used. Samples were prepared from these three strains at time points similar to those in Example 6.

At least three POX gene groups of the present invention (e.g., R2184, R2576, R2693, and the like), a control gene (e.g., conventional POX genes), other marker genes and the like were bound and immobilized onto DNA microarrays. DNAs were immobilized in accordance with a method described in http://cmgm.stanford.edu/pbrown, and the like.

Next, mRNAs were isolated from total RNA prepared above (Qiagen Midi Kit, Chatsworth, CA), and transcribed using Superscript II reverse transcriptase (Life Technologies, Grand Island, NY) and oligo(dT) in accordance with a method recommended by the manufacturers. The resultant cDNAs were treated with one unit of RNase H for 30 minutes at 37°C, and purified using a Centricon-30 spin filtration column (Amicon, Beverly, MA) to condense to less than 20 µl. One tenth of this amount of the cDNAs were labeled by a random primer polymerization reaction using Cy-3 labeled dUTP or Cy-5 labeled dUTP (each available from Amersham). To perform random primary polymerization, briefly, cDNA was added to 20 µl of the labeled reaction mixture (2 µl of 10×Klenow buffered solution (United States Biochemical)), 0.5 µl of fluorescent dUTP (25 nmol), 3 µl of random primer (Life Technologies), 2 µl of 250 µM dATP, dCTP and dGTP each and 90 µl of dTTP, and one unit of Klenow enzyme (United States Biochemical). After 3-hour incubation at 37°C, two (i.e., Cy-3 and Cy-5) reaction products were combined, and condensed and purified using a Centricon-30 spin filtration column. Thereafter, this sample was lyophilized, and dissolved in 14 µl of hybridization buffered solution (supra), followed by thermal denaturation at 99°C. The resultant sample was applied to DNA microarrays.

The thus-prepared fluorescent hybridization probes were added to DNA microarrays on which the POX gene groups were immobilized. The microarrays were covered with 22×22 mm² Hybrislip (Research Products International). Thereafter, these slides were placed in a waterproof hybridization chamber, followed by hybridization in a water bath at 65°C for 12 to 16 hours. After the hybridization, the slides were washed in 1×SSC containing 0.03% SDS, and then in 0.2×SSC and 0.05×SSC. The slides were scanned by ScanArray 3000 (GSI Lumonics, Oxnard, CA). Scanning was also carried out for the slides before the hybridization.

Thereafter, the resultant raw data on the fluorescence was subjected to correction. When there was little change in an expression amount over time, it is not assumed that a total gene expression amount fluctuate. A correction coefficient was calculated from the total or median of fluorescence signals obtained from spots so as to correct fluorescence strength (referred to as global normalization) (Reference 59). Alternatively, in the case where the expression amount of one kind of cell can be considered to be different, such as a comparison of different lineages, a correction coefficient was calculated from a fluorescence signal from a gene, such as a housekeeping gene, whose expression amount is constant. Further, amethodusing an internal reference at the time of fluorescence labeling may be used.

Based on the thus-obtained corrected data, the relationship between the POX gene groups of the present invention, and the transition of a change in expression of a gene pattern caused by infection with rice blast fungus can be simultaneously analyzed.

### (Effect of the Invention)

As described above, all peroxidase (POX) genes belonging to B1 and B2 were constitutively expressed in roots in 5-day-old and 16-day-old seedlings. Except for prxRPA, these genes were constitutively expressed in aerial parts at various levels. According to these results, the present inventors speculate that genes belonging to B1 and B2 are involved in the developmentally regulated basic metabolism and stress-responsive reactions in plants.

R2184 and C52903 belong to B1. These two POXs have putative N-terminal signal peptides and C-terminal extensions. This structure suggests that these POXs are localized in vacuoles. In contrast, R2693, prxRPA and R2576 belonging to B1, and R2329 and S11222 belonging to B2 have only putative N-terminal signal peptides. Therefore, the latter are released outside cells, i.e., they are suggested to be apoplastic peptides. Wound stresses and paraquat treatment induced the expression of both apoplastic (R2329, prx2576 and R2576) and vacuolar (e.g., R2184 and C52903) POXs. Therefore, it is suggested a plurality of POXs having different characteristics, including both vacuolar POXs and apoplastic (i.e., extracellular secretory) POXs, function differently or cooperatively in the same physiological reactions. One of the reasons why a variety of POXs are contained in a single plant is considered to be that plant physiology has to be controlled in such a subtle manner.

### (Industrially Applicability)

The disclosure the present invention provides a set of peroxidase (POX) genes useful for evaluation of the characteristics of any plants including plant varieties of the family rice. Further, various POX genes and promoters therefor having a variety of expression specificities are provided. These genes and promoters are useful as materials for modification of plants to confer desired characteristics. In the present invention, the genes of the present invention and promoters thereof can be used to analyze gene expression in plants.

### References

(1) Welinder K.G. et al. (1992), Curr. Opine Struct. Biol. , 2:388
(2) Whetten R. et al. (1995), Plant Cell 7, 1001-1013
(3) Espelie K.E. et al. (1986), Plant Physiol. 81, 487-492
(4) Fry S.C. (1986) Ann. Rev. Plant. Physiol. 37, 165-186
(5) Hinman R.L. et al. (1965), Biochemistry 4, 144-158
(6) Chittoor J.M. et al. (1999), Pathogenesis-Related Proteins in Plants (Datta and Muthukrishnan Ed.) 171-193, CRC Press, Boca Raton
(7) Amaya I. et al. (1999), FEBS Lett. 457, 80-84
(8) Abeles F.B. et al. (1988), Plant Physiol. 87. 609-615
(9) Horton R.F. et al. (1993), J. Plant Physiol. 141: 690
(10) Chittoor J.M. et al. (1997), Mol. Plant-Microbe Interact. 7: 861-867
(11) Ito H. et al. (1991), Agric. Biol. Chem. 55 : 2445-2454
(12) Ito H. et al. (1994), Plant Cell Rep. 13: 361-366
(13) Langrimini L.M. et al. (1987), Plant Physiol. 84: 438-442
(14) Oohashi Y. (1975), Doctoral dissertation in the Agricultural Chemistry field in the Department of Agriculture, Graduate School of Nogoya University
(15) Yamamoto K. et al. (1997), Plant Mol. Biol. 35 : 135-144
(16) Østergaard L. et al. (1998), FEBS Lett. 433: 98-102
(17) Pearson et al. (1988),PNAS 85: 2444-2448
(18) Tijssen(1993),Laboratory TechnniquesIn Biochemistry And Molecular Biology-Hybridization With Nucleic Acid Probes Part I, Second Chapter "Overview of principles of hybridization and the strategy of nucleic acid probe assay", Elsevier,New York
(19) Nagel et al. (1990), Microbiol. Lett., 67, 325
(20) Gelvin et al. Ed. (1994), Plant Molecular Biology Manual Kluwer Academic Press Publishers)
(21) Shimamoto et al. (1989), Nature, 338: 274-276
(22) Rhodes et al. (1989), Science, 240: 204-207
(23) Christou et al. (1991), Bio/Technology 9: 957-962
(24) Datta et al.,(1990), Bio/Technology 8: 736-740
(25) Moore et al. ,Current Biology(1995),Vol. 5, 737-739
(26) Nagamura et al. (1997), Nogyo-Seibutsu-Sigen-Kenkyujo News [News fromNational Institute of Agricultural Research], No.57, 1-2
(27) Yoshida K.et al.(1998), Syokubutu-no-Kagaku-Tyosetsu [Chemical Control of Plants], Vol. 33, No. 2, 121-125
(28) Kawaoka A. et al. (1998), Syokubutu-no-Kagaku-Tyosetsu [Chemical Control of Plants], Vol. 33, No. 2, 125-127
(29) Kwak S-S. et al. (1998), Syokubutu-no-Kagaku-Tyosetsu [Chemical Control of Plants], Vol. 33,No. 2, 127-130
(30) Hiraga S. et al. (1998), Syokubutu-no-Kagaku-Tyosetsu [Chemical Control of Plants], Vol. 33, No. 2, 131-136
(31) Kimura et al. (1980), J. Mol. Evol. 16: 111-120
(32) Saitou et al. (1987), Mol. Biol. Evol. 5: 729-731
(33) Studier et al. (1988), Mol. Biol. Evol. 5: 729-731
(34) Welinder K.G. (1979), Eur. J. Biochem. 96: 483-502
(35) Nagy et al. (1988), Plant Molecular Biology Manual (Gelvin et al. Ed.), B4:1-29, Kluwer Academic Publiishers, Dordrecht, Netherlands
(36) Ausubel F.A. et al. Ed. (1988), Current Protocols in Molecular Biology, Wiley, New York, NY
(37) Mohan R. et al. (1990), Plant Physiol. 92, 276-280
(38) Schweizer P. et al. (1998), Plant J. 14, 475-481
(39) Schweizer P. et al. (1997), Plant Physiol. 114 : 79-88
(40) Babbs C.F. et al. (1989), Plant Physiol. 90: 1267-1270
(41) Murphy T.M. et al. (1990), Physiol. Plant. 78 : 247-253
(42) Asada K.(1992), Physiol.Plant. 85:235-241
(43) Mehlhorn H. et al. (1996), FEBS Lett. 378, 203-206
(44) Kvaratskhelia M. et al. (1997), Plant Physiol.114, 1237-1245
(45) Fambrough D et al. (1999), Cell 97, 727-741
(46) Iyer VR et al., (1999), Science 283: 83-87
(47) Marton MJ, (1999), Nat. Med. 4: 1293-1301
(48) Cargill M et al., (1999), Nat. Genet. 22:231-238
(49) Halushka MK et al., (1999) Nat. Genet. 22:239-246
(50) Marshall A et al., (1998) Nat. Biotechnol. 16: 27-31
(51) Ramsay G et al., (1998) Nat. Biotechnol. 16 40-44
(52) Fodor SP et al., (1991) Science 251: 767-773
(53) Lockart DJ et al. (1996) Nat . Biotechnol. : 14 : 1675-1680.
(54) Schena M et al. (1996) Science 270: 467-470
(55) Schena M et al. (1996) Proc. Natl. Acad. Sci. (USA) 93: 10614-10619
(56) DeRisi J et al. (1998) Nat. Genet. 14: 457-460
(57) Ermolaeva O et al. (1998) Nat. Genet. 20:19-23
(58) Schenk PM et al. (2000) Proc. Natl. Acad. Sci. (USA) 97: 11655-11660
(59) Wilson M et al. (1999) Proc. Natl. Acad. Sci. (USA) 96 :12833-12838
(60) Sambrook J et al. (1987) Molecular Cloning: A Laboratory Manual, 2nd Ed., Cold Spring Harbor Laboratory Press, Cold Spring Harbor, NY
(61) IseK. etal. (1998) Ikuzatu [Breeding] 38(Special Issue 2) : 404-405

SEQUENCE LISTING

<110> National Institute of Agrobiological Sciences

<120> Rice peroxidases with various characteristics

<130> P014401EPA

<150> JP1999-352472
<151> 1999-12-10

<160> 59

<170> PatentIn Ver. 2.1

<210> 1
<211> 1204
<212> DNA
<213> Oryza sativa

<220>
<221> CDS
<222> (9)..(989)

<220>
<223> prxRPA

<400> 1
tgtgagag atg gag tac tct tac agc tac agg ttc atg ctt gta tgc tct 50
Met Glu Tyr Ser Tyr Ser Tyr Arg Phe Met Leu Val Cys Ser
1               5                   10

gtt ctt gta ctg tgc ctt aat act cgg ggt gcg aga tgc cag tta tcc 98
Val Leu Val Leu Cys Leu Asn Thr Arg Gly Ala Arg Cys Gln Leu Ser
15                  20                  25                  30

gac gat ttc tac gac tac ata tgt cct gat gtg tac acc gtt gtc cag 146
Asp Asp Phe Tyr Asp Tyr Ile Cys Pro Asp Val Tyr Thr Val Val Gln
                35                  40                  45

cag cat gtt tat gct gcc atg agg act gag atg agg atg ggt gcc tcc 194
Gln His Val Tyr Ala Ala Met Arg Thr Glu Met Arg Met Gly Ala Ser
            50                  55                  60

ctc cta agg ctc cat ttc cat gac tgc ttt gtc aat ggg tgt gac ggt 242
Leu Leu Arg Leu His Phe His Asp Cys Phe Val Asn Gly Cys Asp Gly
        65                  70                  75

tcc atc ctt ctg gac ggt gac gac ggc gag aaa ttt gca ctt ccc aac 290
Ser Ile Leu Leu Asp Gly Asp Asp Gly Glu Lys Phe Ala Leu Pro Asn
    80                  85                  90

aag acc tct gtc aga ggg ttc gaa gtc att gac gcg ata aag gaa gat 338
Lys Thr Ser Val Arg Gly Phe Glu Val Ile Asp Ala Ile Lys Glu Asp
95                  100                 105                 110

ctc gag aac atc tgc cct gaa gtt gtt tcc tgc gcc gac att gta gcc 386
Leu Glu Asn Ile Cys Pro Glu Val Val Ser Cys Ala Asp Ile Val Ala
                115                 120                 125

ctt gca gct ggc tat gga gta cta ttt agt gga ggc cct tac tat gac 434
Leu Ala Ala Gly Tyr Gly Val Leu Phe Ser Gly Gly Pro Tyr Tyr Asp
            130                 135                 140

gtt ctt ctc ggt aga agg gat ggt ctt gtc gca aat caa tca gga gct    482
Val Leu Leu Gly Arg Arg Asp Gly Leu Val Ala Asn Gln Ser Gly Ala
        145                 150                 155

gac aac ggc ctc cct tca ccg ttc gaa ccc atc aaa tcg atc ata cag    530
Asp Asn Gly Leu Pro Ser Pro Phe Glu Pro Ile Lys Ser Ile Ile Gln
    160                 165                 170

aag ttc aat gat gtc ggc ctc gac aca acc gat gtt gtc gtc cta tca    578
Lys Phe Asn Asp Val Gly Leu Asp Thr Thr Asp Val Val Val Leu Ser
175                 180                 185                 190

gga ggg cac acg atc gga cga gcc cgg tgc acg ctg ttc agc aac cgg    626
Gly Gly His Thr Ile Gly Arg Ala Arg Cys Thr Leu Phe Ser Asn Arg
                195                 200                 205

ttg tcg acc acc tca agc tca gcc gac ccg acg ctg gac gcc acc atg    674
Leu Ser Thr Thr Ser Ser Ser Ala Asp Pro Thr Leu Asp Ala Thr Met
            210                 215                 220

gcc gcc aac ctc cag agc ctc tgt gcc ggt gga gac ggc aac gag acc    722
Ala Ala Asn Leu Gln Ser Leu Cys Ala Gly Gly Asp Gly Asn Glu Thr
        225                 230                 235

acc gtg ctg gac atc acc tcc gcc tac gtt ttc gac aac cgc tac tac    770
Thr Val Leu Asp Ile Thr Ser Ala Tyr Val Phe Asp Asn Arg Tyr Tyr
    240                 245                 250

cag aac ctc ctc aat cag aaa ggc ctc ctg tcc tcc gac cag ggc ctc    818
Gln Asn Leu Leu Asn Gln Lys Gly Leu Leu Ser Ser Asp Gln Gly Leu
255                 260                 265                 270

ttc tcc agc gac gac ggc atc gcc aac acc aag gag ctg gtg gag act    866
Phe Ser Ser Asp Asp Gly Ile Ala Asn Thr Lys Glu Leu Val Glu Thr
                275                 280                 285

tac agc gca gat gcc cac aag ttc ttc tgg gat ttt ggc aga tcc atg    914
Tyr Ser Ala Asp Ala His Lys Phe Phe Trp Asp Phe Gly Arg Ser Met
            290                 295                 300

gtg aag atg ggc aac atc agc cca ctc acc ggt gac gac ggc cag att    962
Val Lys Met Gly Asn Ile Ser Pro Leu Thr Gly Asp Asp Gly Gln Ile
        305                 310                 315

cgc aag aac tgc agg gtt gtt aat taa ctgagcttca gtgtgttgaa    1009
Arg Lys Asn Cys Arg Val Val Asn
    320                 325

aagatagtaa atttcttgta cttttcacaa ggcgattgag acgatgtgtg ttatgttttg 1069

atgttacatg aatgcttgaa gaagcagaag taataacgat gtgattgtga ctagtttgtg 1129

aactgatcgt caacaaagat gtgatccatt ggaaaaaaag aactgaatgt atgtgcctta 1189

aaaaaaaaaa aaaaa                                                  1204

<210> 2
<211> 326
<212> PRT
<213> Oryza sativa
<223> prxRPA

<400> 2

Met Glu Tyr Ser Tyr Ser Tyr Arg Phe Met Leu Val Cys Ser Val Leu
1               5                   10                  15

Val Leu Cys Leu Asn Thr Arg Gly Ala Arg Cys Gln Leu Ser Asp Asp
            20                  25                  30

Phe Tyr Asp Tyr Ile Cys Pro Asp Val Tyr Thr Val Val Gln Gln His
        35                  40                  45

Val Tyr Ala Ala Met Arg Thr Glu Met Arg Met Gly Ala Ser Leu Leu
    50                  55                  60

Arg Leu His Phe His Asp Cys Phe Val Asn Gly Cys Asp Gly Ser Ile
65                  70                  75                  80

Leu Leu Asp Gly Asp Asp Gly Glu Lys Phe Ala Leu Pro Asn Lys Thr
                85                  90                  95

Ser Val Arg Gly Phe Glu Val Ile Asp Ala Ile Lys Glu Asp Leu Glu
            100                 105                 110

Asn Ile Cys Pro Glu Val Val Ser Cys Ala Asp Ile Val Ala Leu Ala
        115                 120                 125

Ala Gly Tyr Gly Val Leu Phe Ser Gly Gly Pro Tyr Tyr Asp Val Leu
    130                 135                 140

Leu Gly Arg Arg Asp Gly Leu Val Ala Asn Gln Ser Gly Ala Asp Asn
145                 150                 155                 160

Gly Leu Pro Ser Pro Phe Glu Pro Ile Lys Ser Ile Ile Gln Lys Phe
                165                 170                 175

Asn Asp Val Gly Leu Asp Thr Thr Asp Val Val Val Leu Ser Gly Gly
            180                 185                 190

His Thr Ile Gly Arg Ala Arg Cys Thr Leu Phe Ser Asn Arg Leu Ser
        195                 200                 205

Thr Thr Ser Ser Ser Ala Asp Pro Thr Leu Asp Ala Thr Met Ala Ala
    210                 215                 220

Asn Leu Gln Ser Leu Cys Ala Gly Gly Asp Gly Asn Glu Thr Thr Val
225                 230                 235                 240

Leu Asp Ile Thr Ser Ala Tyr Val Phe Asp Asn Arg Tyr Tyr Gln Asn
                245                 250                 255

Leu Leu Asn Gln Lys Gly Leu Leu Ser Ser Asp Gln Gly Leu Phe Ser
            260                 265                 270

Ser Asp Asp Gly Ile Ala Asn Thr Lys Glu Leu Val Glu Thr Tyr Ser
        275                 280                 285

Ala Asp Ala His Lys Phe Phe Trp Asp Phe Gly Arg Ser Met Val Lys
    290                 295                 300

Met Gly Asn Ile Ser Pro Leu Thr Gly Asp Asp Gly Gln Ile Arg Lys
305                 310                 315                 320

Asn Cys Arg Val Val Asn
                325

<210> 3
<211> 1218
<212> DNA
<213> Oryza sativa

<220>
<221> CDS
<222> (50)..(1021)

<220>
<223> r2877

<400> 3
gtccatcgat catctcttct cttccagcaa ggaagcagca gagaagaca atg gca gca 58
Met Ala Ala
1

tcg gcc atg aag ctc gcc atg gcg gtg gcg tgc gcg ctg gcg ctc gcg 106
Ser Ala Met Lys Leu Ala Met Ala Val Ala Cys Ala Leu Ala Leu Ala
    5                   10                  15

tcg gcg tgc cac ggc ctg cag ctg ggc tac tac aag cag tcg tgc ccc 154
Ser Ala Cys His Gly Leu Gln Leu Gly Tyr Tyr Lys Gln Ser Cys Pro
20                  25                  30                  35

cgc gtg gag gcc atc gtg agg gac gag gtg aag aag ttc gtc tac aag 202
Arg Val Glu Ala Ile Val Arg Asp Glu Val Lys Lys Phe Val Tyr Lys
                40                  45                  50

gac gcc ggc atc ggc gcc gga ctc atc cgc ctc gtc ttc cac gac tgc 250
Asp Ala Gly Ile Gly Ala Gly Leu Ile Arg Leu Val Phe His Asp Cys
            55                  60                  65

ttc gtc gag gga tgt gat ggc tcg gtg ctc ctg gac cca act ccg gcg 298
Phe Val Glu Gly Cys Asp Gly Ser Val Leu Leu Asp Pro Thr Pro Ala
        70                  75                  80

aac ccg aag ccg gag aag ctc agc ccg ccc aac atg ccc agc ctc cgc 346
Asn Pro Lys Pro Glu Lys Leu Ser Pro Pro Asn Met Pro Ser Leu Arg
    85                  90                  95

ggc ttc gag gtg atc gac gcc gcc aag gac gcc gtc gag aag gtc tgc 394
Gly Phe Glu Val Ile Asp Ala Ala Lys Asp Ala Val Glu Lys Val Cys
100                 105                 110                 115

ccc ggc gtg gtc tcg tgc gcc gac atc gtc gcc ttc gcc gcc cgc gac 442
Pro Gly Val Val Ser Cys Ala Asp Ile Val Ala Phe Ala Ala Arg Asp
                120                 125                 130

gcc gcc tac ttc ctc agc aga ttc agg gtc aag atc aac gtc cca ggt 490
Ala Ala Tyr Phe Leu Ser Arg Phe Arg Val Lys Ile Asn Val Pro Gly
            135                 140                 145

gga cgc ctc gat ggc cgc cgc tcc ctc gac tcc gac gcc ctc aac aac 538
Gly Arg Leu Asp Gly Arg Arg Ser Leu Asp Ser Asp Ala Leu Asn Asn
        150                 155                 160

ctg ccg ccg ccc aac ttc aac gtg aac cag ctc atc ggc gcg ttc gcc 586
Leu Pro Pro Pro Asn Phe Asn Val Asn Gln Leu Ile Gly Ala Phe Ala
    165                 170                 175

gcc aag ggc ctc gac gcc gag gac atg gtg gtg ctc tcc ggc gcc cac 634
Ala Lys Gly Leu Asp Ala Glu Asp Met Val Val Leu Ser Gly Ala His
180                 185                 190                 195

acc gtc ggc cgc tcc cac tgc tcc tcc ttc gtc tcg gac cgc gtc gcc 682
Thr Val Gly Arg Ser His Cys Ser Ser Phe Val Ser Asp Arg Val Ala
                200                 205                 210

gcg ccc tcc gac atc aac ggc ggc ttc gcc aac ttc ctc aag cag agg 730
Ala Pro Ser Asp Ile Asn Gly Gly Phe Ala Asn Phe Leu Lys Gln Arg
            215                 220                 225

tgc ccg gcc aac ccg acc tcc agc aac gac ccg acg gtg aac cag gac 778
Cys Pro Ala Asn Pro Thr Ser Ser Asn Asp Pro Thr Val Asn Gln Asp
        230                 235                 240

gcc gtc acg ccc aac gcg ttc gac aac cag tac tac aag aac gtg gtg 826
Ala Val Thr Pro Asn Ala Phe Asp Asn Gln Tyr Tyr Lys Asn Val Val
    245                 250                 255

gcg cac aag gtg ctc ttc gcg tcg gac gcg gcg ctg ctg acg tcg ccg 874
Ala His Lys Val Leu Phe Ala Ser Asp Ala Ala Leu Leu Thr Ser Pro
260                 265                 270                 275

gcg acg gcg aag atg gtg tcg gac aac gcc aac atc ccg ggg tgg tgg 922
Ala Thr Ala Lys Met Val Ser Asp Asn Ala Asn Ile Pro Gly Trp Trp
                280                 285                 290

gag gac aag ttc gcc aag gcg ttc gtc aag atg gca tcc gtc ggt gtc 970
Glu Asp Lys Phe Ala Lys Ala Phe Val Lys Met Ala Ser Val Gly Val
            295                 300                 305

aag acc ggc tac ccc ggc gag atc agg agg cac tgc agg gtc gtc aac 1018
Lys Thr Gly Tyr Pro Gly Glu Ile Arg Arg His Cys Arg Val Val Asn
        310                 315                 320

taa tcatctatat attgaattca acgtgtgttc catcaaattt tctcattgct 1071

acgttttgtt tttgtattta ttcgttcatt cttttgtgta gtgtattttg tgttgattat 1131

tatatatgtc ctctcctatt atgtaattgt gtaatatgac actttatcat gtaataacat 1191

tggaatatat ttttggagta aatttca 1218

<210> 4
<211> 323
<212> PRT
<213> Oryza sativa
<223> r2877

<400> 4
Met Ala Ala Ser Ala Met Lys Leu Ala Met Ala Val Ala Cys Ala Leu
1               5                   10                  15

Ala Leu Ala Ser Ala Cys His Gly Leu Gln Leu Gly Tyr Tyr Lys Gln
            20                  25                  30

Ser Cys Pro Arg Val Glu Ala Ile Val Arg Asp Glu Val Lys Lys Phe
        35                  40                  45

Val Tyr Lys Asp Ala Gly Ile Gly Ala Gly Leu Ile Arg Leu Val Phe
    50                  55                  60

His Asp Cys Phe Val Glu Gly Cys Asp Gly Ser Val Leu Leu Asp Pro
65                  70                  75                  80

Thr Pro Ala Asn Pro Lys Pro Glu Lys Leu Ser Pro Pro Asn Met Pro
                85                  90                  95

Ser Leu Arg Gly Phe Glu Val Ile Asp Ala Ala Lys Asp Ala Val Glu
            100                 105                 110

Lys Val Cys Pro Gly Val Val Ser Cys Ala Asp Ile Val Ala Phe Ala
        115                 120                 125

Ala Arg Asp Ala Ala Tyr Phe Leu Ser Arg Phe Arg Val Lys Ile Asn
    130                 135                 140

Val Pro Gly Gly Arg Leu Asp Gly Arg Arg Ser Leu Asp Ser Asp Ala
145                 150                 155                 160

Leu Asn Asn Leu Pro Pro Pro Asn Phe Asn Val Asn Gln Leu Ile Gly
                165                 170                 175

Ala Phe Ala Ala Lys Gly Leu Asp Ala Glu Asp Met Val Val Leu Ser
            180                 185                 190

Gly Ala His Thr Val Gly Arg Ser His Cys Ser Ser Phe Val Ser Asp
        195                 200                 205

Arg Val Ala Ala Pro Ser Asp Ile Asn Gly Gly Phe Ala Asn Phe Leu
    210                 215                 220

Lys Gln Arg Cys Pro Ala Asn Pro Thr Ser Ser Asn Asp Pro Thr Val
225                 230                 235                 240

Asn Gln Asp Ala Val Thr Pro Asn Ala Phe Asp Asn Gln Tyr Tyr Lys
                245                 250                 255

Asn Val Val Ala His Lys Val Leu Phe Ala Ser Asp Ala Ala Leu Leu
            260                 265                 270

Thr Ser Pro Ala Thr Ala Lys Met Val Ser Asp Asn Ala Asn Ile Pro
        275                 280                 285

Gly Trp Trp Glu Asp Lys Phe Ala Lys Ala Phe Val Lys Met Ala Ser
    290                 295                 300

Val Gly Val Lys Thr Gly Tyr Pro Gly Glu Ile Arg Arg His Cys Arg
305                 310                 315                 320

Val Val Asn

<210> 5
<211> 1444
<212> DNA
<213> Oryza sativa

<220>
<221> CDS
<222> (108)..(1109)

<220>
<223> r1420

<400> 5
cgagcacttc tctctcttcc tgttattagc tatagcagct taattagctt aagcacaaaa 60

ggtacagtaa gcctaagcta gctacacact aagcaagctt attagcc atg gct tca 116
Met Ala Ser
1

tca cca agt ctg cca ttg gtg acg tgt gcc ctg ctg ctg ctg ctg gcc 164
Ser Pro Ser Leu Pro Leu Val Thr Cys Ala Leu Leu Leu Leu Leu Ala
    5                   10                  15

gtg gca tgc cag gct cac cct tac tgg cca ctg gag ttg gcg tac tac 212
Val Ala Cys Gln Ala His Pro Tyr Trp Pro Leu Glu Leu Ala Tyr Tyr
20                  25                  30                  35

cgc gac aag tgc ccc cag gcc gag gcc gtc gtc aag gcc gtc gtc ggg 260
Arg Asp Lys Cys Pro Gln Ala Glu Ala Val Val Lys Ala Val Val Gly
                40                  45                  50

gag gcc gtc cgc cag aac ccc ggc aat ggc gcc gcc gtc atc cgc atg 308
Glu Ala Val Arg Gln Asn Pro Gly Asn Gly Ala Ala Val Ile Arg Met
            55                  60                  65

ctc ttc cac gac tgc ttt gtc gag ggt tgt gat gct tcg atc ctc ctg 356
Leu Phe His Asp Cys Phe Val Glu Gly Cys Asp Ala Ser Ile Leu Leu
        70                  75                  80

gac ccg acg ccg ttc aac ccg acg cca gag aag ctg agc gcg ccg aac 404
Asp Pro Thr Pro Phe Asn Pro Thr Pro Glu Lys Leu Ser Ala Pro Asn
    85                  90                  95

aac ccg tcc atg cgg ggc ttc gac ctc atc gac gcg atc aag cac gcc 452
Asn Pro Ser Met Arg Gly Phe Asp Leu Ile Asp Ala Ile Lys His Ala
100                 105                 110                 115

gtg gag gcg gcg tgc ccg ggc gtc gtc tcg tgc gcc gac atc atc gcc 500
Val Glu Ala Ala Cys Pro Gly Val Val Ser Cys Ala Asp Ile Ile Ala
                120                 125                 130

ttc gcg gcg cgc gac gcc acc tac ttc ctc agc ggc ggg aag gtc tac 548
Phe Ala Ala Arg Asp Ala Thr Tyr Phe Leu Ser Gly Gly Lys Val Tyr
            135                 140                 145

ttc gac atg ccg tcg ggc cgc cgc gac ggg acc ttc tcc aac gac tcc 596
Phe Asp Met Pro Ser Gly Arg Arg Asp Gly Thr Phe Ser Asn Asp Ser
        150                 155                 160

ggc ccg atc gac ttc ctc ccg ccg ccg acg tcc aac ctc agc gac ctc 644
Gly Pro Ile Asp Phe Leu Pro Pro Pro Thr Ser Asn Leu Ser Asp Leu
    165                 170                 175

gtc tcc agc ttc gcc gtc aag ggc ctc tcc gtg gag gac atg gtg gtg 692
Val Ser Ser Phe Ala Val Lys Gly Leu Ser Val Glu Asp Met Val Val
180                 185                 190                 195

ctc tcg ggc gcc cac acc gtc ggc cgc tcc cac tgc tcc tcc ttc gtc 740
Leu Ser Gly Ala His Thr Val Gly Arg Ser His Cys Ser Ser Phe Val
                200                 205                 210

ccc gac cgc ctc aac gcc tcc gtc ttc tcc gac atc gac ggc ggc ttc 788
Pro Asp Arg Leu Asn Ala Ser Val Phe Ser Asp Ile Asp Gly Gly Phe
            215                 220                 225

gcc tgg ttc ctc agg tcg cag tgc ccg ctc gac gcg acg ccc ggc ggc 836
Ala Trp Phe Leu Arg Ser Gln Cys Pro Leu Asp Ala Thr Pro Gly Gly
        230                 235                 240

aac gat ccc acg gtg atg ctg gac ttc gtg acg ccc aac acg ctg gac 884
Asn Asp Pro Thr Val Met Leu Asp Phe Val Thr Pro Asn Thr Leu Asp
    245                 250                 255

aac cag tac tac aag aac gtg ctc gac cac aag gtg ctc ttc acc tct 932
Asn Gln Tyr Tyr Lys Asn Val Leu Asp His Lys Val Leu Phe Thr Ser
260                 265                 270                 275

gac gcg gcg ctc ctg acg tcg ccg gag acg gcg aag atg gtg gtg gac 980
Asp Ala Ala Leu Leu Thr Ser Pro Glu Thr Ala Lys Met Val Val Asp
                280                 285                 290

aac gcc gtc atc ccc ggg tgg tgg gag gac agg ttc aag gcg gcc atg 1028
Asn Ala Val Ile Pro Gly Trp Trp Glu Asp Arg Phe Lys Ala Ala Met
            295                 300                 305

gtg aag ttg gcg agc atc cag gtg aag acc ggg tac cag ggg cag atc 1076
Val Lys Leu Ala Ser Ile Gln Val Lys Thr Gly Tyr Gln Gly Gln Ile
        310                 315                 320

agg aag aac tgc agg gtc atc aac tac tga tta atctacaagt ttagctgcgt 1129
Arg Lys Asn Cys Arg Val Ile Asn Tyr Leu
    325                 330

gattcaatta cctggtgaag tacgtgctca acgatgacga cgtacacggt ttgatagttt 1189

cacaccctcc ttgatcaagt cgcaagcgta cgtgttgatg atgttatgat tggatgtgac 1249

ttttgcttca catttatgtt ctccctcttt tgttaccatt ccttaattcc tcatttgtga 1309

accccaaggt ttacatgcta tggatttctt tgttttgacc taattgtgta ataaggtcat 1369

atgtacttgg aataattgta agttaagttt tttgcaacaa gtgcccgcta tatattgatg 1429

tttatctgaa atttg 1444

<210> 6
<211> 332
<212> PRT
<213> Oryza sativa
<223> r1420

<400> 6
Met Ala Ser Ser Pro Ser Leu Pro Leu Val Thr Cys Ala Leu Leu Leu
1               5                   10                  15

Leu Leu Ala Val Ala Cys Gln Ala His Pro Tyr Trp Pro Leu Glu Leu
            20                  25                  30

Ala Tyr Tyr Arg Asp Lys Cys Pro Gln Ala Glu Ala Val Val Lys Ala
        35                  40                  45

Val Val Gly Glu Ala Val Arg Gln Asn Pro Gly Asn Gly Ala Ala Val
    50                  55                  60

Ile Arg Met Leu Phe His Asp Cys Phe Val Glu Gly Cys Asp Ala Ser
65                  70                  75                  80

Ile Leu Leu Asp Pro Thr Pro Phe Asn Pro Thr Pro Glu Lys Leu Ser
                85                  90                  95

Ala Pro Asn Asn Pro Ser Met Arg Gly Phe Asp Leu Ile Asp Ala Ile
            100                 105                 110

Lys His Ala Val Glu Ala Ala Cys Pro Gly Val Val Ser Cys Ala Asp
        115                 120                 125

Ile Ile Ala Phe Ala Ala Arg Asp Ala Thr Tyr Phe Leu Ser Gly Gly
    130                 135                 140

Lys Val Tyr Phe Asp Met Pro Ser Gly Arg Arg Asp Gly Thr Phe Ser
145                 150                 155                 160

Asn Asp Ser Gly Pro Ile Asp Phe Leu Pro Pro Pro Thr Ser Asn Leu
                165                 170                 175

Ser Asp Leu Val Ser Ser Phe Ala Val Lys Gly Leu Ser Val Glu Asp
            180                 185                 190

Met Val Val Leu Ser Gly Ala His Thr Val Gly Arg Ser His Cys Ser
        195                 200                 205

Ser Phe Val Pro Asp Arg Leu Asn Ala Ser Val Phe Ser Asp Ile Asp
    210                 215                 220

Gly Gly Phe Ala Trp Phe Leu Arg Ser Gln Cys Pro Leu Asp Ala Thr
225                 230                 235                 240

Pro Gly Gly Asn Asp Pro Thr Val Met Leu Asp Phe Val Thr Pro Asn
                245                 250                 255

Thr Leu Asp Asn Gln Tyr Tyr Lys Asn Val Leu Asp His Lys Val Leu
            260                 265                 270

Phe Thr Ser Asp Ala Ala Leu Leu Thr Ser Pro Glu Thr Ala Lys Met
        275                 280                 285

Val Val Asp Asn Ala Val Ile Pro Gly Trp Trp Glu Asp Arg Phe Lys
    290                 295                 300

Ala Ala Met Val Lys Leu Ala Ser Ile Gln Val Lys Thr Gly Tyr Gln
305                 310                 315                 320

Gly Gln Ile Arg Lys Asn Cys Arg Val Ile Asn Tyr
                325                 330


<210> 7
<211> 1325
<212> DNA
<213> Oryza sativa

<220>
<221> CDS
<222> (66)..(1046)

<220>
<223> r0317

<400> 7
ctcttctagc cttagcctag ctagctagct ttgttgtcta gctctgatcg aggttggtgg 60

tgatc atg gcg tcg tcg agg gtg atc cta gcg ctg ctg ctc gcg gcg gcg 110
      Met Ala Ser Ser Arg Val Ile Leu Ala Leu Leu Leu Ala Ala Ala
      1               5                   10                  15

gcg gtg atg gcg tcg tcg gcg cag ctg gac gag aag ttc tac agc aat   158

Ala Val Met Ala Ser Ser Ala Gln Leu Asp Glu Lys Phe Tyr Ser Asn
                20                  25                  30

tcg tgc ccc agc gtg gag gcc gtc gtc cgg aag gag atg gtg cgc gcg 206
Ser Cys Pro Ser Val Glu Ala Val Val Arg Lys Glu Met Val Arg Ala
            35                  40                  45

ctc ggc cgc gcg ccc agc ctc gcc ggc ccg ctc ctc agg atg cac ttc 254
Leu Gly Arg Ala Pro Ser Leu Ala Gly Pro Leu Leu Arg Met His Phe
        50                  55                  60

cac gac tgt ttc gtc agg ggt tgc gac ggc tcg gtg ctg ctc gac tcg 302
His Asp Cys Phe Val Arg Gly Cys Asp Gly Ser Val Leu Leu Asp Ser
    65                  70                  75

gcc ggg aac agc acg gcg gag aag gac gcg acg ccg aac cag acg ctg 350
Ala Gly Asn Ser Thr Ala Glu Lys Asp Ala Thr Pro Asn Gln Thr Leu
80                  85                  90                  95

cgc ggg ttc ggc ttc gtc gag agg gtg aag gcc gcc gtg gag aag gca 398
Arg Gly Phe Gly Phe Val Glu Arg Val Lys Ala Ala Val Glu Lys Ala
                100                 105                 110

tgc ccg ggc acc gtc tcc tgc gcc gac gtg ctc gcg ctc atg gcc agg 446
Cys Pro Gly Thr Val Ser Cys Ala Asp Val Leu Ala Leu Met Ala Arg
            115                 120                 125

gac gcc gtg tgg ctg agc aag ggc ccg ttc tgg gcg gtg cct ctc ggc 494
Asp Ala Val Trp Leu Ser Lys Gly Pro Phe Trp Ala Val Pro Leu Gly
        130                 135                 140

cgc cgc gac ggc agg gtg tcc atc gcc aac gag acc gac cag ctg ccg 542
Arg Arg Asp Gly Arg Val Ser Ile Ala Asn Glu Thr Asp Gln Leu Pro
    145                 150                 155

cct ccc acc gcc aac ttc acc gag ctc acc cag atg ttc gcc gcc aag 590
Pro Pro Thr Ala Asn Phe Thr Glu Leu Thr Gln Met Phe Ala Ala Lys
160                 165                 170                 175

aac ctc gac ctc aag gac ctc gtc gtc ctc tcc gct ggg cac acg atc 638
Asn Leu Asp Leu Lys Asp Leu Val Val Leu Ser Ala Gly His Thr Ile
                180                 185                 190

ggg acg tcg cac tgc ttc tcc ttc act gac agg ctg tac aac ttc acc 686
Gly Thr Ser His Cys Phe Ser Phe Thr Asp Arg Leu Tyr Asn Phe Thr
            195                 200                 205

ggc ctg gac aac gcc cac gac atc gac ccg acg ctg gag ctg cag tac 734
Gly Leu Asp Asn Ala His Asp Ile Asp Pro Thr Leu Glu Leu Gln Tyr
        210                 215                 220

atg gcg agg ctg agg agc aag tgc acg agc ctc caa gac aac acg acg 782
Met Ala Arg Leu Arg Ser Lys Cys Thr Ser Leu Gln Asp Asn Thr Thr
    225                 230                 235

ctg gtg gag atg gac ccg ggg agc ttc aag acg ttc gac ctg ggg tac 830
Leu Val Glu Met Asp Pro Gly Ser Phe Lys Thr Phe Asp Leu Gly Tyr
240                 245                 250                 255

ttc aag aac gtg gcc aag cgg cgg ggg ctc ttc cac tcc gac ggc gag 878
Phe Lys Asn Val Ala Lys Arg Arg Gly Leu Phe His Ser Asp Gly Glu
                260                 265                 270

ctg ctc acc aac ggc ttc acc cgc gcc tac gtg cag cgc cac gcc ggc 926

Leu Leu Thr Asn Gly Phe Thr Arg Ala Tyr Val Gln Arg His Ala Gly
            275                 280                 285

ggc ggc tac aag gac gag ttc ttc gcc gac ttc gcc gcc tcc atg gtc      974
Gly Gly Tyr Lys Asp Glu Phe Phe Ala Asp Phe Ala Ala Ser Met Val
        290                 295                 300

aag atg ggc ggc gtc gaa gtg ctc acc ggc agc cag ggc gag atc agg      1022
Lys Met Gly Gly Val Glu Val Leu Thr Gly Ser Gln Gly Glu Ile Arg
    305                 310                 315

aag aag tgc aac gtg gtt aac taa tcatctctct taattatcgt catatatcat    1076
Lys Lys Cys Asn Val Val Asn
320                 325

cagctgctcg ccgagactgt gattatggat ttcagctttt gattccggcc gtgaccatct 1136

tataattaat ttccatgtag atcattatcg taagatttac ctgtcccttc tttttttttc 1196

ttgatttcaa tcacttcatt aattaattgt tttgtttata tagcgagcca tgcatgtatg 1256

taacaaagtt ttctccaagt acgtgctcat catgatcata tgaataaact tcattttttt 1316

tcaccttgc                                                          1325

<210> 8
<211> 326
<212> PRT
<213> Oryza sativa
<223> r0317

<400> 8
Met Ala Ser Ser Arg Val Ile Leu Ala Leu Leu Leu Ala Ala Ala Ala
 1               5                  10                  15

Val Met Ala Ser Ser Ala Gln Leu Asp Glu Lys Phe Tyr Ser Asn Ser
            20                  25                  30

Cys Pro Ser Val Glu Ala Val Val Arg Lys Glu Met Val Arg Ala Leu
        35                  40                  45

Gly Arg Ala Pro Ser Leu Ala Gly Pro Leu Leu Arg Met His Phe His
    50                  55                  60

Asp Cys Phe Val Arg Gly Cys Asp Gly Ser Val Leu Leu Asp Ser Ala
65                  70                  75                  80

Gly Asn Ser Thr Ala Glu Lys Asp Ala Thr Pro Asn Gln Thr Leu Arg
                85                  90                  95

Gly Phe Gly Phe Val Glu Arg Val Lys Ala Ala Val Glu Lys Ala Cys
            100                 105                 110

Pro Gly Thr Val Ser Cys Ala Asp Val Leu Ala Leu Met Ala Arg Asp
        115                 120                 125

Ala Val Trp Leu Ser Lys Gly Pro Phe Trp Ala Val Pro Leu Gly Arg
    130                 135                 140

Arg Asp Gly Arg Val Ser Ile Ala Asn Glu Thr Asp Gln Leu Pro Pro
145                 150                 155                 160

Pro Thr Ala Asn Phe Thr Glu Leu Thr Gln Met Phe Ala Ala Lys Asn

165                 170                 175

Leu Asp Leu Lys Asp Leu Val Val Leu Ser Ala Gly His Thr Ile Gly
            180                 185                 190

Thr Ser His Cys Phe Ser Phe Thr Asp Arg Leu Tyr Asn Phe Thr Gly
        195                 200                 205

Leu Asp Asn Ala His Asp Ile Asp Pro Thr Leu Glu Leu Gln Tyr Met
    210                 215                 220

Ala Arg Leu Arg Ser Lys Cys Thr Ser Leu Gln Asp Asn Thr Thr Leu
225                 230                 235                 240

Val Glu Met Asp Pro Gly Ser Phe Lys Thr Phe Asp Leu Gly Tyr Phe
                245                 250                 255

Lys Asn Val Ala Lys Arg Arg Gly Leu Phe His Ser Asp Gly Glu Leu
            260                 265                 270

Leu Thr Asn Gly Phe Thr Arg Ala Tyr Val Gln Arg His Ala Gly Gly
        275                 280                 285

Gly Tyr Lys Asp Glu Phe Phe Ala Asp Phe Ala Ala Ser Met Val Lys
    290                 295                 300

Met Gly Gly Val Glu Val Leu Thr Gly Ser Gln Gly Glu Ile Arg Lys
305                 310                 315                 320

Lys Cys Asn Val Val Asn
                325

<210> 9
<211> 1317
<212> DNA
<213> Oryza sativa

<220>
<221> CDS
<222> (71)..(1078)

<220>
<223> s13316

<400> 9
ctcaatcgtg tgaagaagct agagcataaa ctgcaactga taggaagcta gctagctagc 60

aggaggatca atg gcg tcg tct ccg acg atg ttg gtg gtg atg tgt agt     109
           Met Ala Ser Ser Pro Thr Met Leu Val Val Met Cys Ser
           1               5                   10

agc ttg gcc atg gcg gtg atc ctg tcg tcg agc tcg ccg gcg atg gcg     157
Ser Leu Ala Met Ala Val Ile Leu Ser Ser Ser Ser Pro Ala Met Ala
    15                  20                  25

cag ctg gac gtg ggg ttc tac agc aag acg tgc ccc aag gtg gag gag     205
Gln Leu Asp Val Gly Phe Tyr Ser Lys Thr Cys Pro Lys Val Glu Glu
30                  35                  40                  45

atc gtg cgg gag gag atg atc agg atc ctc gcc gtc gcc ccc acc ctc     253
Ile Val Arg Glu Glu Met Ile Arg Ile Leu Ala Val Ala Pro Thr Leu
                50                  55                  60

gcc ggc cct ctc ctc cgc ctc cat ttc cac gac tgc ttc gtc agg ggt     301

Ala Gly Pro Leu Leu Arg Leu His Phe His Asp Cys Phe Val Arg Gly
            65                  70                  75

tgc gac ggg tcg gtg ctg atc gac tcg acg gcg agc aac acg gcg gag     349
Cys Asp Gly Ser Val Leu Ile Asp Ser Thr Ala Ser Asn Thr Ala Glu
        80                  85                  90

aag gac gcg ccg ccg aac cag acg ctg cga ggg ttc ggc tcc gtg cag     397
Lys Asp Ala Pro Pro Asn Gln Thr Leu Arg Gly Phe Gly Ser Val Gln
    95                  100                 105

cgg atc aag gcg agg ctc gac gcc gcc tgc ccg ggc acc gtc tcc tgc     445
Arg Ile Lys Ala Arg Leu Asp Ala Ala Cys Pro Gly Thr Val Ser Cys
110                 115                 120                 125

gcc gac gtg ctc gcg ctc atg gcc cgc gac gcc gtc gcc ctc tcc ggc     493
Ala Asp Val Leu Ala Leu Met Ala Arg Asp Ala Val Ala Leu Ser Gly
                130                 135                 140

ggc ccc cgc tgg gcc gtg ccc ctc ggc cgg cga gac ggc cgc gtc tcc     541
Gly Pro Arg Trp Ala Val Pro Leu Gly Arg Arg Asp Gly Arg Val Ser
            145                 150                 155

gcc gcc aac gat acc acc acc cag ctg ccg ccg ccc acc gcc aac atc     589
Ala Ala Asn Asp Thr Thr Thr Gln Leu Pro Pro Pro Thr Ala Asn Ile
        160                 165                 170

acg cag ctc gcc cgg atg ttc gcc gcc aag ggc ctc gac atg aaa gac     637
Thr Gln Leu Ala Arg Met Phe Ala Ala Lys Gly Leu Asp Met Lys Asp
    175                 180                 185

ctc gtc gtg ctc tcc ggc ggc cac acg ctc ggc acg gcg cac tgc tcg     685
Leu Val Val Leu Ser Gly Gly His Thr Leu Gly Thr Ala His Cys Ser
190                 195                 200                 205

gcg ttc acg gac agg ctc tac aac ttc acc ggc gcc aac aac gcc ggc     733
Ala Phe Thr Asp Arg Leu Tyr Asn Phe Thr Gly Ala Asn Asn Ala Gly
                210                 215                 220

gat gtc gac ccc gcc ttg gac cgg agt tat cta gcg cgg ctc cgg tcg     781
Asp Val Asp Pro Ala Leu Asp Arg Ser Tyr Leu Ala Arg Leu Arg Ser
            225                 230                 235

cgg tgc gcc agc ctc gcc ggc gac aac acg acg ctg gcg gag atg gac     829
Arg Cys Ala Ser Leu Ala Gly Asp Asn Thr Thr Leu Ala Glu Met Asp
        240                 245                 250

ccc ggg agc ttc ctc acc ttc gac gcc ggc tac tac cgg ctg gtg gcg     877
Pro Gly Ser Phe Leu Thr Phe Asp Ala Gly Tyr Tyr Arg Leu Val Ala
    255                 260                 265

agg cgg cgg ggg ctc ttc cac tcc gac tcg tcg ctg cta gac gac gcg     925
Arg Arg Arg Gly Leu Phe His Ser Asp Ser Ser Leu Leu Asp Asp Ala
270                 275                 280                 285

ttc acc gcc ggc tac gtg cgg cgg cag gcc acc ggc atg tac gcc gcc     973
Phe Thr Ala Gly Tyr Val Arg Arg Gln Ala Thr Gly Met Tyr Ala Ala
                290                 295                 300

gag ttc ttc agg gat ttt gcc gag tcg atg gtc aag atg ggc ggc gtc     1021
Glu Phe Phe Arg Asp Phe Ala Glu Ser Met Val Lys Met Gly Gly Val
            305                 310                 315

ggc gtg ctc acc ggc ggc gaa ggc gag atc agg aag aag tgc tac gtc     1069

Gly Val Leu Thr Gly Gly Glu Gly Glu Ile Arg Lys Lys Cys Tyr Val
        320                 325                 330

atc aac taa taattaatct acgtattata gcttaattaa gcaattaagc          1118
Ile Asn
    335

tcgtaattat gtgtcttaat ttttcctgct gattattgat taagtgttcc ctaattaatc 1178

atgcgtgtaa ctgatgtatg tgtagagtgg tggcgacata tcgatctata ctagttttac 1238

ttggattact ttgtttgtta attgtattat tatgcaatgt aactaagaaa ttaatcaaat 1298

aatgaatcaa gtattgcac                                              1317

<210> 10
<211> 335
<212> PRT
<213> Oryza sativa
<223> s13316

<400> 10
Met Ala Ser Ser Pro Thr Met Leu Val Val Met Cys Ser Ser Leu Ala
  1               5                  10                  15

Met Ala Val Ile Leu Ser Ser Ser Ser Pro Ala Met Ala Gln Leu Asp
             20                  25                  30

Val Gly Phe Tyr Ser Lys Thr Cys Pro Lys Val Glu Glu Ile Val Arg
         35                  40                  45

Glu Glu Met Ile Arg Ile Leu Ala Val Ala Pro Thr Leu Ala Gly Pro
     50                  55                  60

Leu Leu Arg Leu His Phe His Asp Cys Phe Val Arg Gly Cys Asp Gly
 65                  70                  75                  80

Ser Val Leu Ile Asp Ser Thr Ala Ser Asn Thr Ala Glu Lys Asp Ala
                 85                  90                  95

Pro Pro Asn Gln Thr Leu Arg Gly Phe Gly Ser Val Gln Arg Ile Lys
            100                 105                 110

Ala Arg Leu Asp Ala Ala Cys Pro Gly Thr Val Ser Cys Ala Asp Val
        115                 120                 125

Leu Ala Leu Met Ala Arg Asp Ala Val Ala Leu Ser Gly Gly Pro Arg
    130                 135                 140

Trp Ala Val Pro Leu Gly Arg Arg Asp Gly Arg Val Ser Ala Ala Asn
145                 150                 155                 160

Asp Thr Thr Thr Gln Leu Pro Pro Pro Thr Ala Asn Ile Thr Gln Leu
                165                 170                 175

Ala Arg Met Phe Ala Ala Lys Gly Leu Asp Met Lys Asp Leu Val Val
            180                 185                 190

Leu Ser Gly Gly His Thr Leu Gly Thr Ala His Cys Ser Ala Phe Thr
        195                 200                 205

Asp Arg Leu Tyr Asn Phe Thr Gly Ala Asn Asn Ala Gly Asp Val Asp
    210                 215                 220

Pro Ala Leu Asp Arg Ser Tyr Leu Ala Arg Leu Arg Ser Arg Cys Ala
225                 230                 235                 240

Ser Leu Ala Gly Asp Asn Thr Thr Leu Ala Glu Met Asp Pro Gly Ser
                245                 250                 255

Phe Leu Thr Phe Asp Ala Gly Tyr Tyr Arg Leu Val Ala Arg Arg Arg
            260                 265                 270

Gly Leu Phe His Ser Asp Ser Ser Leu Leu Asp Asp Ala Phe Thr Ala
        275                 280                 285

Gly Tyr Val Arg Arg Gln Ala Thr Gly Met Tyr Ala Ala Glu Phe Phe
    290                 295                 300

Arg Asp Phe Ala Glu Ser Met Val Lys Met Gly Gly Val Gly Val Leu
305                 310                 315                 320

Thr Gly Gly Glu Gly Glu Ile Arg Lys Lys Cys Tyr Val Ile Asn
                325                 330                 335

<210> 11
<211> 1256
<212> DNA
<213> Oryza sativa

<220>
<221> CDS
<222> (134)..(1108)

<220>
<223> r2151

<400> 11
caaacgcagg cacacaaaca cagagagcag cccaaaccga ccaaaatcac tgcagagttc 60

aggcacttct tcagatagtt ctccccctct gtttctttct ctctttattt ctttctcgag 120

ttcagtttga gag atg gat ttg gcg tgg tgg ttc gcg gtg gcc gtg gtg     169
               Met Asp Leu Ala Trp Trp Phe Ala Val Ala Val Val
               1               5                   10

gtg tgc ggc ctt gtg ggg ggc ggc agc gcc ggg ctg ctg gag acc aac     217
Val Cys Gly Leu Val Gly Gly Gly Ser Ala Gly Leu Leu Glu Thr Asn
        15                  20                  25

ccg ggt ttg gcg tac aac ttc tac cag aag tcg tgc ccc aac gtg gac     265
Pro Gly Leu Ala Tyr Asn Phe Tyr Gln Lys Ser Cys Pro Asn Val Asp
    30                  35                  40

tcc atc gtc cgc agc gtc acc tgg gcg cag gtc gcc gcc aac ccg gcg     313
Ser Ile Val Arg Ser Val Thr Trp Ala Gln Val Ala Ala Asn Pro Ala
45                  50                  55                  60

ctc ccc ggc cgc ctc ctc cgc ctc cac ttc cat gac tgc ttc gtc cag     361
Leu Pro Gly Arg Leu Leu Arg Leu His Phe His Asp Cys Phe Val Gln
                65                  70                  75

ggg tgc gac gcg tcg atc ttg ctg gac aac gcc ggg agc gag aag acg     409
Gly Cys Asp Ala Ser Ile Leu Leu Asp Asn Ala Gly Ser Glu Lys Thr
            80                  85                  90

gcg ggg ccg aac cta tcg gtg ggg gga tac gag gtg atc gac gcc atc     457

Ala Gly Pro Asn Leu Ser Val Gly Gly Tyr Glu Val Ile Asp Ala Ile
        95                  100                 105

aag acg cag ctg gag cag gcg tgc ccc ggg gtg gtg tcg tgc gcg gac     505
Lys Thr Gln Leu Glu Gln Ala Cys Pro Gly Val Val Ser Cys Ala Asp
    110                 115                 120

atc gtg gcg ctc gcc gcg cgc gac gcc gtg tcg tac cag ttc aag gcg     553
Ile Val Ala Leu Ala Ala Arg Asp Ala Val Ser Tyr Gln Phe Lys Ala
125                 130                 135                 140

tcg ctg tgg cag gtg gag acc ggg agg cgc gac ggg ccc gtg tct ctg     601
Ser Leu Trp Gln Val Glu Thr Gly Arg Arg Asp Gly Pro Val Ser Leu
                145                 150                 155

gcg tcc aac acc ggc gcg ctg ccg tcg ccg ttc gcc ggg ttc agc acg     649
Ala Ser Asn Thr Gly Ala Leu Pro Ser Pro Phe Ala Gly Phe Ser Thr
            160                 165                 170

ctc ctc cag agc ttc gcc aac cgc ggg ctc aac ctg acc gac ctc gtc     697
Leu Leu Gln Ser Phe Ala Asn Arg Gly Leu Asn Leu Thr Asp Leu Val
        175                 180                 185

gcg ctc tcc ggc gcg cac acc atc ggc aag gcc agc tgc tcc agc gtc     745
Ala Leu Ser Gly Ala His Thr Ile Gly Lys Ala Ser Cys Ser Ser Val
    190                 195                 200

acg ccg cgg ctg tac cag ggg aac acc acc tcc ctc gac ccg ctg ctc     793
Thr Pro Arg Leu Tyr Gln Gly Asn Thr Thr Ser Leu Asp Pro Leu Leu
205                 210                 215                 220

gac tcc gcc tac gcc aag gcg ctc atg tcg tcg tgc ccc aac ccg tcg     841
Asp Ser Ala Tyr Ala Lys Ala Leu Met Ser Ser Cys Pro Asn Pro Ser
                225                 230                 235

ccg tcg tcg tcc acc atc gac ctc gac gtc gcc acg ccg ctc aag ttc     889
Pro Ser Ser Ser Thr Ile Asp Leu Asp Val Ala Thr Pro Leu Lys Phe
            240                 245                 250

gac agc ggt tac tac gcc aac ctg cag aag aag cag ggc gcg ctg gcg     937
Asp Ser Gly Tyr Tyr Ala Asn Leu Gln Lys Lys Gln Gly Ala Leu Ala
        255                 260                 265

tcc gac gcc gcg ctc acc cag aac gcc gcc gcg gcg cag atg gtg gca     985
Ser Asp Ala Ala Leu Thr Gln Asn Ala Ala Ala Ala Gln Met Val Ala
    270                 275                 280

gac ctc acc aac ccg atc aag ttc tac gcc gcg ttc tcc atg tcc atg     1033
Asp Leu Thr Asn Pro Ile Lys Phe Tyr Ala Ala Phe Ser Met Ser Met
285                 290                 295                 300

aag aag atg gga cgc atc gac gtg ctc acc ggc agc aaa ggg aat atc     1081
Lys Lys Met Gly Arg Ile Asp Val Leu Thr Gly Ser Lys Gly Asn Ile
                305                 310                 315

agg aag cag tgc cgc tcc gcc tcc tga atcctgacga tcaccataca           1128
Arg Lys Gln Cys Arg Ser Ala Ser
            320

aacatatttt cttcttggtt tcttgaattt ttttttcttc ttctcctctc tcgtttcatc 1188

ggcgctgatc aaaatatatt gtgcgccatg atgattaatt aaggtcccaa gatctgtatt 1248

aatttgtt                                                          1256

<210> 12
<211> 324
<212> PRT
<213> Oryza sativa
<223> r2151

<400> 12
Met Asp Leu Ala Trp Trp Phe Ala Val Ala Val Val Val Cys Gly Leu
1               5                   10                  15

Val Gly Gly Gly Ser Ala Gly Leu Leu Glu Thr Asn Pro Gly Leu Ala
            20                  25                  30

Tyr Asn Phe Tyr Gln Lys Ser Cys Pro Asn Val Asp Ser Ile Val Arg
        35                  40                  45

Ser Val Thr Trp Ala Gln Val Ala Ala Asn Pro Ala Leu Pro Gly Arg
    50                  55                  60

Leu Leu Arg Leu His Phe His Asp Cys Phe Val Gln Gly Cys Asp Ala
65                  70                  75                  80

Ser Ile Leu Leu Asp Asn Ala Gly Ser Glu Lys Thr Ala Gly Pro Asn
                85                  90                  95

Leu Ser Val Gly Gly Tyr Glu Val Ile Asp Ala Ile Lys Thr Gln Leu
            100                 105                 110

Glu Gln Ala Cys Pro Gly Val Val Ser Cys Ala Asp Ile Val Ala Leu
        115                 120                 125

Ala Ala Arg Asp Ala Val Ser Tyr Gln Phe Lys Ala Ser Leu Trp Gln
    130                 135                 140

Val Glu Thr Gly Arg Arg Asp Gly Pro Val Ser Leu Ala Ser Asn Thr
145                 150                 155                 160

Gly Ala Leu Pro Ser Pro Phe Ala Gly Phe Ser Thr Leu Leu Gln Ser
                165                 170                 175

Phe Ala Asn Arg Gly Leu Asn Leu Thr Asp Leu Val Ala Leu Ser Gly
            180                 185                 190

Ala His Thr Ile Gly Lys Ala Ser Cys Ser Ser Val Thr Pro Arg Leu
        195                 200                 205

Tyr Gln Gly Asn Thr Thr Ser Leu Asp Pro Leu Leu Asp Ser Ala Tyr
    210                 215                 220

Ala Lys Ala Leu Met Ser Ser Cys Pro Asn Pro Ser Pro Ser Ser Ser
225                 230                 235                 240

Thr Ile Asp Leu Asp Val Ala Thr Pro Leu Lys Phe Asp Ser Gly Tyr
                245                 250                 255

Tyr Ala Asn Leu Gln Lys Lys Gln Gly Ala Leu Ala Ser Asp Ala Ala
            260                 265                 270

Leu Thr Gln Asn Ala Ala Ala Ala Gln Met Val Ala Asp Leu Thr Asn
        275                 280                 285

Pro Ile Lys Phe Tyr Ala Ala Phe Ser Met Ser Met Lys Lys Met Gly

290                 295                 300

Arg Ile Asp Val Leu Thr Gly Ser Lys Gly Asn Ile Arg Lys Gln Cys
305                 310                 315                 320

Arg Ser Ala Ser

<210> 13
<211> 1156
<212> DNA
<213> Oryza sativa

<220>
<221> CDS
<222> (75)..(1058)

<220>
<223> s4235

<400> 13
   gcgtgacgcg agaggctgag aggagcattt tggcccttgg tttctttcgg ttcttctgct 60
   cttttttgct ggcg atg ggg tgg tcg tcg tca agg gct atg ctc gtg gcg   110
                   Met Gly Trp Ser Ser Ser Arg Ala Met Leu Val Ala
                   1               5                   10
   cgc gct gtg gcc ttg gcg gtg gtg ttc ctc gcg gcg gag gct cag ctg    158
   Arg Ala Val Ala Leu Ala Val Val Phe Leu Ala Ala Glu Ala Gln Leu
           15                  20                  25
   tcg ccg ggg tac tac aac gcg acg tgc ccc ggg gtg gtg tcc atc gtg    206
   Ser Pro Gly Tyr Tyr Asn Ala Thr Cys Pro Gly Val Val Ser Ile Val
       30                  35                  40
   cgc cgc ggc atg gcg cag gca gtg cag aag gag tcg cgc atg ggc gcc    254
   Arg Arg Gly Met Ala Gln Ala Val Gln Lys Glu Ser Arg Met Gly Ala
   45                  50                  55                  60
   tcc atc ctc cgc ctc ttc ttc cac gac tgc ttc gtc aac ggg tgc gac    302
   Ser Ile Leu Arg Leu Phe Phe His Asp Cys Phe Val Asn Gly Cys Asp
                   65                  70                  75
   gcc tcc atc ttg ctc gac gac acg gcg aac ttc acc ggg gag aag aac    350
   Ala Ser Ile Leu Leu Asp Asp Thr Ala Asn Phe Thr Gly Glu Lys Asn
               80                  85                  90
   gcc ggg ccg aac gcc aac tcg gtg cgc ggg tac gag gtc atc gac gcc    398
   Ala Gly Pro Asn Ala Asn Ser Val Arg Gly Tyr Glu Val Ile Asp Ala
           95                  100                 105
   atc aag gcg cag ctc gag gcc tcc tgc aag gcc acc gtc tcc tgc gcc    446
   Ile Lys Ala Gln Leu Glu Ala Ser Cys Lys Ala Thr Val Ser Cys Ala
       110                 115                 120
   gac atc atc acg ctc gcc gcg cgc gac gcc gtc aac ctg ctc ggg ggc    494
   Asp Ile Ile Thr Leu Ala Ala Arg Asp Ala Val Asn Leu Leu Gly Gly
   125                 130                 135                 140
   ccg aac tgg acg gtg ccg ctg ggg cgg cgt gac gcg cgc acg acg agc    542
   Pro Asn Trp Thr Val Pro Leu Gly Arg Arg Asp Ala Arg Thr Thr Ser
                   145                 150                 155
   cag agc gcg gcg aac acc aac ctg ccg ccg ccc ggc gcg agc ctc gcg    590
   Gln Ser Ala Ala Asn Thr Asn Leu Pro Pro Pro Gly Ala Ser Leu Ala
   160                 165                 170
   tcg ctc ctg tcg atg ttc agc gcc aag ggc ctc gac gcg cgg gac ctc     638
   Ser Leu Leu Ser Met Phe Ser Ala Lys Gly Leu Asp Ala Arg Asp Leu
           175                 180                 185
   acc gcg ctg tcg ggc gcg cac acc gtc ggg tgg gcg cgc tgc tcc acc     686
   Thr Ala Leu Ser Gly Ala His Thr Val Gly Trp Ala Arg Cys Ser Thr
       190                 195                 200
   ttc cgc acg cac atc tac aac gac acc ggc gtg aac gcc acc ttc gcc     734
   Phe Arg Thr His Ile Tyr Asn Asp Thr Gly Val Asn Ala Thr Phe Ala
   205                 210                 215                 220
   tcg cag ctg cgc acc aag tcc tgc ccg acc acc ggc ggc gac ggc aac     782
   Ser Gln Leu Arg Thr Lys Ser Cys Pro Thr Thr Gly Gly Asp Gly Asn
                   225                 230                 235
   ctc gcg ccg ctc gag ctg cag gcg ccc aac acc ttc gac aac gcc tac     830
   Leu Ala Pro Leu Glu Leu Gln Ala Pro Asn Thr Phe Asp Asn Ala Tyr
               240                 245                 250
   ttc acg gac ctc ctc agc cgc cgc gtc ctg ctg cgc tcc gac cag gag     878
   Phe Thr Asp Leu Leu Ser Arg Arg Val Leu Leu Arg Ser Asp Gln Glu
           255                 260                 265
   ctc ttc ggc agc ggc gcc ggc aat ggc acc acg gac gcg ttc gtg cgc     926
   Leu Phe Gly Ser Gly Ala Gly Asn Gly Thr Thr Asp Ala Phe Val Arg
       270                 275                 280
   gcg tac gcc gcc aac gcg acg acg ttc gcg gcg gac ttc gcc gcc gcg     974
   Ala Tyr Ala Ala Asn Ala Thr Thr Phe Ala Ala Asp Phe Ala Ala Ala
   285                 290                 295                 300
   atg gtg agg ctg ggc aac ctg agc ccg ctc acc ggg aag aac ggc gag     1022
   Met Val Arg Leu Gly Asn Leu Ser Pro Leu Thr Gly Lys Asn Gly Glu
                   305                 310                 315
   gta cgg atc aac tgc cgg cga gtg aac tca tca tga acatgaaatg          1068
   Val Arg Ile Asn Cys Arg Arg Val Asn Ser Ser
               320                 325
   gattcttgat gtacgcttcg tgaacgccat aaacttatta tcaaatcttg tttttaccaa 1128
   aagcaaatgg aaaaaatgct gttcttgc                                    1156

<210> 14
<211> 327
<212> PRT
<213> Oryza sativa
<223> s4235

<400> 14
Met Gly Trp Ser Ser Ser Arg Ala Met Leu Val Ala Arg Ala Val Ala
1               5                   10                  15

Leu Ala Val Val Phe Leu Ala Ala Glu Ala Gln Leu Ser Pro Gly Tyr
            20                  25                  30

Tyr Asn Ala Thr Cys Pro Gly Val Val Ser Ile Val Arg Arg Gly Met
        35                  40                  45

Ala Gln Ala Val Gln Lys Glu Ser Arg Met Gly Ala Ser Ile Leu Arg

50                  55                  60

Leu Phe Phe His Asp Cys Phe Val Asn Gly Cys Asp Ala Ser Ile Leu
65                  70                  75                  80

Leu Asp Asp Thr Ala Asn Phe Thr Gly Glu Lys Asn Ala Gly Pro Asn
                85                  90                  95

Ala Asn Ser Val Arg Gly Tyr Glu Val Ile Asp Ala Ile Lys Ala Gln
            100                 105                 110

Leu Glu Ala Ser Cys Lys Ala Thr Val Ser Cys Ala Asp Ile Ile Thr
        115                 120                 125

Leu Ala Ala Arg Asp Ala Val Asn Leu Leu Gly Gly Pro Asn Trp Thr
    130                 135                 140

Val Pro Leu Gly Arg Arg Asp Ala Arg Thr Thr Ser Gln Ser Ala Ala
145                 150                 155                 160

Asn Thr Asn Leu Pro Pro Pro Gly Ala Ser Leu Ala Ser Leu Leu Ser
                165                 170                 175

Met Phe Ser Ala Lys Gly Leu Asp Ala Arg Asp Leu Thr Ala Leu Ser
            180                 185                 190

Gly Ala His Thr Val Gly Trp Ala Arg Cys Ser Thr Phe Arg Thr His
        195                 200                 205

Ile Tyr Asn Asp Thr Gly Val Asn Ala Thr Phe Ala Ser Gln Leu Arg
    210                 215                 220

Thr Lys Ser Cys Pro Thr Thr Gly Gly Asp Gly Asn Leu Ala Pro Leu
225                 230                 235                 240

Glu Leu Gln Ala Pro Asn Thr Phe Asp Asn Ala Tyr Phe Thr Asp Leu
                245                 250                 255

Leu Ser Arg Arg Val Leu Leu Arg Ser Asp Gln Glu Leu Phe Gly Ser
            260                 265                 270

Gly Ala Gly Asn Gly Thr Thr Asp Ala Phe Val Arg Ala Tyr Ala Ala
        275                 280                 285

Asn Ala Thr Thr Phe Ala Ala Asp Phe Ala Ala Ala Met Val Arg Leu
    290                 295                 300

Gly Asn Leu Ser Pro Leu Thr Gly Lys Asn Gly Glu Val Arg Ile Asn
305                 310                 315                 320

Cys Arg Arg Val Asn Ser Ser
                325

<210> 15
<211> 1370
<212> DNA
<213> Oryza sativa

<220>
<221> CDS
<222> (137)..(1147)

<220>

<223> c62847

<400> 15
ggcaaggaga gagagagaag agagagagag agagagagag agagtaatta aggctgggag 60

gataggcagc agcagcagcg agagggaaac gagcgagcga gcttagctgg tgcttgccta 120

taggtagcta agcaaa atg ggg cag agg agg agg tcg ggg ccg cgg cgt cag 172
                  Met Gly Gln Arg Arg Arg Ser Gly Pro Arg Arg Gln
                  1               5                   10

agc cag agc gtg gtg gtg gtg gtg gtc gcc gtg ttg ctg gcg acg gcg 220
Ser Gln Ser Val Val Val Val Val Val Ala Val Leu Leu Ala Thr Ala
        15                  20                  25

tcc tgc gcg gcg gcg cag ctg agc cag agc tac tac gcg tcg acg tgc 268
Ser Cys Ala Ala Ala Gln Leu Ser Gln Ser Tyr Tyr Ala Ser Thr Cys
    30                  35                  40

ccc aac gtg gag acg ctc gtc cgc ggc gcc gtc acg cag aag ctc aag 316
Pro Asn Val Glu Thr Leu Val Arg Gly Ala Val Thr Gln Lys Leu Lys
45                  50                  55                  60

gag acc ttc aac gcc gcg cct ggg acg ctc cgc ctc ttc ttc cac gac 364
Glu Thr Phe Asn Ala Ala Pro Gly Thr Leu Arg Leu Phe Phe His Asp
                65                  70                  75

tgc ttc gtc agg ggg tgc gac gcg tcg gtg ctg atc gcg ggg ccg gac 412
Cys Phe Val Arg Gly Cys Asp Ala Ser Val Leu Ile Ala Gly Pro Asp
            80                  85                  90

gac gag cac agc gcg ggc gcg gac acg acg ctg tcg ccg gac gcg ctg 460
Asp Glu His Ser Ala Gly Ala Asp Thr Thr Leu Ser Pro Asp Ala Leu
        95                  100                 105

gac ctc atc acc cgc gcc aag gcc gcc gtc gac gcc gac gcc cag tgc 508
Asp Leu Ile Thr Arg Ala Lys Ala Ala Val Asp Ala Asp Ala Gln Cys
    110                 115                 120

gcc aac aag gtc tcc tgc gcc gac atc ctc gcc ctc gcc gcc cgc gac 556
Ala Asn Lys Val Ser Cys Ala Asp Ile Leu Ala Leu Ala Ala Arg Asp
125                 130                 135                 140

gtc gtc tcc cag gca gga gga ccc tac tac cag gtg gag ctg ggg cgg 604
Val Val Ser Gln Ala Gly Gly Pro Tyr Tyr Gln Val Glu Leu Gly Arg
                145                 150                 155

ctt gac ggc aag gtc ggg acg cgc gcc gtg gtg aag cac agc ctc ccc 652
Leu Asp Gly Lys Val Gly Thr Arg Ala Val Val Lys His Ser Leu Pro
            160                 165                 170

ggc gcc gcc ttc gac ctc gac cag ctc aac aag ctc ttc gcc acc aac 700
Gly Ala Ala Phe Asp Leu Asp Gln Leu Asn Lys Leu Phe Ala Thr Asn
        175                 180                 185

ggc ctc acc cag acc gac atg atc gcc ctc tca gga ggg cac acg ata 748
Gly Leu Thr Gln Thr Asp Met Ile Ala Leu Ser Gly Gly His Thr Ile
    190                 195                 200

ggg gtg acg cac tgc gac aag ttc gtg cgg cgg ctg tac cag ttc aag 796
Gly Val Thr His Cys Asp Lys Phe Val Arg Arg Leu Tyr Gln Phe Lys
205                 210                 215                 220

ggg gcg gcg ccg cag tac agc ccg ccg atg aac ctg gcg ttc ctg cgg 844

Gly Ala Ala Pro Gln Tyr Ser Pro Pro Met Asn Leu Ala Phe Leu Arg
                225                 230                 235

cag atg agg cag acg tgc ccg ctc agc tac agc ccg acc acc gtc gcc     892
Gln Met Arg Gln Thr Cys Pro Leu Ser Tyr Ser Pro Thr Thr Val Ala
            240                 245                 250

atg ctc gac gcc gtc tcg ccc aac aag ttc gac aat ggc tac ttc cag     940
Met Leu Asp Ala Val Ser Pro Asn Lys Phe Asp Asn Gly Tyr Phe Gln
        255                 260                 265

acg ctg cag cag ctc aag ggc ctc ctc gcc tcc gac cag gtg ctc ttc     988
Thr Leu Gln Gln Leu Lys Gly Leu Leu Ala Ser Asp Gln Val Leu Phe
    270                 275                 280

gcc gac cgc cgc tcg cgc gcc acc gtc aac tac ttc gcc gcc aac cag     1036
Ala Asp Arg Arg Ser Arg Ala Thr Val Asn Tyr Phe Ala Ala Asn Gln
285                 290                 295                 300

acc gcc ttc ttc gac gcc ttc gtc gcc gcc atc acc aag ctc ggc cgc     1084
Thr Ala Phe Phe Asp Ala Phe Val Ala Ala Ile Thr Lys Leu Gly Arg
                305                 310                 315

gtc ggc gtc aag acg gcc gcc ggc tcc gac gcc gag atc cgg cga gtc     1132
Val Gly Val Lys Thr Ala Ala Gly Ser Asp Ala Glu Ile Arg Arg Val
            320                 325                 330

tgc acc aag gtc aac taataacgta gagcagtata gctcacattt gggtggtaga     1187
Cys Thr Lys Val Asn
        335

gagggtaaat tggtgaattc ttctctgtgt ctctcttctt cttcttcctc ttcttctcca 1247

tgatgatctt cgattctcac tgtccatgtt cacaagtcca ccgcgcacag ttgttgtatt 1307

tgttcccgcc aattcttttc ttgattctta taatcataaa aagtgtcata cattaattag 1367

gat                                                               1370


<210> 16
<211> 337
<212> PRT
<213> Oryza sativa

<220>
<223> c62847

<400> 16
Met Gly Gln Arg Arg Arg Ser Gly Pro Arg Arg Gln Ser Gln Ser Val
  1               5                  10                  15

Val Val Val Val Val Ala Val Leu Leu Ala Thr Ala Ser Cys Ala Ala
             20                  25                  30

Ala Gln Leu Ser Gln Ser Tyr Tyr Ala Ser Thr Cys Pro Asn Val Glu
         35                  40                  45

Thr Leu Val Arg Gly Ala Val Thr Gln Lys Leu Lys Glu Thr Phe Asn
     50                  55                  60

Ala Ala Pro Gly Thr Leu Arg Leu Phe Phe His Asp Cys Phe Val Arg
 65                  70                  75                  80

Gly Cys Asp Ala Ser Val Leu Ile Ala Gly Pro Asp Asp Glu His Ser
                85                  90                  95

Ala Gly Ala Asp Thr Thr Leu Ser Pro Asp Ala Leu Asp Leu Ile Thr
            100                 105                 110

Arg Ala Lys Ala Ala Val Asp Ala Asp Ala Gln Cys Ala Asn Lys Val
        115                 120                 125

Ser Cys Ala Asp Ile Leu Ala Leu Ala Ala Arg Asp Val Val Ser Gln
    130                 135                 140

Ala Gly Gly Pro Tyr Tyr Gln Val Glu Leu Gly Arg Leu Asp Gly Lys
145                 150                 155                 160

Val Gly Thr Arg Ala Val Val Lys His Ser Leu Pro Gly Ala Ala Phe
                165                 170                 175

Asp Leu Asp Gln Leu Asn Lys Leu Phe Ala Thr Asn Gly Leu Thr Gln
            180                 185                 190

Thr Asp Met Ile Ala Leu Ser Gly Gly His Thr Ile Gly Val Thr His
        195                 200                 205

Cys Asp Lys Phe Val Arg Arg Leu Tyr Gln Phe Lys Gly Ala Ala Pro
    210                 215                 220

Gln Tyr Ser Pro Pro Met Asn Leu Ala Phe Leu Arg Gln Met Arg Gln
225                 230                 235                 240

Thr Cys Pro Leu Ser Tyr Ser Pro Thr Thr Val Ala Met Leu Asp Ala
                245                 250                 255

Val Ser Pro Asn Lys Phe Asp Asn Gly Tyr Phe Gln Thr Leu Gln Gln
            260                 265                 270

Leu Lys Gly Leu Leu Ala Ser Asp Gln Val Leu Phe Ala Asp Arg Arg
        275                 280                 285

Ser Arg Ala Thr Val Asn Tyr Phe Ala Ala Asn Gln Thr Ala Phe Phe
    290                 295                 300

Asp Ala Phe Val Ala Ala Ile Thr Lys Leu Gly Arg Val Gly Val Lys
305                 310                 315                 320

Thr Ala Ala Gly Ser Asp Ala Glu Ile Arg Arg Val Cys Thr Lys Val
                325                 330                 335

Asn


<210> 17
<211> 1138
<212> DNA
<213> Oryza sativa

<220>
<221> CDS
<222> (29)..(997)

<220>
<223> r1617

<400> 17

gtagctagct aggtgagatc gaggggca atg gag ttg gtg gtg gcg gta gca    52
                              Met Glu Leu Val Val Ala Val Ala
                               1               5

ggt gca gta gtc gtg gca ctg agc ttg tgc att gga ggt gtg caa ggt    100
Gly Ala Val Val Val Ala Leu Ser Leu Cys Ile Gly Gly Val Gln Gly
     10                  15                  20

cag ctc cag gta ggg ttc tac gac caa tcg tgc ccc cag gcc gaa gtg    148
Gln Leu Gln Val Gly Phe Tyr Asp Gln Ser Cys Pro Gln Ala Glu Val
 25                  30                  35                  40

att gtg aga gac gag gtc ggc aag gcc gtg agc gcc aac gtt ggc ctc    196
Ile Val Arg Asp Glu Val Gly Lys Ala Val Ser Ala Asn Val Gly Leu
                 45                  50                  55

gcc gcc ggc ctt gtc cgg atg cac ttc cat gac tgc ttc gtc aag gga    244
Ala Ala Gly Leu Val Arg Met His Phe His Asp Cys Phe Val Lys Gly
             60                  65                  70

tgt gac gcg tcg gtt ttg ctg tat tcg acg gcg aac agc acg gcg gag    292
Cys Asp Ala Ser Val Leu Leu Tyr Ser Thr Ala Asn Ser Thr Ala Glu
         75                  80                  85

aag gac gcg ata ccg aac aag agc ctg agg ggg ttc gag gtg gtg gac    340
Lys Asp Ala Ile Pro Asn Lys Ser Leu Arg Gly Phe Glu Val Val Asp
     90                  95                 100

agc gcc aag cgg cgg ctg gag agc gcg tgc aag ggg gtg gtc tcc tgc    388
Ser Ala Lys Arg Arg Leu Glu Ser Ala Cys Lys Gly Val Val Ser Cys
105                 110                 115                 120

gcg gac ata ctc gcc ttc gcc gcc aga gac agc gtc gtg ctg gcc ggc    436
Ala Asp Ile Leu Ala Phe Ala Ala Arg Asp Ser Val Val Leu Ala Gly
                125                 130                 135

ggc acc ccg tac cgc gtt ccg gcc ggg agg agg gac ggc aac acc tcc    484
Gly Thr Pro Tyr Arg Val Pro Ala Gly Arg Arg Asp Gly Asn Thr Ser
            140                 145                 150

gtg gcg tcc gac gcg atg gcc aac ctg ccc cgt ccc acc tca gat gtg    532
Val Ala Ser Asp Ala Met Ala Asn Leu Pro Arg Pro Thr Ser Asp Val
        155                 160                 165

gcc caa ctc aca caa agt ttc gcc act cat ggg ctt tcc cag gac gac    580
Ala Gln Leu Thr Gln Ser Phe Ala Thr His Gly Leu Ser Gln Asp Asp
    170                 175                 180

atg gtc atc ctt tca ggg gcg cac acg ata ggg gtg gcg cat tgc agc    628
Met Val Ile Leu Ser Gly Ala His Thr Ile Gly Val Ala His Cys Ser
185                 190                 195                 200

tcg ttc agc tcg agg ctg tac ggg tac aac tcc agc acg ggg cag gac    676
Ser Phe Ser Ser Arg Leu Tyr Gly Tyr Asn Ser Ser Thr Gly Gln Asp
                205                 210                 215

ccg gcg ctg aac gcg gcg atg gcg tcg cgg ctg tcg cgg agt tgc ccg    724
Pro Ala Leu Asn Ala Ala Met Ala Ser Arg Leu Ser Arg Ser Cys Pro
            220                 225                 230

cag ggg agc gcc aac acg gtg gcc atg gac gac ggc agc gag aac acg    772
Gln Gly Ser Ala Asn Thr Val Ala Met Asp Asp Gly Ser Glu Asn Thr
        235                 240                 245

ttc gac acc agc tac tac cag aac ctc ctc gcc ggc cgc ggc gtc ctc 820
Phe Asp Thr Ser Tyr Tyr Gln Asn Leu Leu Ala Gly Arg Gly Val Leu
    250                 255                 260

gcc tcc gac cag acg ctc acc gcc gac aac gcc acc gcc gcg ctc gtg 868
Ala Ser Asp Gln Thr Leu Thr Ala Asp Asn Ala Thr Ala Ala Leu Val
265                 270                 275                 280

gcg cag aac gcc tac aac atg tac ctc ttc gcc acc aag ttc ggc cag 916
Ala Gln Asn Ala Tyr Asn Met Tyr Leu Phe Ala Thr Lys Phe Gly Gln
                285                 290                 295

gcc atg gtc aag atg ggc gcc atc cag gtg ctc acc ggc agc gac ggc 964
Ala Met Val Lys Met Gly Ala Ile Gln Val Leu Thr Gly Ser Asp Gly
            300                 305                 310

cag atc cgc aca aac tgc agg gtt gca aac tga gctaggcctc gcttgctgaa 1017
Gln Ile Arg Thr Asn Cys Arg Val Ala Asn
        315                 320

caacaacatt tttgaagcct ctacttgttc ctatcatcag agtttttgtt cttcttttgt 1077

ctctatgctt taatttgtca aacggcaaaa attcaatgga attacgcatt ggttttgaac 1137

t 1138

<210> 18
<211> 322
<212> PRT
<213> Oryza sativa
<223> r1617

<400> 18
Met Glu Leu Val Val Ala Val Ala Gly Ala Val Val Val Ala Leu Ser
1               5                   10                  15

Leu Cys Ile Gly Gly Val Gln Gly Gln Leu Gln Val Gly Phe Tyr Asp
            20                  25                  30

Gln Ser Cys Pro Gln Ala Glu Val Ile Val Arg Asp Glu Val Gly Lys
        35                  40                  45

Ala Val Ser Ala Asn Val Gly Leu Ala Ala Gly Leu Val Arg Met His
    50                  55                  60

Phe His Asp Cys Phe Val Lys Gly Cys Asp Ala Ser Val Leu Leu Tyr
65                  70                  75                  80

Ser Thr Ala Asn Ser Thr Ala Glu Lys Asp Ala Ile Pro Asn Lys Ser
                85                  90                  95

Leu Arg Gly Phe Glu Val Val Asp Ser Ala Lys Arg Arg Leu Glu Ser
            100                 105                 110

Ala Cys Lys Gly Val Val Ser Cys Ala Asp Ile Leu Ala Phe Ala Ala
        115                 120                 125

Arg Asp Ser Val Val Leu Ala Gly Gly Thr Pro Tyr Arg Val Pro Ala
    130                 135                 140

Gly Arg Arg Asp Gly Asn Thr Ser Val Ala Ser Asp Ala Met Ala Asn
145                 150                 155                 160

Leu Pro Arg Pro Thr Ser Asp Val Ala Gln Leu Thr Gln Ser Phe Ala
                165                 170                 175

Thr His Gly Leu Ser Gln Asp Asp Met Val Ile Leu Ser Gly Ala His
            180                 185                 190

Thr Ile Gly Val Ala His Cys Ser Ser Phe Ser Ser Arg Leu Tyr Gly
        195                 200                 205

Tyr Asn Ser Ser Thr Gly Gln Asp Pro Ala Leu Asn Ala Ala Met Ala
    210                 215                 220

Ser Arg Leu Ser Arg Ser Cys Pro Gln Gly Ser Ala Asn Thr Val Ala
225                 230                 235                 240

Met Asp Asp Gly Ser Glu Asn Thr Phe Asp Thr Ser Tyr Tyr Gln Asn
                245                 250                 255

Leu Leu Ala Gly Arg Gly Val Leu Ala Ser Asp Gln Thr Leu Thr Ala
            260                 265                 270

Asp Asn Ala Thr Ala Ala Leu Val Ala Gln Asn Ala Tyr Asn Met Tyr
        275                 280                 285

Leu Phe Ala Thr Lys Phe Gly Gln Ala Met Val Lys Met Gly Ala Ile
    290                 295                 300

Gln Val Leu Thr Gly Ser Asp Gly Gln Ile Arg Thr Asn Cys Arg Val
305                 310                 315                 320

Ala Asn

<210> 19
<211> 1158
<212> DNA
<213> Oryza sativa

<220>
<221> CDS
<222> (14)..(997)

<220>
<223> r3025

<400> 19
gagcaatatc aat atg gct cag ccg act tgg tca gca agg cgc gtc act      49
               Met Ala Gln Pro Thr Trp Ser Ala Arg Arg Val Thr
                 1               5                  10

gcc gcc ctg gtc gtg atg gtg gtg gtg gtg ctc gcg gtc gcc ggc ggg      97
Ala Ala Leu Val Val Met Val Val Val Val Leu Ala Val Ala Gly Gly
        15                  20                  25

tcg tgg gcg cag ctg tcg ccg agc ttc tac tcg tac tcg tgc ccg gga      145
Ser Trp Ala Gln Leu Ser Pro Ser Phe Tyr Ser Tyr Ser Cys Pro Gly
    30                  35                  40

gtg ttc aac gcg gtg aag cgg ggg atg cag tcg gcc atc gcc agg gag      193
Val Phe Asn Ala Val Lys Arg Gly Met Gln Ser Ala Ile Ala Arg Glu
45                  50                  55                  60

aag cgc atc ggc gcc tcc atc gtc cgc ctc ttc ttc cac gac tgc ttc      241
Lys Arg Ile Gly Ala Ser Ile Val Arg Leu Phe Phe His Asp Cys Phe
                65                  70                  75

gtc caa ggt tgc gac gca tcg ctg ctg ctg gac gac acg gcg agc ttc     289
Val Gln Gly Cys Asp Ala Ser Leu Leu Leu Asp Asp Thr Ala Ser Phe
                80                  85                  90

acc ggc gag aag acg gcg aac ccc aac aac ggc tcc gtc aga ggg ttt     337
Thr Gly Glu Lys Thr Ala Asn Pro Asn Asn Gly Ser Val Arg Gly Phe
        95                  100                 105

gag gtg atc gac gcc atc aag tcg gcg gtg gag acc atc tgc ccc ggc     385
Glu Val Ile Asp Ala Ile Lys Ser Ala Val Glu Thr Ile Cys Pro Gly
    110                 115                 120

gtc gtc tcc tgc gcc gac atc ctc gcc atc gct gcc agg gac agc gtc     433
Val Val Ser Cys Ala Asp Ile Leu Ala Ile Ala Ala Arg Asp Ser Val
125                 130                 135                 140

gcc atc ctg ggt ggg ccg agc tgg gac gtg aag gtt ggt cgg aga gac     481
Ala Ile Leu Gly Gly Pro Ser Trp Asp Val Lys Val Gly Arg Arg Asp
                145                 150                 155

tcg cgc acg gcg agc ctc agc ggc gca aac aac aac atc ccg ccg ccg     529
Ser Arg Thr Ala Ser Leu Ser Gly Ala Asn Asn Asn Ile Pro Pro Pro
            160                 165                 170

acg tcg gga ctc gcc aac ctc acc tcc ctc ttc gcc gcg cag gcc ctc     577
Thr Ser Gly Leu Ala Asn Leu Thr Ser Leu Phe Ala Ala Gln Ala Leu
        175                 180                 185

tcc cag aag gac atg gtc gcc ctc tcc gga tct cac acc att ggg caa     625
Ser Gln Lys Asp Met Val Ala Leu Ser Gly Ser His Thr Ile Gly Gln
    190                 195                 200

gca cga tgc aca aac ttc aga gct cat ata tac aac gaa acc aac atc     673
Ala Arg Cys Thr Asn Phe Arg Ala His Ile Tyr Asn Glu Thr Asn Ile
205                 210                 215                 220

gac agt ggc ttt gcg atg agg agg caa tca ggt tgc cct cgt aac tca     721
Asp Ser Gly Phe Ala Met Arg Arg Gln Ser Gly Cys Pro Arg Asn Ser
                225                 230                 235

gga tca ggt gac aat aac ctg gca cct ctg gat ctt cag acg cca acc     769
Gly Ser Gly Asp Asn Asn Leu Ala Pro Leu Asp Leu Gln Thr Pro Thr
            240                 245                 250

gtg ttc gag aac aac tac tac aag aac ctc gtc gtc aag aag ggg ctc     817
Val Phe Glu Asn Asn Tyr Tyr Lys Asn Leu Val Val Lys Lys Gly Leu
        255                 260                 265

ctg cat tct gat cag gag ctc ttc aat ggc gga gcc act gat gct ctt     865
Leu His Ser Asp Gln Glu Leu Phe Asn Gly Gly Ala Thr Asp Ala Leu
    270                 275                 280

gtt cag tct tac ata agt agc cag agc aca ttc ttt gcg gat ttt gtg     913
Val Gln Ser Tyr Ile Ser Ser Gln Ser Thr Phe Phe Ala Asp Phe Val
285                 290                 295                 300

acg ggc atg atc aag atg ggg gac atc aca ccg ttg acg gga tca aac     961
Thr Gly Met Ile Lys Met Gly Asp Ile Thr Pro Leu Thr Gly Ser Asn
                305                 310                 315

ggg gag atc agg aag aac tgc aga agg att aat taa gaaatgatta          1007
Gly Glu Ile Arg Lys Asn Cys Arg Arg Ile Asn
            320                 325

agaggcaagc tgtgttcatg tccagtagtg caaacgcaca ttgtgtcgtg tttcttgcgg 1067

atatgtttgc aacatctccc ttaatttctt tcatgtgttt atgtatcatt atgttcgttt 1127

gaaataatga aagtactatg tgatttgttt t 1158

<210> 20
<211> 327
<212> PRT
<213> Oryza sativa
<223> r3025

<400> 20
Met Ala Gln Pro Thr Trp Ser Ala Arg Arg Val Thr Ala Ala Leu Val
1               5                   10                  15

Val Met Val Val Val Val Leu Ala Val Ala Gly Gly Ser Trp Ala Gln
            20                  25                  30

Leu Ser Pro Ser Phe Tyr Ser Tyr Ser Cys Pro Gly Val Phe Asn Ala
        35                  40                  45

Val Lys Arg Gly Met Gln Ser Ala Ile Ala Arg Glu Lys Arg Ile Gly
    50                  55                  60

Ala Ser Ile Val Arg Leu Phe Phe His Asp Cys Phe Val Gln Gly Cys
65                  70                  75                  80

Asp Ala Ser Leu Leu Leu Asp Asp Thr Ala Ser Phe Thr Gly Glu Lys
                85                  90                  95

Thr Ala Asn Pro Asn Asn Gly Ser Val Arg Gly Phe Glu Val Ile Asp
            100                 105                 110

Ala Ile Lys Ser Ala Val Glu Thr Ile Cys Pro Gly Val Val Ser Cys
        115                 120                 125

Ala Asp Ile Leu Ala Ile Ala Ala Arg Asp Ser Val Ala Ile Leu Gly
    130                 135                 140

Gly Pro Ser Trp Asp Val Lys Val Gly Arg Arg Asp Ser Arg Thr Ala
145                 150                 155                 160

Ser Leu Ser Gly Ala Asn Asn Asn Ile Pro Pro Pro Thr Ser Gly Leu
                165                 170                 175

Ala Asn Leu Thr Ser Leu Phe Ala Ala Gln Ala Leu Ser Gln Lys Asp
            180                 185                 190

Met Val Ala Leu Ser Gly Ser His Thr Ile Gly Gln Ala Arg Cys Thr
        195                 200                 205

Asn Phe Arg Ala His Ile Tyr Asn Glu Thr Asn Ile Asp Ser Gly Phe
    210                 215                 220

Ala Met Arg Arg Gln Ser Gly Cys Pro Arg Asn Ser Gly Ser Gly Asp
225                 230                 235                 240

Asn Asn Leu Ala Pro Leu Asp Leu Gln Thr Pro Thr Val Phe Glu Asn
                245                 250                 255

Asn Tyr Tyr Lys Asn Leu Val Val Lys Lys Gly Leu Leu His Ser Asp

260                 265                 270

Gln Glu Leu Phe Asn Gly Gly Ala Thr Asp Ala Leu Val Gln Ser Tyr
        275                 280                 285

Ile Ser Ser Gln Ser Thr Phe Phe Ala Asp Phe Val Thr Gly Met Ile
    290                 295                 300

Lys Met Gly Asp Ile Thr Pro Leu Thr Gly Ser Asn Gly Glu Ile Arg
305                 310                 315                 320

Lys Asn Cys Arg Arg Ile Asn
                325

<210> 21
<211> 1230
<212> DNA
<213> Oryza sativa

<220>
<221> CDS
<222> (110)..(1090)

<220>
<223> r2391

<400> 21
cgatcgagct taactaatta accatcagta gcaaattaag cctcgaccag ctaatcaatt 60

aattgagctt gtgctgttag ccgtgtcatc tatagctcgc tagcttgcc atg gtg tcc 118
                                                      Met Val Ser
                                                      1

tct gct atg gcg gcg gtg gcg gtg gcg ttt gct gta gtg gtg gcc gcg    166
Ser Ala Met Ala Ala Val Ala Val Ala Phe Ala Val Val Val Ala Ala
    5                   10                  15

acg atg agc agc gcg caa ctt gac ccg cac ttc tac gac ggc ttg tgc    214
Thr Met Ser Ser Ala Gln Leu Asp Pro His Phe Tyr Asp Gly Leu Cys
20                  25                  30                  35

ccg gcg gcg ctg ccc acc atc aag cgg atc gtc gag gag gcc gtc gcg    262
Pro Ala Ala Leu Pro Thr Ile Lys Arg Ile Val Glu Glu Ala Val Ala
                40                  45                  50

gcg gag ccc cgc atg ggc gcc tcg ctc ctg cgc ctg cac ttc cac gac    310
Ala Glu Pro Arg Met Gly Ala Ser Leu Leu Arg Leu His Phe His Asp
            55                  60                  65

tgc ttc gtc aac ggt tgc gac ggg tcc atc ctg ctg gac gac acg cca    358
Cys Phe Val Asn Gly Cys Asp Gly Ser Ile Leu Leu Asp Asp Thr Pro
        70                  75                  80

ttc ttc acg ggg gag aag aac gcg gcg ccc aac atg aac tcc gtc cgc    406
Phe Phe Thr Gly Glu Lys Asn Ala Ala Pro Asn Met Asn Ser Val Arg
    85                  90                  95

ggc ttc gac gtc atc gac cgc atc aag gac gcc gtc aac gcc gcc tgc    454
Gly Phe Asp Val Ile Asp Arg Ile Lys Asp Ala Val Asn Ala Ala Cys
100                 105                 110                 115

cgc cgc aac gtc gtc tcc tgc gcc gac atc gtc gcc gtc gcc gcg cgc    502
Arg Arg Asn Val Val Ser Cys Ala Asp Ile Val Ala Val Ala Ala Arg
                120                 125                 130

gac tcc atc gtc acc ctg gga ggg ccg tcg tac cac gtg ccg ctg ggc     550
Asp Ser Ile Val Thr Leu Gly Gly Pro Ser Tyr His Val Pro Leu Gly
            135                 140                 145

cgg agg gac gcg cgg acg gcg agc cag gcg gcg gcg aac agc agc atc     598
Arg Arg Asp Ala Arg Thr Ala Ser Gln Ala Ala Ala Asn Ser Ser Ile
        150                 155                 160

ccg gcg ccg acg ctc aac ctc gac ggc ctc gtc tcc agc ttc gcc gcg     646
Pro Ala Pro Thr Leu Asn Leu Asp Gly Leu Val Ser Ser Phe Ala Ala
    165                 170                 175

cag ggc ctc tcc gtg cag gac ctc gtc ctc ctc tcc ggc gcc cac acg     694
Gln Gly Leu Ser Val Gln Asp Leu Val Leu Leu Ser Gly Ala His Thr
180                 185                 190                 195

ctg ggc ttc tcc cgc tgc acc aac ttc cgc gac cgc ctc tac aac gag     742
Leu Gly Phe Ser Arg Cys Thr Asn Phe Arg Asp Arg Leu Tyr Asn Glu
                200                 205                 210

acg gcc acg ctc gac gcc tcc ctc gcc gcg tcg ctc ggg ggg acc tgc     790
Thr Ala Thr Leu Asp Ala Ser Leu Ala Ala Ser Leu Gly Gly Thr Cys
            215                 220                 225

ccg cgt acc gcc ggc gcc ggc gac gac aac ctc gcg ccg ctc gac ccg     838
Pro Arg Thr Ala Gly Ala Gly Asp Asp Asn Leu Ala Pro Leu Asp Pro
        230                 235                 240

acg ccg gcg agg ttc gac gcc gcg tac tac gcc tcg ctg ctg cgc gcc     886
Thr Pro Ala Arg Phe Asp Ala Ala Tyr Tyr Ala Ser Leu Leu Arg Ala
    245                 250                 255

agg ggg ctc ctg cac tcg gac cag cag ctg ttc gcc ggc ggc ggc ctc     934
Arg Gly Leu Leu His Ser Asp Gln Gln Leu Phe Ala Gly Gly Gly Leu
260                 265                 270                 275

ggc gcc acc gac ggg ctc gtc agg ttc tac gcc gcc aac ccg gac gcg     982
Gly Ala Thr Asp Gly Leu Val Arg Phe Tyr Ala Ala Asn Pro Asp Ala
                280                 285                 290

ttc cgg cga gac ttc gcc gag tcc atg gtg agg atg gcc agc ctg agc     1030
Phe Arg Arg Asp Phe Ala Glu Ser Met Val Arg Met Ala Ser Leu Ser
            295                 300                 305

ccg ctc gtc ggg agc caa ggc gag gtc cgc gtc aac tgc agg aag gtg     1078
Pro Leu Val Gly Ser Gln Gly Glu Val Arg Val Asn Cys Arg Lys Val
        310                 315                 320

aac tac tac tag caagaacgcg agatggccta aattaaatga gttccgaatg     1130
Asn Tyr Tyr
    325

aattaatagt gcttaagtat gttaattaat taccttctcc tgtggatgag agttcttcat 1190

tgtggtttaa ttagctcatt taattcagtt tgtgtgagtg                       1230

<210> 22
<211> 326
<212> PRT
<213> Oryza sativa
<223> r2391

<400> 22
Met Val Ser Ser Ala Met Ala Ala Val Ala Val Ala Phe Ala Val Val
1               5                   10                  15

Val Ala Ala Thr Met Ser Ser Ala Gln Leu Asp Pro His Phe Tyr Asp
            20                  25                  30

Gly Leu Cys Pro Ala Ala Leu Pro Thr Ile Lys Arg Ile Val Glu Glu
        35                  40                  45

Ala Val Ala Ala Glu Pro Arg Met Gly Ala Ser Leu Leu Arg Leu His
    50                  55                  60

Phe His Asp Cys Phe Val Asn Gly Cys Asp Gly Ser Ile Leu Leu Asp
65                  70                  75                  80

Asp Thr Pro Phe Phe Thr Gly Glu Lys Asn Ala Ala Pro Asn Met Asn
                85                  90                  95

Ser Val Arg Gly Phe Asp Val Ile Asp Arg Ile Lys Asp Ala Val Asn
            100                 105                 110

Ala Ala Cys Arg Arg Asn Val Val Ser Cys Ala Asp Ile Val Ala Val
        115                 120                 125

Ala Ala Arg Asp Ser Ile Val Thr Leu Gly Gly Pro Ser Tyr His Val
    130                 135                 140

Pro Leu Gly Arg Arg Asp Ala Arg Thr Ala Ser Gln Ala Ala Ala Asn
145                 150                 155                 160

Ser Ser Ile Pro Ala Pro Thr Leu Asn Leu Asp Gly Leu Val Ser Ser
                165                 170                 175

Phe Ala Ala Gln Gly Leu Ser Val Gln Asp Leu Val Leu Leu Ser Gly
            180                 185                 190

Ala His Thr Leu Gly Phe Ser Arg Cys Thr Asn Phe Arg Asp Arg Leu
        195                 200                 205

Tyr Asn Glu Thr Ala Thr Leu Asp Ala Ser Leu Ala Ala Ser Leu Gly
    210                 215                 220

Gly Thr Cys Pro Arg Thr Ala Gly Ala Gly Asp Asp Asn Leu Ala Pro
225                 230                 235                 240

Leu Asp Pro Thr Pro Ala Arg Phe Asp Ala Ala Tyr Tyr Ala Ser Leu
                245                 250                 255

Leu Arg Ala Arg Gly Leu Leu His Ser Asp Gln Gln Leu Phe Ala Gly
            260                 265                 270

Gly Gly Leu Gly Ala Thr Asp Gly Leu Val Arg Phe Tyr Ala Ala Asn
        275                 280                 285

Pro Asp Ala Phe Arg Arg Asp Phe Ala Glu Ser Met Val Arg Met Ala
    290                 295                 300

Ser Leu Ser Pro Leu Val Gly Ser Gln Gly Glu Val Arg Val Asn Cys
305                 310                 315                 320

Arg Lys Val Asn Tyr Tyr
                325

<210> 23
<211> 1171
<212> DNA
<213> Oryza sativa

<220>
<221> CDS
<222> (53)..(1033)

<220>
<223> s10927

<400> 23
tctgcttgct tctcttgctg gttcgtcggt cggtggagtt tgagcctgcg cc atg gct 58
Met Ala
1

gtc ggc ggg aga ggg aga cgg ccg ctg ctc ctg ctc ctg ctc ctc ctc 106
Val Gly Gly Arg Gly Arg Arg Pro Leu Leu Leu Leu Leu Leu Leu Leu
        5                   10                  15

gcc gtg gcg ctg gcg ctg gcg gcg cgc gcg cgg gcg cag ctg tcg ccg 154
Ala Val Ala Leu Ala Leu Ala Ala Arg Ala Arg Ala Gln Leu Ser Pro
    20                  25                  30

ggg ttc tac tcg gcg agc tgc ccc acc gtg cac ggc gtc gtg cgg cag 202
Gly Phe Tyr Ser Ala Ser Cys Pro Thr Val His Gly Val Val Arg Gln
35                  40                  45                  50

gtc atg tcg cag gcc gtc atg aac gac acg cgc gcc ggc gcc gcc gtc 250
Val Met Ser Gln Ala Val Met Asn Asp Thr Arg Ala Gly Ala Ala Val
                55                  60                  65

ctc cgc ctc ttc tac cac gac tgc ttc gtc ggc ggc tgc gac gcg tcg 298
Leu Arg Leu Phe Tyr His Asp Cys Phe Val Gly Gly Cys Asp Ala Ser
            70                  75                  80

gtg ctc ctc gac gac acc ccc gcg gcg ccc ggc gag aag ggc gtc ggc 346
Val Leu Leu Asp Asp Thr Pro Ala Ala Pro Gly Glu Lys Gly Val Gly
        85                  90                  95

ccc aac gcc gtc ggc tcg acg acc gtc ttc gac ctc gtc gac acc atc 394
Pro Asn Ala Val Gly Ser Thr Thr Val Phe Asp Leu Val Asp Thr Ile
    100                 105                 110

aag gcc cag gtc gag gcc gtc tgc ccc gcc acc gtc tcc tgc gcc gac 442
Lys Ala Gln Val Glu Ala Val Cys Pro Ala Thr Val Ser Cys Ala Asp
115                 120                 125                 130

gtc ctc gcc atc gcc gcg cgc gac agc gtc aac ctg ctc ggc ggg ccg 490
Val Leu Ala Ile Ala Ala Arg Asp Ser Val Asn Leu Leu Gly Gly Pro
                135                 140                 145

agc tgg gcg gtg ccg ctc ggc cgc cgc gac gcg ctg tcg ccg agt cgg 538
Ser Trp Ala Val Pro Leu Gly Arg Arg Asp Ala Leu Ser Pro Ser Arg
            150                 155                 160

agc gcg gtg tcg acc gac ctc ccg ggc ccc gag gcc gac atc tcc gcg 586
Ser Ala Val Ser Thr Asp Leu Pro Gly Pro Glu Ala Asp Ile Ser Ala
        165                 170                 175

ctc gtc tcc gcc ttc gcc gcc aag ggc ctg agc tcg cgc gac ctc gcc 634
Leu Val Ser Ala Phe Ala Ala Lys Gly Leu Ser Ser Arg Asp Leu Ala
    180                 185                 190

gcg ctg tcc ggc gcg cac acc gtc ggc cgc gcc agc tgc gtc aac ttc 682
Ala Leu Ser Gly Ala His Thr Val Gly Arg Ala Ser Cys Val Asn Phe
195                 200                 205                 210

cgc acc cgc gtc tac tgc gac gcc aac gtg agc ccg gcg ttc gcg tcg 730
Arg Thr Arg Val Tyr Cys Asp Ala Asn Val Ser Pro Ala Phe Ala Ser
                215                 220                 225

cac cag cgg cag tcc tgc ccg gcg tcc ggc ggc gac gcc gcg ctg gcg 778
His Gln Arg Gln Ser Cys Pro Ala Ser Gly Gly Asp Ala Ala Leu Ala
            230                 235                 240

ccg ctg gac tcc ctg acc ccc gac gcg ttc gac aac ggc tac tac cgc 826
Pro Leu Asp Ser Leu Thr Pro Asp Ala Phe Asp Asn Gly Tyr Tyr Arg
        245                 250                 255

aac ctc gtc gcc ggc gcc ggg ctg ctg cac tcc gac cag gag ctg ttc 874
Asn Leu Val Ala Gly Ala Gly Leu Leu His Ser Asp Gln Glu Leu Phe
    260                 265                 270

aac aac ggg ccg gtg gac tcg gtg gtg cag ctg tac agc tcc aac gcc 922
Asn Asn Gly Pro Val Asp Ser Val Val Gln Leu Tyr Ser Ser Asn Ala
275                 280                 285                 290

gcc gcc ttc tcg tcg gac ttc gcc gcg tcc atg atc agg ctc ggg aac 970
Ala Ala Phe Ser Ser Asp Phe Ala Ala Ser Met Ile Arg Leu Gly Asn
                295                 300                 305

atc ggc ccg ttg acc ggg tca acc ggc gag gtc agg ctc aac tgc agg 1018
Ile Gly Pro Leu Thr Gly Ser Thr Gly Glu Val Arg Leu Asn Cys Arg
            310                 315                 320

aaa gtg aat tcc tga tcgatactac tagatagcat tgccgcgcgc aaattgttca 1073
Lys Val Asn Ser
        325

tagtacaaag tttaaagtta tataatcagc tgtcaatttc atgtattaag tgaattatga 1133

cataagaatg caatagaaat gtcccaattt tagacatc 1171

<210> 24
<211> 326
<212> PRT
<213> Oryza sativa
<223> s10927

<400> 24
Met Ala Val Gly Gly Arg Gly Arg Arg Pro Leu Leu Leu Leu Leu Leu
1               5                   10                  15

Leu Leu Ala Val Ala Leu Ala Leu Ala Ala Arg Ala Arg Ala Gln Leu
            20                  25                  30

Ser Pro Gly Phe Tyr Ser Ala Ser Cys Pro Thr Val His Gly Val Val
        35                  40                  45

Arg Gln Val Met Ser Gln Ala Val Met Asn Asp Thr Arg Ala Gly Ala
    50                  55                  60

Ala Val Leu Arg Leu Phe Tyr His Asp Cys Phe Val Gly Gly Cys Asp
65                  70                  75                  80

Ala Ser Val Leu Leu Asp Asp Thr Pro Ala Ala Pro Gly Glu Lys Gly
                85                  90                  95

Val Gly Pro Asn Ala Val Gly Ser Thr Thr Val Phe Asp Leu Val Asp
            100                 105                 110

Thr Ile Lys Ala Gln Val Glu Ala Val Cys Pro Ala Thr Val Ser Cys
        115                 120                 125

Ala Asp Val Leu Ala Ile Ala Ala Arg Asp Ser Val Asn Leu Leu Gly
    130                 135                 140

Gly Pro Ser Trp Ala Val Pro Leu Gly Arg Arg Asp Ala Leu Ser Pro
145                 150                 155                 160

Ser Arg Ser Ala Val Ser Thr Asp Leu Pro Gly Pro Glu Ala Asp Ile
                165                 170                 175

Ser Ala Leu Val Ser Ala Phe Ala Ala Lys Gly Leu Ser Ser Arg Asp
            180                 185                 190

Leu Ala Ala Leu Ser Gly Ala His Thr Val Gly Arg Ala Ser Cys Val
        195                 200                 205

Asn Phe Arg Thr Arg Val Tyr Cys Asp Ala Asn Val Ser Pro Ala Phe
    210                 215                 220

Ala Ser His Gln Arg Gln Ser Cys Pro Ala Ser Gly Gly Asp Ala Ala
225                 230                 235                 240

Leu Ala Pro Leu Asp Ser Leu Thr Pro Asp Ala Phe Asp Asn Gly Tyr
                245                 250                 255

Tyr Arg Asn Leu Val Ala Gly Ala Gly Leu Leu His Ser Asp Gln Glu
            260                 265                 270

Leu Phe Asn Asn Gly Pro Val Asp Ser Val Val Gln Leu Tyr Ser Ser
        275                 280                 285

Asn Ala Ala Ala Phe Ser Ser Asp Phe Ala Ala Ser Met Ile Arg Leu
    290                 295                 300

Gly Asn Ile Gly Pro Leu Thr Gly Ser Thr Gly Glu Val Arg Leu Asn
305                 310                 315                 320

Cys Arg Lys Val Asn Ser
                325

<210> 25
<211> 1223
<212> DNA
<213> Oryza sativa

<220>
<221> CDS
<222> (20)..(982)

<220>
<223> s14493

<400> 25
gatatttcat tttgaccat atg ggt tac tcc tac tct tcc gcc gcc gtg gcg   52
                     Met Gly Tyr Ser Tyr Ser Ser Ala Ala Val Ala

1                 5                   10

gtg agc gtt ttg gtg gtg gcg ttg gcg gcg gcg gcg tcg ggc cag ctg     100
Val Ser Val Leu Val Val Ala Leu Ala Ala Ala Ala Ser Gly Gln Leu
                15                  20                  25

tcg acg acg ttc tac gcc tcg tcg tgc ccg acc gcg ctg tcg acg atc     148
Ser Thr Thr Phe Tyr Ala Ser Ser Cys Pro Thr Ala Leu Ser Thr Ile
            30                  35                  40

agg agc gcc gtg aac gcg gcg gtg gcc agg gag ccc cgc atg ggc gcc     196
Arg Ser Ala Val Asn Ala Ala Val Ala Arg Glu Pro Arg Met Gly Ala
        45                  50                  55

tcc ctg ctc agg ctc cac ttc cac gac tgc ttt gtc caa gga tgc gac     244
Ser Leu Leu Arg Leu His Phe His Asp Cys Phe Val Gln Gly Cys Asp
    60                  65                  70                  75

gcg tcg ata ctg ctg gcc gac aat gcc acc ttc cgg ggg gag cag ggt     292
Ala Ser Ile Leu Leu Ala Asp Asn Ala Thr Phe Arg Gly Glu Gln Gly
                80                  85                  90

gcg ttc cct aat gtc aac tcg ctg agg gga ttc gag gtc atc tct agc     340
Ala Phe Pro Asn Val Asn Ser Leu Arg Gly Phe Glu Val Ile Ser Ser
            95                  100                 105

att aag atg caa ctc gag gca tct tgc agg cag acc gtc tcc tgc gcc     388
Ile Lys Met Gln Leu Glu Ala Ser Cys Arg Gln Thr Val Ser Cys Ala
        110                 115                 120

gac atc ctt gct gtc gcc gcc cgc gac tcc gtc gtc gcc cta gga ggt     436
Asp Ile Leu Ala Val Ala Ala Arg Asp Ser Val Val Ala Leu Gly Gly
    125                 130                 135

cca tcg tac ccg gtg gag ctc ggg agg agg gac ggg atg acg acg aac     484
Pro Ser Tyr Pro Val Glu Leu Gly Arg Arg Asp Gly Met Thr Thr Asn
140                 145                 150                 155

caa acc atg gcg aac acc aac ctc cat cca ccg acc acc gac ctg ggt     532
Gln Thr Met Ala Asn Thr Asn Leu His Pro Pro Thr Thr Asp Leu Gly
                160                 165                 170

aac ttc gtc act agc ttc gcc ggg aaa ggg ctc agc ccc acc gac ctg     580
Asn Phe Val Thr Ser Phe Ala Gly Lys Gly Leu Ser Pro Thr Asp Leu
            175                 180                 185

gtt gta ctc act gga gcg cac acg gtg ggc gtg gcg cag tgc acc aac     628
Val Val Leu Thr Gly Ala His Thr Val Gly Val Ala Gln Cys Thr Asn
        190                 195                 200

ttc cgg tcg cgg ctc tac ggc gag tcc aac atc aac gcg ccg ttc gcg     676
Phe Arg Ser Arg Leu Tyr Gly Glu Ser Asn Ile Asn Ala Pro Phe Ala
    205                 210                 215

gcg tcg ctc cgg gcg agc tgc ccg cag gcc ggc ggc gac acc aac ctg     724
Ala Ser Leu Arg Ala Ser Cys Pro Gln Ala Gly Gly Asp Thr Asn Leu
220                 225                 230                 235

gcg ccg ctg gac tcc acc ccc aac gcc ttc gac aac gcc ttc ttc acc     772
Ala Pro Leu Asp Ser Thr Pro Asn Ala Phe Asp Asn Ala Phe Phe Thr
                240                 245                 250

gac ctc atc gcc ggc cgc ggc ctc ctc cac tcc gac cag gag ctc tac     820
Asp Leu Ile Ala Gly Arg Gly Leu Leu His Ser Asp Gln Glu Leu Tyr

            255                 260                 265

cgc ggc gac ggc tcc ggc acc gac gcc ctc gtc cgc gtc tac gcc gcc 868
Arg Gly Asp Gly Ser Gly Thr Asp Ala Leu Val Arg Val Tyr Ala Ala
        270                 275                 280

aac ccc gcc cgc ttc aac gcc gac ttc gcc gcc gcc atg gtg cgc atg 916
Asn Pro Ala Arg Phe Asn Ala Asp Phe Ala Ala Ala Met Val Arg Met
    285                 290                 295

ggc gcc atc agg ccg ctc acc ggc acg cag ggc gag atc agg ctc aac 964
Gly Ala Ile Arg Pro Leu Thr Gly Thr Gln Gly Glu Ile Arg Leu Asn
300                 305                 310                 315

tgc tcc agg gtc aac tga tctgtatatc tctacgaaat gcgtcgatct 1012
Cys Ser Arg Val Asn
                320

actgtaccta cgtggataga atcgaatcga ataagactag actattaata agctaataag 1072

tgattagcga tatcgcgata tgtataagtg cgcaacgtga tattgaattt atgtaagggc 1132

ttgtccggtt tattgccatt ttcaacacta tcgactttta atattattat taaattttgg 1192

tagaataaac gtactcttgt cattgctgcc t 1223

<210> 26
<211> 320
<212> PRT
<213> Oryza sativa
<223> s14493

<400> 26
Met Gly Tyr Ser Tyr Ser Ser Ala Ala Val Ala Val Ser Val Leu Val
1               5                   10                  15

Val Ala Leu Ala Ala Ala Ala Ser Gly Gln Leu Ser Thr Thr Phe Tyr
            20                  25                  30

Ala Ser Ser Cys Pro Thr Ala Leu Ser Thr Ile Arg Ser Ala Val Asn
        35                  40                  45

Ala Ala Val Ala Arg Glu Pro Arg Met Gly Ala Ser Leu Leu Arg Leu
    50                  55                  60

His Phe His Asp Cys Phe Val Gln Gly Cys Asp Ala Ser Ile Leu Leu
65                  70                  75                  80

Ala Asp Asn Ala Thr Phe Arg Gly Glu Gln Gly Ala Phe Pro Asn Val
                85                  90                  95

Asn Ser Leu Arg Gly Phe Glu Val Ile Ser Ser Ile Lys Met Gln Leu
            100                 105                 110

Glu Ala Ser Cys Arg Gln Thr Val Ser Cys Ala Asp Ile Leu Ala Val
        115                 120                 125

Ala Ala Arg Asp Ser Val Val Ala Leu Gly Gly Pro Ser Tyr Pro Val
    130                 135                 140

Glu Leu Gly Arg Arg Asp Gly Met Thr Thr Asn Gln Thr Met Ala Asn
145                 150                 155                 160

Thr Asn Leu His Pro Pro Thr Thr Asp Leu Gly Asn Phe Val Thr Ser
                165                 170                 175

Phe Ala Gly Lys Gly Leu Ser Pro Thr Asp Leu Val Val Leu Thr Gly
            180                 185                 190

Ala His Thr Val Gly Val Ala Gln Cys Thr Asn Phe Arg Ser Arg Leu
        195                 200                 205

Tyr Gly Glu Ser Asn Ile Asn Ala Pro Phe Ala Ala Ser Leu Arg Ala
    210                 215                 220

Ser Cys Pro Gln Ala Gly Gly Asp Thr Asn Leu Ala Pro Leu Asp Ser
225                 230                 235                 240

Thr Pro Asn Ala Phe Asp Asn Ala Phe Phe Thr Asp Leu Ile Ala Gly
                245                 250                 255

Arg Gly Leu Leu His Ser Asp Gln Glu Leu Tyr Arg Gly Asp Gly Ser
            260                 265                 270

Gly Thr Asp Ala Leu Val Arg Val Tyr Ala Ala Asn Pro Ala Arg Phe
        275                 280                 285

Asn Ala Asp Phe Ala Ala Ala Met Val Arg Met Gly Ala Ile Arg Pro
    290                 295                 300

Leu Thr Gly Thr Gln Gly Glu Ile Arg Leu Asn Cys Ser Arg Val Asn
305                 310                 315                 320


<210> 27
<211> 1348
<212> DNA
<213> Oryza sativa

<220>
<221> CDS
<222> (4)..(1011)

<220>
<223> prxRPN

<400> 27
   ggc atg gag tac gct act cgt gga gat cgc acg gct agc tgc ctg agt      48
       Met Glu Tyr Ala Thr Arg Gly Asp Arg Thr Ala Ser Cys Leu Ser
       1               5                   10                  15
   ttc ctc tgc aat atc gtc gtt ctg ctg ggc ctc gcc gcc gcg gcg ggc      96
   Phe Leu Cys Asn Ile Val Val Leu Leu Gly Leu Ala Ala Ala Ala Gly
                   20                  25                  30
   agc ggg cag ctg acg gac gac tac tac gac tat tgc tgc ccc cag gtt     144
   Ser Gly Gln Leu Thr Asp Asp Tyr Tyr Asp Tyr Cys Cys Pro Gln Val
               35                  40                  45
   tac cgc atc gtc cgg tcc cgc gtg gcc gcc gcg atg aag gcc gag atg     192
   Tyr Arg Ile Val Arg Ser Arg Val Ala Ala Ala Met Lys Ala Glu Met
           50                  55                  60
   cgc atg ggc gcc tcc ctg ctc agg ctt cac ttc cac gac tgc ttc gtc     240
   Arg Met Gly Ala Ser Leu Leu Arg Leu His Phe His Asp Cys Phe Val
       65                  70                  75
   aat ggc tgt gac gcg tcc atc ctc ctt gac ggc aca aac agc gag aag     288
   Asn Gly Cys Asp Ala Ser Ile Leu Leu Asp Gly Thr Asn Ser Glu Lys
    80                  85                  90                  95
   ttt gca ctt ccc aac aag aac tcg gtg aga ggg tac gaa gtc atc gat     336
   Phe Ala Leu Pro Asn Lys Asn Ser Val Arg Gly Tyr Glu Val Ile Asp
                   100                 105                 110
   gcg ata aag gcc gac ctc gag ggc gcc tgc ccg gga gtc gtc tcc tgc     384
   Ala Ile Lys Ala Asp Leu Glu Gly Ala Cys Pro Gly Val Val Ser Cys
               115                 120                 125
   gcc gac ata gta gcc ctt gca gct aaa tac gga gta cta ctt agt gga     432
   Ala Asp Ile Val Ala Leu Ala Ala Lys Tyr Gly Val Leu Leu Ser Gly
           130                 135                 140
   gga cct gat tat gat gtc ctc ctg gga aga aga gat ggt ctg gtg gca     480
   Gly Pro Asp Tyr Asp Val Leu Leu Gly Arg Arg Asp Gly Leu Val Ala
       145                 150                 155
   aat cag acg ggg gcg aac agt aac ttg cct agc cct ttc gat tcg atc     528
   Asn Gln Thr Gly Ala Asn Ser Asn Leu Pro Ser Pro Phe Asp Ser Ile
   160                 165                 170                 175
   agc gtt atc act gcg agg ttc aag gat gtc ggt ctc aac gca acc gat     576
   Ser Val Ile Thr Ala Arg Phe Lys Asp Val Gly Leu Asn Ala Thr Asp
                   180                 185                 190
   gtt gtg gtc tta tca ggg gcg cac acg atc ggg cga tct cgc tgc ctg     624
   Val Val Val Leu Ser Gly Ala His Thr Ile Gly Arg Ser Arg Cys Leu
               195                 200                 205
   ctg ttc agc aac cgg ctg gcg aac ttc tcg gcg acc aac tcc gtc gac     672
   Leu Phe Ser Asn Arg Leu Ala Asn Phe Ser Ala Thr Asn Ser Val Asp
           210                 215                 220
   ccg acg ctg gac tcg tcg ctg gcg tcc agc ctg cag cag gtg tgc cgc     720
   Pro Thr Leu Asp Ser Ser Leu Ala Ser Ser Leu Gln Gln Val Cys Arg
       225                 230                 235
   ggc ggc gct gac cag ctg gcg gcg ctg gac gtc aac tcc gcc gac gcg     768
   Gly Gly Ala Asp Gln Leu Ala Ala Leu Asp Val Asn Ser Ala Asp Ala
   240                 245                 250                 255
   ttc gac aac cac tac tac cag aac ctg ctg gcc aac aag ggc ctc ctc     816
   Phe Asp Asn His Tyr Tyr Gln Asn Leu Leu Ala Asn Lys Gly Leu Leu
                   260                 265                 270
   gcc tcc gac cag ggc ctc gtc tcc agc tcc ggc gac ccc gcc gtc gcc     864
   Ala Ser Asp Gln Gly Leu Val Ser Ser Ser Gly Asp Pro Ala Val Ala
               275                 280                 285
   gcc acc aag gcg ctg gtg cag gcc tac agc gcc aat ggc cag cgc ttc     912
   Ala Thr Lys Ala Leu Val Gln Ala Tyr Ser Ala Asn Gly Gln Arg Phe
           290                 295                 300
   tcc tgc gac ttc ggc aac tcc atg gtc aag atg ggc aac atc agc cct     960
   Ser Cys Asp Phe Gly Asn Ser Met Val Lys Met Gly Asn Ile Ser Pro
       305                 310                 315
   ctc acc ggc tct gcc ggc cag att cgc aag aac tgc agg gcc gtc aac     1008
   Leu Thr Gly Ser Ala Gly Gln Ile Arg Lys Asn Cys Arg Ala Val Asn
   320                 325                 330                 335
   tga tgagcaaaaa ggcaaagatt ttttgcatct gccatgaccc catcttggat 1061
   ttgccagaag ctatatcgtc ttcttggact caagtgtgaa tctgtcgttt ttaatgtgtt 1121
   gtggagcgct acatgtttcg ttttgttcaa gctatctagg attctctctc caatgcacga 1181
   gtagaataag caattagcat gcaaagttgc tcgtcctaca cgaatctgca gctgcatttt 1241
   cagtgggtgt atcaccaatt attagagcac agacaaacgc gctgtgtcta tcagagtaaa 1301
   agaaagaata tgcaactgcc atatggttta aaaaaaaaaa aaaaaaa 1348

<210> 28
<211> 335
<212> PRT
<213> Oryza sativa
<223> prxRPN

<400> 28
Met Glu Tyr Ala Thr Arg Gly Asp Arg Thr Ala Ser Cys Leu Ser Phe
1               5                   10                  15

Leu Cys Asn Ile Val Val Leu Leu Gly Leu Ala Ala Ala Ala Gly Ser
            20                  25                  30

Gly Gln Leu Thr Asp Asp Tyr Tyr Asp Tyr Cys Cys Pro Gln Val Tyr
        35                  40                  45

Arg Ile Val Arg Ser Arg Val Ala Ala Ala Met Lys Ala Glu Met Arg
    50                  55                  60

Met Gly Ala Ser Leu Leu Arg Leu His Phe His Asp Cys Phe Val Asn
65                  70                  75                  80

Gly Cys Asp Ala Ser Ile Leu Leu Asp Gly Thr Asn Ser Glu Lys Phe
                85                  90                  95

Ala Leu Pro Asn Lys Asn Ser Val Arg Gly Tyr Glu Val Ile Asp Ala
            100                 105                 110

Ile Lys Ala Asp Leu Glu Gly Ala Cys Pro Gly Val Val Ser Cys Ala
        115                 120                 125

Asp Ile Val Ala Leu Ala Ala Lys Tyr Gly Val Leu Leu Ser Gly Gly
    130                 135                 140

Pro Asp Tyr Asp Val Leu Leu Gly Arg Arg Asp Gly Leu Val Ala Asn
145                 150                 155                 160

Gln Thr Gly Ala Asn Ser Asn Leu Pro Ser Pro Phe Asp Ser Ile Ser
                165                 170                 175

Val Ile Thr Ala Arg Phe Lys Asp Val Gly Leu Asn Ala Thr Asp Val
            180                 185                 190

Val Val Leu Ser Gly Ala His Thr Ile Gly Arg Ser Arg Cys Leu Leu
        195                 200                 205

Phe Ser Asn Arg Leu Ala Asn Phe Ser Ala Thr Asn Ser Val Asp Pro
    210                 215                 220

Thr Leu Asp Ser Ser Leu Ala Ser Ser Leu Gln Gln Val Cys Arg Gly

225                 230                 235                 240

Gly Ala Asp Gln Leu Ala Ala Leu Asp Val Asn Ser Ala Asp Ala Phe
                245                 250                 255

Asp Asn His Tyr Tyr Gln Asn Leu Leu Ala Asn Lys Gly Leu Leu Ala
            260                 265                 270

Ser Asp Gln Gly Leu Val Ser Ser Ser Gly Asp Pro Ala Val Ala Ala
        275                 280                 285

Thr Lys Ala Leu Val Gln Ala Tyr Ser Ala Asn Gly Gln Arg Phe Ser
    290                 295                 300

Cys Asp Phe Gly Asn Ser Met Val Lys Met Gly Asn Ile Ser Pro Leu
305                 310                 315                 320

Thr Gly Ser Ala Gly Gln Ile Arg Lys Asn Cys Arg Ala Val Asn
                325                 330                 335


<210> 29
<211> 1310
<212> DNA
<213> Oryza sativa

<220>
<221> CDS
<222> (81)..(1025)

<220>
<223> r2576

<400> 29
cgaaaaagct taaaaaaaac tgcagttatt taggtaggat taagcattac gcgcacggcg 60

catcatatcg ctcactctgc atg gct tcg gct tct tct gtc agc tta atg ctg 113
                      Met Ala Ser Ala Ser Ser Val Ser Leu Met Leu
                      1               5                   10

ctc gtg gct gca gct atg gcc tca gcc gcc tcg gcg cag ctg tcg gcg   161
Leu Val Ala Ala Ala Met Ala Ser Ala Ala Ser Ala Gln Leu Ser Ala
            15                  20                  25

acg ttc tac gac acg tcg tgc cca aac gca ttg tcc acc atc aag agc   209
Thr Phe Tyr Asp Thr Ser Cys Pro Asn Ala Leu Ser Thr Ile Lys Ser
        30                  35                  40

gca gtg acg gcc gcc gtg aac agc gag ccc cga atg gga gcc tcg ctg   257
Ala Val Thr Ala Ala Val Asn Ser Glu Pro Arg Met Gly Ala Ser Leu
    45                  50                  55

gtc agg ctg cac ttc cac gac tgc ttc gtc caa ggc tgt gac gcg tct   305
Val Arg Leu His Phe His Asp Cys Phe Val Gln Gly Cys Asp Ala Ser
60                  65                  70                  75

gtt ctg ctg tcc ggc cag gag cag aat gca ggc ccg aac gct ggg tca   353
Val Leu Leu Ser Gly Gln Glu Gln Asn Ala Gly Pro Asn Ala Gly Ser
                80                  85                  90

ctt cgg gga ttc aac gtc gtc gac aac atc aag acg cag gtc gag gcc   401
Leu Arg Gly Phe Asn Val Val Asp Asn Ile Lys Thr Gln Val Glu Ala
            95                  100                 105

atc tgc agc cag acc gtc tcc tgc gcc gac atc ctc gcc gtc gcc gcc 449
Ile Cys Ser Gln Thr Val Ser Cys Ala Asp Ile Leu Ala Val Ala Ala
        110                 115                 120

cgt gac tcc gtc gtc gcg ctc gga ggg ccg tcg tgg acg gtt cta ttg 497
Arg Asp Ser Val Val Ala Leu Gly Gly Pro Ser Trp Thr Val Leu Leu
    125                 130                 135

gga aga agg gac tcc acc act gcg aac gaa agc caa gca aat acc gac 545
Gly Arg Arg Asp Ser Thr Thr Ala Asn Glu Ser Gln Ala Asn Thr Asp
140                 145                 150                 155

ctc cct gcc cct tcc tct agc ctc gct gaa ctt atc ggc aat ttc tcc 593
Leu Pro Ala Pro Ser Ser Ser Leu Ala Glu Leu Ile Gly Asn Phe Ser
                160                 165                 170

aga aag gga ctc gac gta acc gac atg gtt gct ctc tca ggc gca cac 641
Arg Lys Gly Leu Asp Val Thr Asp Met Val Ala Leu Ser Gly Ala His
            175                 180                 185

acg atc ggg cag gcg cag tgc cag aac ttc agg gac agg ctc tac aac 689
Thr Ile Gly Gln Ala Gln Cys Gln Asn Phe Arg Asp Arg Leu Tyr Asn
        190                 195                 200

gag aca aac atc gac tcc agc ttc gcg acg gcg ctc aag gcc aac tgc 737
Glu Thr Asn Ile Asp Ser Ser Phe Ala Thr Ala Leu Lys Ala Asn Cys
    205                 210                 215

cca cgg ccg acg ggc agc ggc gac agc aac ctg gcg ccg ctg gac acg 785
Pro Arg Pro Thr Gly Ser Gly Asp Ser Asn Leu Ala Pro Leu Asp Thr
220                 225                 230                 235

acg acg ccc aac gcc ttc gac agc gcc tac tac acc aac ctg ctg tcc 833
Thr Thr Pro Asn Ala Phe Asp Ser Ala Tyr Tyr Thr Asn Leu Leu Ser
                240                 245                 250

aac aag ggc ctc ctg cac tcc gac cag gtg ctc ttc aac ggc ggc agc 881
Asn Lys Gly Leu Leu His Ser Asp Gln Val Leu Phe Asn Gly Gly Ser
            255                 260                 265

acg gac aac acg gtc agg aac ttc tcc tcc aac acg gcg gcg ttc aac 929
Thr Asp Asn Thr Val Arg Asn Phe Ser Ser Asn Thr Ala Ala Phe Asn
        270                 275                 280

agc gcc ttc acg gcg gcc atg gtg aag atg ggg aac atc tcg ccc ttg 977
Ser Ala Phe Thr Ala Ala Met Val Lys Met Gly Asn Ile Ser Pro Leu
    285                 290                 295

act gga acc cag ggg cag atc agg ctc aac tgc tcc aag gtt aac tag 1025
Thr Gly Thr Gln Gly Gln Ile Arg Leu Asn Cys Ser Lys Val Asn
300                 305                 310

ccggtgatga cgagtacgtg atggtctcag ggaataaagc taggccccga cattgctgcc 1085

aatagcgtgc agctagctgt ggccatctca tcactttcat ggttcaatgg tttcatcagc 1145

aagtgcacgg aacagactag agagtgtagg tgtatgcatg tgtgatgtca tgtaacgagt 1205

tgccatgcaa aggcatgagg attacggacc tctctttcgt cgtaatccta gcagctacag 1265

atttcaactg tcgtgttaac caagtgaagt gctagtttcg aatgt 1310

<210> 30

<211> 314
<212> PRT
<213> Oryza sativa
<223> r2576

<400> 30
Met Ala Ser Ala Ser Ser Val Ser Leu Met Leu Leu Val Ala Ala Ala
1               5                   10                  15

Met Ala Ser Ala Ala Ser Ala Gln Leu Ser Ala Thr Phe Tyr Asp Thr
            20                  25                  30

Ser Cys Pro Asn Ala Leu Ser Thr Ile Lys Ser Ala Val Thr Ala Ala
        35                  40                  45

Val Asn Ser Glu Pro Arg Met Gly Ala Ser Leu Val Arg Leu His Phe
    50                  55                  60

His Asp Cys Phe Val Gln Gly Cys Asp Ala Ser Val Leu Leu Ser Gly
65                  70                  75                  80

Gln Glu Gln Asn Ala Gly Pro Asn Ala Gly Ser Leu Arg Gly Phe Asn
                85                  90                  95

Val Val Asp Asn Ile Lys Thr Gln Val Glu Ala Ile Cys Ser Gln Thr
            100                 105                 110

Val Ser Cys Ala Asp Ile Leu Ala Val Ala Ala Arg Asp Ser Val Val
        115                 120                 125

Ala Leu Gly Gly Pro Ser Trp Thr Val Leu Leu Gly Arg Arg Asp Ser
    130                 135                 140

Thr Thr Ala Asn Glu Ser Gln Ala Asn Thr Asp Leu Pro Ala Pro Ser
145                 150                 155                 160

Ser Ser Leu Ala Glu Leu Ile Gly Asn Phe Ser Arg Lys Gly Leu Asp
                165                 170                 175

Val Thr Asp Met Val Ala Leu Ser Gly Ala His Thr Ile Gly Gln Ala
            180                 185                 190

Gln Cys Gln Asn Phe Arg Asp Arg Leu Tyr Asn Glu Thr Asn Ile Asp
        195                 200                 205

Ser Ser Phe Ala Thr Ala Leu Lys Ala Asn Cys Pro Arg Pro Thr Gly
    210                 215                 220

Ser Gly Asp Ser Asn Leu Ala Pro Leu Asp Thr Thr Thr Pro Asn Ala
225                 230                 235                 240

Phe Asp Ser Ala Tyr Tyr Thr Asn Leu Leu Ser Asn Lys Gly Leu Leu
                245                 250                 255

His Ser Asp Gln Val Leu Phe Asn Gly Gly Ser Thr Asp Asn Thr Val
            260                 265                 270

Arg Asn Phe Ser Ser Asn Thr Ala Ala Phe Asn Ser Ala Phe Thr Ala
        275                 280                 285

Ala Met Val Lys Met Gly Asn Ile Ser Pro Leu Thr Gly Thr Gln Gly
    290                 295                 300

Gln Ile Arg Leu Asn Cys Ser Lys Val Asn

305                 310

<210> 31
<211> 1306
<212> DNA
<213> Oryza sativa

<220>
<221> CDS
<222> (44)..(1084)

<220>
<223> r2184

<400> 31
cgagctgaga gtgatcgatc tttgttagct agagtgttga gca atg gcg tcc aag     55
                                                Met Ala Ser Lys
                                                1

ctg ggt atg gtt gtg cta ctg atc tcg ggc ctt ttt gct gcc cgt tgc     103
Leu Gly Met Val Val Leu Leu Ile Ser Gly Leu Phe Ala Ala Arg Cys
5                   10                  15                  20

gcg gcc gtg gtg acc acc ggc gaa ccc gtc gtc gcc ggc ctc tcc tgg     151
Ala Ala Val Val Thr Thr Gly Glu Pro Val Val Ala Gly Leu Ser Trp
                25                  30                  35

ggg ttc tat gac acg tcg tgc ccg tcg gtg gag ggc atc gtg agg tgg     199
Gly Phe Tyr Asp Thr Ser Cys Pro Ser Val Glu Gly Ile Val Arg Trp
            40                  45                  50

cac gtc acc gag gcc ctc cgc cgc gac atc ggc atc gcc gcc ggc ctc     247
His Val Thr Glu Ala Leu Arg Arg Asp Ile Gly Ile Ala Ala Gly Leu
        55                  60                  65

gtc cgc atc ttc ttc cac gac tgc ttc ccg cag ggg tgc gac gcg tcg     295
Val Arg Ile Phe Phe His Asp Cys Phe Pro Gln Gly Cys Asp Ala Ser
    70                  75                  80

gtc ctc ctg acg ggt tcc caa agc gag ctg ggt gag ata ccc aac cag     343
Val Leu Leu Thr Gly Ser Gln Ser Glu Leu Gly Glu Ile Pro Asn Gln
85                  90                  95                  100

acg ctg cgg ccg tcg gcg ctg aag ctc atc gag gac atc cgc gcc gcc     391
Thr Leu Arg Pro Ser Ala Leu Lys Leu Ile Glu Asp Ile Arg Ala Ala
                105                 110                 115

gta cac tcc gcc tgc ggc gcc aag gtg tcc tgc gcc gac atc acc acg     439
Val His Ser Ala Cys Gly Ala Lys Val Ser Cys Ala Asp Ile Thr Thr
            120                 125                 130

ctc gcc acg cgt gac gcc atc gtc gcc tcc ggc ggg ccc tac ttc gac     487
Leu Ala Thr Arg Asp Ala Ile Val Ala Ser Gly Gly Pro Tyr Phe Asp
        135                 140                 145

gtg cct ctg ggg cgg cgc gac ggg ctg gca ccg gcg tcg agc gac aag     535
Val Pro Leu Gly Arg Arg Asp Gly Leu Ala Pro Ala Ser Ser Asp Lys
    150                 155                 160

gtg ggc ctc ctg ccg gcg ccc ttc ttc gac gtg ccc acg ctc atc cag     583
Val Gly Leu Leu Pro Ala Pro Phe Phe Asp Val Pro Thr Leu Ile Gln
165                 170                 175                 180

gcg ttc aag gac cga aac ctg gac aag acg gac ctg gtg gcg ctg tcc     631

Ala Phe Lys Asp Arg Asn Leu Asp Lys Thr Asp Leu Val Ala Leu Ser
                185                 190                 195

ggc gcg cac acc atc gga cta ggc cac tgc ggc agc ttc aac gac cgc     679
Gly Ala His Thr Ile Gly Leu Gly His Cys Gly Ser Phe Asn Asp Arg
            200                 205                 210

ttc gat ggc tcc aag ccc atc atg gac cct gtg ctg gtg aag aag ctg     727
Phe Asp Gly Ser Lys Pro Ile Met Asp Pro Val Leu Val Lys Lys Leu
        215                 220                 225

cag gcc aag tgc gcc aag gac gtg ccg gtg aac tcg gtc acg cag gag     775
Gln Ala Lys Cys Ala Lys Asp Val Pro Val Asn Ser Val Thr Gln Glu
    230                 235                 240

ctg gac gtc cgc acg ccc aac gcc ttc gac aac aag tac tac ttc gac     823
Leu Asp Val Arg Thr Pro Asn Ala Phe Asp Asn Lys Tyr Tyr Phe Asp
245                 250                 255                 260

ctc atc gcc aag cag ggg atc ttc aag tcc gac cag ggc ctc atc gag     871
Leu Ile Ala Lys Gln Gly Ile Phe Lys Ser Asp Gln Gly Leu Ile Glu
                265                 270                 275

gac gcg cag acc aac cgc acc gcc gtc cgc ttc gcc ctc aac cag gcc     919
Asp Ala Gln Thr Asn Arg Thr Ala Val Arg Phe Ala Leu Asn Gln Ala
            280                 285                 290

gcc ttc ttc gac cag ttc gca cgc tcc atg gtc aag atg agc cag atg     967
Ala Phe Phe Asp Gln Phe Ala Arg Ser Met Val Lys Met Ser Gln Met
        295                 300                 305

gac gtc ctc acc ggc aac gcc ggc gag atc cgc aac aac tgc gcc gct     1015
Asp Val Leu Thr Gly Asn Ala Gly Glu Ile Arg Asn Asn Cys Ala Ala
    310                 315                 320

ccc aac cgc cgc tcc tcc gac ctc ctc aac gct gcc gac gac gac caa     1063
Pro Asn Arg Arg Ser Ser Asp Leu Leu Asn Ala Ala Asp Asp Asp Gln
325                 330                 335                 340

ggc ttc gcc gcc gac gcc taa ttaacttatg gagtaattag tgatcgtttt       1114
Gly Phe Ala Ala Asp Ala
                345

atgtttatgt tttgtgctag taataataat taagaggatg ccatctgcgc gtggtgtttg 1174

gtttccatgc attctctgct tagttagaat ggttttgctt cataaaaagt aaagttacta 1234

ctcgattcct cggtcgggac agagtaactg catgtcaaat gtatcatcat catcagttag 1294

tgctacatca tc                                                      1306

<210> 32
<211> 346
<212> PRT
<213> Oryza sativa
<223> r2184

<400> 32
Met Ala Ser Lys Leu Gly Met Val Val Leu Leu Ile Ser Gly Leu Phe
  1               5                  10                  15

Ala Ala Arg Cys Ala Ala Val Val Thr Thr Gly Glu Pro Val Val Ala
             20                  25                  30

Gly Leu Ser Trp Gly Phe Tyr Asp Thr Ser Cys Pro Ser Val Glu Gly
        35                  40                  45

Ile Val Arg Trp His Val Thr Glu Ala Leu Arg Arg Asp Ile Gly Ile
    50                  55                  60

Ala Ala Gly Leu Val Arg Ile Phe Phe His Asp Cys Phe Pro Gln Gly
65                  70                  75                  80

Cys Asp Ala Ser Val Leu Leu Thr Gly Ser Gln Ser Glu Leu Gly Glu
                85                  90                  95

Ile Pro Asn Gln Thr Leu Arg Pro Ser Ala Leu Lys Leu Ile Glu Asp
            100                 105                 110

Ile Arg Ala Ala Val His Ser Ala Cys Gly Ala Lys Val Ser Cys Ala
        115                 120                 125

Asp Ile Thr Thr Leu Ala Thr Arg Asp Ala Ile Val Ala Ser Gly Gly
    130                 135                 140

Pro Tyr Phe Asp Val Pro Leu Gly Arg Arg Asp Gly Leu Ala Pro Ala
145                 150                 155                 160

Ser Ser Asp Lys Val Gly Leu Leu Pro Ala Pro Phe Phe Asp Val Pro
                165                 170                 175

Thr Leu Ile Gln Ala Phe Lys Asp Arg Asn Leu Asp Lys Thr Asp Leu
            180                 185                 190

Val Ala Leu Ser Gly Ala His Thr Ile Gly Leu Gly His Cys Gly Ser
        195                 200                 205

Phe Asn Asp Arg Phe Asp Gly Ser Lys Pro Ile Met Asp Pro Val Leu
    210                 215                 220

Val Lys Lys Leu Gln Ala Lys Cys Ala Lys Asp Val Pro Val Asn Ser
225                 230                 235                 240

Val Thr Gln Glu Leu Asp Val Arg Thr Pro Asn Ala Phe Asp Asn Lys
                245                 250                 255

Tyr Tyr Phe Asp Leu Ile Ala Lys Gln Gly Ile Phe Lys Ser Asp Gln
            260                 265                 270

Gly Leu Ile Glu Asp Ala Gln Thr Asn Arg Thr Ala Val Arg Phe Ala
        275                 280                 285

Leu Asn Gln Ala Ala Phe Phe Asp Gln Phe Ala Arg Ser Met Val Lys
    290                 295                 300

Met Ser Gln Met Asp Val Leu Thr Gly Asn Ala Gly Glu Ile Arg Asn
305                 310                 315                 320

Asn Cys Ala Ala Pro Asn Arg Arg Ser Ser Asp Leu Leu Asn Ala Ala
                325                 330                 335

Asp Asp Asp Gln Gly Phe Ala Ala Asp Ala
            340                 345


<210> 33
<211> 1316

<212> DNA
<213> Oryza sativa

<220>
<221> CDS
<222> (68)..(1114)

<220>
<223> r2693

<400> 33
tgatctctca cactcggatc ttcttcttga ttggttgcac acatcatcat catcatcatc 60

atcggag atg gtg aag ctt gtg tgc ttt gtt gtg gtg gtg ttc atg gcg 109
Met Val Lys Leu Val Cys Phe Val Val Val Val Phe Met Ala
1               5                   10

gcg gcg gcg gcg atg gcc gga gcc gat cgc gag ctg aag gtt ggg tac 157
Ala Ala Ala Ala Met Ala Gly Ala Asp Arg Glu Leu Lys Val Gly Tyr
15                  20                  25                  30

tac gag aag acg tgc aag gac gtg gag aag atc gtg aac tcc atc gtc 205
Tyr Glu Lys Thr Cys Lys Asp Val Glu Lys Ile Val Asn Ser Ile Val
                35                  40                  45

gtc aac tcc atc aag gac aac cgc ggc aag ggc gct ggc ctc gtc cgc 253
Val Asn Ser Ile Lys Asp Asn Arg Gly Lys Gly Ala Gly Leu Val Arg
            50                  55                  60

ctc ctc ttc cac gac tgc ttc gtc agg ggg tgt gac gcc tcc gtg ctt 301
Leu Leu Phe His Asp Cys Phe Val Arg Gly Cys Asp Ala Ser Val Leu
        65                  70                  75

ctg gag aag agc gag atg aac agg cag ccg gag aag gaa tct ccg gcg 349
Leu Glu Lys Ser Glu Met Asn Arg Gln Pro Glu Lys Glu Ser Pro Ala
    80                  85                  90

aac atc ggg atc cgg ggg atg gac gtg atc gac gcg atc aag gcg gtg 397
Asn Ile Gly Ile Arg Gly Met Asp Val Ile Asp Ala Ile Lys Ala Val
95                  100                 105                 110

ctg gag gcg cgg tgc ccc aac acg gtg tcg tgc gcc gac atc atc gcg 445
Leu Glu Ala Arg Cys Pro Asn Thr Val Ser Cys Ala Asp Ile Ile Ala
                115                 120                 125

tac gcg gcg cgc gac gcg tcc agg tac ctc agc cac ggc ggc gtc gac 493
Tyr Ala Ala Arg Asp Ala Ser Arg Tyr Leu Ser His Gly Gly Val Asp
            130                 135                 140

ttc ccc gtc cct gcc ggc cgc ctc gac ggc gtg gtc tcc cgc agc cgc 541
Phe Pro Val Pro Ala Gly Arg Leu Asp Gly Val Val Ser Arg Ser Arg
        145                 150                 155

gac gcc gac gcg ttc ctc ccg gac gcc gcc gca aac ctc acc gac ctc 589
Asp Ala Asp Ala Phe Leu Pro Asp Ala Ala Ala Asn Leu Thr Asp Leu
    160                 165                 170

gtc cgc aac ttc cgc cgc aag aac ttc acc gtg gag gag ctc gtc atc 637
Val Arg Asn Phe Arg Arg Lys Asn Phe Thr Val Glu Glu Leu Val Ile
175                 180                 185                 190

ctc tcc ggc gcg cac tcc atc ggc gtc acc cac tgc acc tcc ttc gcc 685
Leu Ser Gly Ala His Ser Ile Gly Val Thr His Cys Thr Ser Phe Ala
                195                 200                 205

ggc cgc ctc acc gcc ccg gac gcc cag atc aac ccg ggc tac cgc agc 733
Gly Arg Leu Thr Ala Pro Asp Ala Gln Ile Asn Pro Gly Tyr Arg Ser
            210                 215                 220

ctc ctc gtc tcc aag tgc ggc ggc gtg tcg ccg acg ccg gcc aac aac 781
Leu Leu Val Ser Lys Cys Gly Gly Val Ser Pro Thr Pro Ala Asn Asn
        225                 230                 235

cac gtc gtg gtg aac aac gtg cgc gac gag gac ggc gcc gcc gtg gcg 829
His Val Val Val Asn Asn Val Arg Asp Glu Asp Gly Ala Ala Val Ala
    240                 245                 250

agg gtc atg ccg ggg ttt gcg gcg agg gtg agg aag gcg agg gac tac 877
Arg Val Met Pro Gly Phe Ala Ala Arg Val Arg Lys Ala Arg Asp Tyr
255                 260                 265                 270

ctg gac aac agc tac tac cac aac aac ctc gcc atg gcg gtg acg ttc 925
Leu Asp Asn Ser Tyr Tyr His Asn Asn Leu Ala Met Ala Val Thr Phe
                275                 280                 285

cac gcg gac tgg gcg ctg ctc acc ggg aag gag gcg cgc ggg cac gtc 973
His Ala Asp Trp Ala Leu Leu Thr Gly Lys Glu Ala Arg Gly His Val
            290                 295                 300

gtg gag tac gcc aag aac gcg acg ctg tgg aac gtg gac ttc ggc gac 1021
Val Glu Tyr Ala Lys Asn Ala Thr Leu Trp Asn Val Asp Phe Gly Asp
        305                 310                 315

gcg ctg gtg aag ctg agc aag ctg ccc atg ccg gcg ggg agc aag ggg 1069
Ala Leu Val Lys Leu Ser Lys Leu Pro Met Pro Ala Gly Ser Lys Gly
    320                 325                 330

gag atc agg gcc aag tgc agc gcc gtc aac ggc tac cac cat tga 1114
Glu Ile Arg Ala Lys Cys Ser Ala Val Asn Gly Tyr His His
335                 340                 345

cgtcgacgac gatccttcca tcgatcgatc gatcgaacga tctcatggat atatgatgca 1174

gtagctttga attaatgttt gactatgata tgatctctgt aactctattg tattttttgt 1234

tccttcgccc gcgtgagtac tagttcaaat tcaattcgtt gtactgcaaa cttttcaaaa 1294

taattatgat gcaaagtagt tc 1316

<210> 34
<211> 348
<212> PRT
<213> Oryza sativa
<223> r2693

<400> 34
Met Val Lys Leu Val Cys Phe Val Val Val Val Phe Met Ala Ala Ala
1               5                   10                  15

Ala Ala Met Ala Gly Ala Asp Arg Glu Leu Lys Val Gly Tyr Tyr Glu
            20                  25                  30

Lys Thr Cys Lys Asp Val Glu Lys Ile Val Asn Ser Ile Val Val Asn
        35                  40                  45

Ser Ile Lys Asp Asn Arg Gly Lys Gly Ala Gly Leu Val Arg Leu Leu
    50                  55                  60

Phe His Asp Cys Phe Val Arg Gly Cys Asp Ala Ser Val Leu Leu Glu
65                  70                  75                  80

Lys Ser Glu Met Asn Arg Gln Pro Glu Lys Glu Ser Pro Ala Asn Ile
                85                  90                  95

Gly Ile Arg Gly Met Asp Val Ile Asp Ala Ile Lys Ala Val Leu Glu
            100                 105                 110

Ala Arg Cys Pro Asn Thr Val Ser Cys Ala Asp Ile Ile Ala Tyr Ala
        115                 120                 125

Ala Arg Asp Ala Ser Arg Tyr Leu Ser His Gly Gly Val Asp Phe Pro
    130                 135                 140

Val Pro Ala Gly Arg Leu Asp Gly Val Val Ser Arg Ser Arg Asp Ala
145                 150                 155                 160

Asp Ala Phe Leu Pro Asp Ala Ala Ala Asn Leu Thr Asp Leu Val Arg
                165                 170                 175

Asn Phe Arg Arg Lys Asn Phe Thr Val Glu Glu Leu Val Ile Leu Ser
            180                 185                 190

Gly Ala His Ser Ile Gly Val Thr His Cys Thr Ser Phe Ala Gly Arg
        195                 200                 205

Leu Thr Ala Pro Asp Ala Gln Ile Asn Pro Gly Tyr Arg Ser Leu Leu
    210                 215                 220

Val Ser Lys Cys Gly Gly Val Ser Pro Thr Pro Ala Asn Asn His Val
225                 230                 235                 240

Val Val Asn Asn Val Arg Asp Glu Asp Gly Ala Ala Val Ala Arg Val
                245                 250                 255

Met Pro Gly Phe Ala Ala Arg Val Arg Lys Ala Arg Asp Tyr Leu Asp
            260                 265                 270

Asn Ser Tyr Tyr His Asn Asn Leu Ala Met Ala Val Thr Phe His Ala
        275                 280                 285

Asp Trp Ala Leu Leu Thr Gly Lys Glu Ala Arg Gly His Val Val Glu
    290                 295                 300

Tyr Ala Lys Asn Ala Thr Leu Trp Asn Val Asp Phe Gly Asp Ala Leu
305                 310                 315                 320

Val Lys Leu Ser Lys Leu Pro Met Pro Ala Gly Ser Lys Gly Glu Ile
                325                 330                 335

Arg Ala Lys Cys Ser Ala Val Asn Gly Tyr His His
            340                 345

<210> 35
<211> 1250
<212> DNA
<213> Oryza sativa

<220>
<221> CDS
<222> (31)..(1101)

<220>
<223> c52903

<400> 35
   agccagttag agcttgcttg gcaaaaagat atg gcc ggt cag cgt gtg gtg gcc 54
   Met Ala Gly Gln Arg Val Val Ala
   1 5
   gcg gtg gcc gtc gct ctg ggc gtg tgc ctg ctg cag ctg ccg gcg gcg 102
   Ala Val Ala Val Ala Leu Gly Val Cys Leu Leu Gln Leu Pro Ala Ala
   10 15 20
   agc cgc ggc cag ctg cag gtg ggg ttc tac aac acc agc tgc ccc aat 150
   Ser Arg Gly Gln Leu Gln Val Gly Phe Tyr Asn Thr Ser Cys Pro Asn
   25 30 35 40
   gcc gag acg ctg gtc cgg cag gcc gtc acc aac gcc ttc gcc aac gac 198
   Ala Glu Thr Leu Val Arg Gln Ala Val Thr Asn Ala Phe Ala Asn Asp
   45 50 55
   tcc ggc atc gcc gcc ggc ctc atc cgc ctc cat ttc cac gac tgc ttc 246
   Ser Gly Ile Ala Ala Gly Leu Ile Arg Leu His Phe His Asp Cys Phe
   60 65 70
   gtc aga ggt tgc gac gcg tcg gtg ctg ctg acg tcg ccc aac aac acg 294
   Val Arg Gly Cys Asp Ala Ser Val Leu Leu Thr Ser Pro Asn Asn Thr
   75 80 85
   gcg gag cgc gac gcg gcg ccg aac aac ccc agc ctc cgt ggc ttc cag 342
   Ala Glu Arg Asp Ala Ala Pro Asn Asn Pro Ser Leu Arg Gly Phe Gln
   90 95 100
   gtg atc gac gcc gcc aag gcc gcc gtc gag cag agc tgc gcg cgc acg 390
   Val Ile Asp Ala Ala Lys Ala Ala Val Glu Gln Ser Cys Ala Arg Thr
   105 110 115 120
   gtg tcc tgc gcc gac atc gtc gcg ttc gcc gcc cgc gac agc gtc aac 438
   Val Ser Cys Ala Asp Ile Val Ala Phe Ala Ala Arg Asp Ser Val Asn
   125 130 135
   ctc acc ggc ggc gtc tcc tac cag gtc ccc tcc ggc cgc cgc gac ggc 486
   Leu Thr Gly Gly Val Ser Tyr Gln Val Pro Ser Gly Arg Arg Asp Gly
   140 145 150
   aac gtc tcc gtc gcc cag gac gcc atc gac aac ctc ccc cag ccc acc 534
   Asn Val Ser Val Ala Gln Asp Ala Ile Asp Asn Leu Pro Gln Pro Thr
   155 160 165
   ttc acc gcc gcc cag ctc gtc gcc agc ttc gcc aac aag tcg ctc acc 582
   Phe Thr Ala Ala Gln Leu Val Ala Ser Phe Ala Asn Lys Ser Leu Thr
   170 175 180
   gcc gag gag atg gtc gtc ctc tcc ggc gcc cac acc gtc ggc cgc tcc 630
   Ala Glu Glu Met Val Val Leu Ser Gly Ala His Thr Val Gly Arg Ser
   185 190 195 200
   ttc tgc tcc tcc ttc ctc gcc cgc atc tgg aac aac acc acc ccc atc 678
   Phe Cys Ser Ser Phe Leu Ala Arg Ile Trp Asn Asn Thr Thr Pro Ile
   205 210 215
   gtg gac acg ggg ctg agc ccg ggg tac gcg gcg ctg ctg agg gcg ctg 726
   Val Asp Thr Gly Leu Ser Pro Gly Tyr Ala Ala Leu Leu Arg Ala Leu
   220 225 230
   tgc ccg tcg aac gcg tcg gcg acg gcg acg acg gcg atc gac gtg agc 774
   Cys Pro Ser Asn Ala Ser Ala Thr Ala Thr Thr Ala Ile Asp Val Ser
   235 240 245
   acg ccg gcg acg ctg gac aac aac tac tac aag ctg ctg ccg ctc aac 822
   Thr Pro Ala Thr Leu Asp Asn Asn Tyr Tyr Lys Leu Leu Pro Leu Asn
   250 255 260
   ctg ggg ctc ttc ttc tcc gac aac cag ctg cgg gtg aac gcg acg ctg 870
   Leu Gly Leu Phe Phe Ser Asp Asn Gln Leu Arg Val Asn Ala Thr Leu
   265 270 275 280
   ggc gcg tcg gtg agc agc ttc gcg gcg aac gag acg ctg tgg aag gag 918
   Gly Ala Ser Val Ser Ser Phe Ala Ala Asn Glu Thr Leu Trp Lys Glu
   285 290 295
   aag ttc gtc gcc gcc atg gtc aag atg ggg agc atc gag gtg ctc acc 966
   Lys Phe Val Ala Ala Met Val Lys Met Gly Ser Ile Glu Val Leu Thr
   300 305 310
   ggc agc cag ggc gag gtc agg ctc aac tgc agc gtc gtc aac aac cgg 1014
   Gly Ser Gln Gly Glu Val Arg Leu Asn Cys Ser Val Val Asn Asn Arg
   315 320 325
   tca tcc tcc tcc gcc gcc ggg atg gag acg tcg tac cac tac tac tcc 1062
   Ser Ser Ser Ser Ala Ala Gly Met Glu Thr Ser Tyr His Tyr Tyr Ser
   330 335 340
   gga tcc acc atg tcc gtc gac gag gtc gcg tcg agc tga tcgacgacga 1111
   Gly Ser Thr Met Ser Val Asp Glu Val Ala Ser Ser
   345 350 355
   tgtacaacct gtttggtgtt aattaggatg tgtgtatccc acgaggtcac gacgatgtag 1171
   aagccgggaa ggtcaaaaca ttaattctat gatgtatgaa aaaaagcgtg tctatgtaaa 1231
   attttatata tttgtctgc 1250

<210> 36
<211> 356
<212> PRT
<213> Oryza sativa
<223> c52903

<400> 36
Met Ala Gly Gln Arg Val Val Ala Ala Val Ala Val Ala Leu Gly Val
1               5                   10                  15

Cys Leu Leu Gln Leu Pro Ala Ala Ser Arg Gly Gln Leu Gln Val Gly
            20                  25                  30

Phe Tyr Asn Thr Ser Cys Pro Asn Ala Glu Thr Leu Val Arg Gln Ala
        35                  40                  45

Val Thr Asn Ala Phe Ala Asn Asp Ser Gly Ile Ala Ala Gly Leu Ile
    50                  55                  60

Arg Leu His Phe His Asp Cys Phe Val Arg Gly Cys Asp Ala Ser Val
65                  70                  75                  80

Leu Leu Thr Ser Pro Asn Asn Thr Ala Glu Arg Asp Ala Ala Pro Asn
                85                  90                  95

Asn Pro Ser Leu Arg Gly Phe Gln Val Ile Asp Ala Ala Lys Ala Ala
            100                 105                 110

Val Glu Gln Ser Cys Ala Arg Thr Val Ser Cys Ala Asp Ile Val Ala
        115                 120                 125

Phe Ala Ala Arg Asp Ser Val Asn Leu Thr Gly Gly Val Ser Tyr Gln
    130                 135                 140

Val Pro Ser Gly Arg Arg Asp Gly Asn Val Ser Val Ala Gln Asp Ala
145                 150                 155                 160

Ile Asp Asn Leu Pro Gln Pro Thr Phe Thr Ala Ala Gln Leu Val Ala
                165                 170                 175

Ser Phe Ala Asn Lys Ser Leu Thr Ala Glu Glu Met Val Val Leu Ser
            180                 185                 190

Gly Ala His Thr Val Gly Arg Ser Phe Cys Ser Ser Phe Leu Ala Arg
        195                 200                 205

Ile Trp Asn Asn Thr Thr Pro Ile Val Asp Thr Gly Leu Ser Pro Gly
    210                 215                 220

Tyr Ala Ala Leu Leu Arg Ala Leu Cys Pro Ser Asn Ala Ser Ala Thr
225                 230                 235                 240

Ala Thr Thr Ala Ile Asp Val Ser Thr Pro Ala Thr Leu Asp Asn Asn
                245                 250                 255

Tyr Tyr Lys Leu Leu Pro Leu Asn Leu Gly Leu Phe Phe Ser Asp Asn
            260                 265                 270

Gln Leu Arg Val Asn Ala Thr Leu Gly Ala Ser Val Ser Ser Phe Ala
        275                 280                 285

Ala Asn Glu Thr Leu Trp Lys Glu Lys Phe Val Ala Ala Met Val Lys
    290                 295                 300

Met Gly Ser Ile Glu Val Leu Thr Gly Ser Gln Gly Glu Val Arg Leu
305                 310                 315                 320

Asn Cys Ser Val Val Asn Asn Arg Ser Ser Ser Ser Ala Ala Gly Met
                325                 330                 335

Glu Thr Ser Tyr His Tyr Tyr Ser Gly Ser Thr Met Ser Val Asp Glu
            340                 345                 350

Val Ala Ser Ser
        355

<210> 37
<211> 1233
<212> DNA
<213> Oryza sativa

<220>
<221> CDS
<222> (34)..(1089)

<220>
<223> r2329

<400> 37

agcagctagc tatagctaag ctctgagcga tcc atg gcc atg aag tgc ctc ttc 54
Met Ala Met Lys Cys Leu Phe
1               5

ctc ttc ttc gcc ttc ctc gtc gcc ttc ttc ccc ggc gcc gcc gtc ggc 102
Leu Phe Phe Ala Phe Leu Val Ala Phe Phe Pro Gly Ala Ala Val Gly
        10                  15                  20

gcc ggg ctg aag gtc ggg ttc tac aac aag acg tgc ccg tcg gcg gag 150
Ala Gly Leu Lys Val Gly Phe Tyr Asn Lys Thr Cys Pro Ser Ala Glu
    25                  30                  35

cgc ctg gtg cag cag gcg gtg gcc gcc gcg ttc aag aac aac agc ggc 198
Arg Leu Val Gln Gln Ala Val Ala Ala Ala Phe Lys Asn Asn Ser Gly
40                  45                  50                  55

gtc gcc ccc ggc ctc atc cgc ctg cac ttc cat gac tgc ttt gtc aga 246
Val Ala Pro Gly Leu Ile Arg Leu His Phe His Asp Cys Phe Val Arg
                60                  65                  70

ggc tgc gac gcc tcg gtt ttg atc gac ggg aac gac acc gag aag acc 294
Gly Cys Asp Ala Ser Val Leu Ile Asp Gly Asn Asp Thr Glu Lys Thr
            75                  80                  85

gcg cca cca aac aac ccc agc ctc cgc gga ttc gag gtg atc gac gcc 342
Ala Pro Pro Asn Asn Pro Ser Leu Arg Gly Phe Glu Val Ile Asp Ala
        90                  95                  100

gcc aag gcc gcc gtc gag gcg gcg tgc ccg cgt gtc gtc tcc tgc gcc 390
Ala Lys Ala Ala Val Glu Ala Ala Cys Pro Arg Val Val Ser Cys Ala
    105                 110                 115

gac atc ctc gcc ttc gcc gcc cgc gac agc gtc gcc ctc acc ggc aac 438
Asp Ile Leu Ala Phe Ala Ala Arg Asp Ser Val Ala Leu Thr Gly Asn
120                 125                 130                 135

gtc act tac aag gtc ccc gcc ggc cgc cgc gac ggc aac gtc tcc atc 486
Val Thr Tyr Lys Val Pro Ala Gly Arg Arg Asp Gly Asn Val Ser Ile
                140                 145                 150

gcc cag gac gcg ctc gac aac ctg ccc cct ccc acc ttc aac gcc acg 534
Ala Gln Asp Ala Leu Asp Asn Leu Pro Pro Pro Thr Phe Asn Ala Thr
            155                 160                 165

gag ctc gtc ggc cgc ttc gcc aac aag tcg ctc acc gcg gag gac atg 582
Glu Leu Val Gly Arg Phe Ala Asn Lys Ser Leu Thr Ala Glu Asp Met
        170                 175                 180

gtg gtg ctc tcc ggc gcc cac acc atc ggc gtc tcc cac tgc gac tcc 630
Val Val Leu Ser Gly Ala His Thr Ile Gly Val Ser His Cys Asp Ser
    185                 190                 195

ttc acc agc cgc ctc tac aac ttc acc ggc gtc ggc gac gcc gac ccg 678
Phe Thr Ser Arg Leu Tyr Asn Phe Thr Gly Val Gly Asp Ala Asp Pro
200                 205                 210                 215

gcg atc agc gcc gcc tac gcg ttc ctc ctc cgc gcc gtg tgc ccc tcc 726
Ala Ile Ser Ala Ala Tyr Ala Phe Leu Leu Arg Ala Val Cys Pro Ser
                220                 225                 230

aac agc agc cag ttc ttc ccc aac acc acg gtg gac atg gac gtg atc 774
Asn Ser Ser Gln Phe Phe Pro Asn Thr Thr Val Asp Met Asp Val Ile
            235                 240                 245

acc ccg gcg gcg ctc gac aac aag tac tac gtc ggg gtc gcc aac aac    822
Thr Pro Ala Ala Leu Asp Asn Lys Tyr Tyr Val Gly Val Ala Asn Asn
        250                 255                 260

ctg ggc ctc ttc acg tcg gac cac gcg ctg ctc acc aac gcc acg ctc    870
Leu Gly Leu Phe Thr Ser Asp His Ala Leu Leu Thr Asn Ala Thr Leu
    265                 270                 275

agg gcg tcg gtg gac gag ttc gtc aag agc gag acg cgg tgg aag agc    918
Arg Ala Ser Val Asp Glu Phe Val Lys Ser Glu Thr Arg Trp Lys Ser
280                 285                 290                 295

aag ttc gtg aag gcc atg gtg aag atg ggc ggc atc gag gtg aag acc    966
Lys Phe Val Lys Ala Met Val Lys Met Gly Gly Ile Glu Val Lys Thr
                300                 305                 310

ggg acg acg cag ggc gag gtc agg ctc aac tgc agg gtc gtc aac aag    1014
Gly Thr Thr Gln Gly Glu Val Arg Leu Asn Cys Arg Val Val Asn Lys
            315                 320                 325

agg agc gcc aac gct gag ctc gag ctc gag ctc gcc gcc gcc atg gat    1062
Arg Ser Ala Asn Ala Glu Leu Glu Leu Glu Leu Ala Ala Ala Met Asp
        330                 335                 340

gat ggg gat gaa gtg gca gcg agc taa ttaattaagg tggtagtagc          1109
Asp Gly Asp Glu Val Ala Ala Ser
    345                 350

tgatctgatg tcgcgtgttc ttcagaggtt tcagcaataa gtttgtgatc ggtgttgtta 1169

cttgttagat gtatgctgct gctgttttta acgagaataa gagttgctgg agataagttt 1229

tttt                                                               1233

<210> 38
<211> 351
<212> PRT
<213> Oryza sativa
<223> r2329

<400> 38
Met Ala Met Lys Cys Leu Phe Leu Phe Phe Ala Phe Leu Val Ala Phe
  1               5                  10                  15

Phe Pro Gly Ala Ala Val Gly Ala Gly Leu Lys Val Gly Phe Tyr Asn
             20                  25                  30

Lys Thr Cys Pro Ser Ala Glu Arg Leu Val Gln Gln Ala Val Ala Ala
         35                  40                  45

Ala Phe Lys Asn Asn Ser Gly Val Ala Pro Gly Leu Ile Arg Leu His
     50                  55                  60

Phe His Asp Cys Phe Val Arg Gly Cys Asp Ala Ser Val Leu Ile Asp
 65                  70                  75                  80

Gly Asn Asp Thr Glu Lys Thr Ala Pro Pro Asn Asn Pro Ser Leu Arg
                 85                  90                  95

Gly Phe Glu Val Ile Asp Ala Ala Lys Ala Ala Val Glu Ala Ala Cys
            100                 105                 110

Pro Arg Val Val Ser Cys Ala Asp Ile Leu Ala Phe Ala Ala Arg Asp

115                 120                 125

Ser Val Ala Leu Thr Gly Asn Val Thr Tyr Lys Val Pro Ala Gly Arg
    130                 135                 140

Arg Asp Gly Asn Val Ser Ile Ala Gln Asp Ala Leu Asp Asn Leu Pro
145                 150                 155                 160

Pro Pro Thr Phe Asn Ala Thr Glu Leu Val Gly Arg Phe Ala Asn Lys
                165                 170                 175

Ser Leu Thr Ala Glu Asp Met Val Val Leu Ser Gly Ala His Thr Ile
            180                 185                 190

Gly Val Ser His Cys Asp Ser Phe Thr Ser Arg Leu Tyr Asn Phe Thr
        195                 200                 205

Gly Val Gly Asp Ala Asp Pro Ala Ile Ser Ala Ala Tyr Ala Phe Leu
    210                 215                 220

Leu Arg Ala Val Cys Pro Ser Asn Ser Ser Gln Phe Phe Pro Asn Thr
225                 230                 235                 240

Thr Val Asp Met Asp Val Ile Thr Pro Ala Ala Leu Asp Asn Lys Tyr
                245                 250                 255

Tyr Val Gly Val Ala Asn Asn Leu Gly Leu Phe Thr Ser Asp His Ala
            260                 265                 270

Leu Leu Thr Asn Ala Thr Leu Arg Ala Ser Val Asp Glu Phe Val Lys
        275                 280                 285

Ser Glu Thr Arg Trp Lys Ser Lys Phe Val Lys Ala Met Val Lys Met
    290                 295                 300

Gly Gly Ile Glu Val Lys Thr Gly Thr Thr Gln Gly Glu Val Arg Leu
305                 310                 315                 320

Asn Cys Arg Val Val Asn Lys Arg Ser Ala Asn Ala Glu Leu Glu Leu
                325                 330                 335

Glu Leu Ala Ala Ala Met Asp Asp Gly Asp Glu Val Ala Ala Ser
            340                 345                 350


<210> 39
<211> 1433
<212> DNA
<213> Oryza sativa

<220>
<221> CDS
<222> (52)..(1062)

<220>
<223> s11222

<400> 39
cacacgacac gaccgtatag gagtagtcgt gtagcagcta gctagctagc a atg gcg    57
                                                         Met Ala
                                                         1

gct tct tct tct tct tct tcc tct ctg gcg gtg gtg gtg gtg gcg gcg    105
Ala Ser Ser Ser Ser Ser Ser Ser Leu Ala Val Val Val Val Ala Ala

        5                   10                  15

gcg gtg gcg ctg gtc gcc ggc ggc ggc gcg gcg gtg gcg cag ctg tgc 153
Ala Val Ala Leu Val Ala Gly Gly Gly Ala Ala Val Ala Gln Leu Cys
    20                  25                  30

gag gag tac tac gac tgc acg tgc ccc gac gcg tac gac atc gtg cgg 201
Glu Glu Tyr Tyr Asp Cys Thr Cys Pro Asp Ala Tyr Asp Ile Val Arg
35                  40                  45                  50

cgg gtg ctg atc gat gcg cac cgg agc gac gcc cgg atc ttc gcg agc 249
Arg Val Leu Ile Asp Ala His Arg Ser Asp Ala Arg Ile Phe Ala Ser
                55                  60                  65

ctg atc cgg ctc cat ttc cac gac tgc ttc gtg cag ggg tgc gac gcg 297
Leu Ile Arg Leu His Phe His Asp Cys Phe Val Gln Gly Cys Asp Ala
            70                  75                  80

tcg ctg ctg ctg gac agc gtc ccc ggg atg ccg tcg gag aag acg tcg 345
Ser Leu Leu Leu Asp Ser Val Pro Gly Met Pro Ser Glu Lys Thr Ser
        85                  90                  95

ccg ccc aac aac aac tcc gcg agg gga ttc ccc gtc gta gac gac gtc 393
Pro Pro Asn Asn Asn Ser Ala Arg Gly Phe Pro Val Val Asp Asp Val
    100                 105                 110

aag gcc gcg ctc gag gac gcc tgc ccc ggc gtt gtc tcc tgc gcc gac 441
Lys Ala Ala Leu Glu Asp Ala Cys Pro Gly Val Val Ser Cys Ala Asp
115                 120                 125                 130

atc ctc gcc ctc gcc gcc gag atc tcc gtc gag ctg tca ggt ggg ccc 489
Ile Leu Ala Leu Ala Ala Glu Ile Ser Val Glu Leu Ser Gly Gly Pro
                135                 140                 145

ggg tgg gga gtg ctg ctg ggg cgg ctc gac ggc aag acc tcc gac ttc 537
Gly Trp Gly Val Leu Leu Gly Arg Leu Asp Gly Lys Thr Ser Asp Phe
            150                 155                 160

aat ggc tcc ctc aac ctg ccg gcc ccc acc gac aac ctc acc gtc ctc 585
Asn Gly Ser Leu Asn Leu Pro Ala Pro Thr Asp Asn Leu Thr Val Leu
        165                 170                 175

cgc caa aag ttc gcc gcc ctc aac ctc aac gac gtc gac ctc gtc gcc 633
Arg Gln Lys Phe Ala Ala Leu Asn Leu Asn Asp Val Asp Leu Val Ala
    180                 185                 190

ctc tca ggt ggg cac acg ttc ggg agg gtg cag tgc cag ttc gtg acg 681
Leu Ser Gly Gly His Thr Phe Gly Arg Val Gln Cys Gln Phe Val Thr
195                 200                 205                 210

gac agg ctg tac aac ttc agc aac acg ggg agg ccg gac ccc acc atg 729
Asp Arg Leu Tyr Asn Phe Ser Asn Thr Gly Arg Pro Asp Pro Thr Met
                215                 220                 225

gac gcc gcc tac cgg agc ttc ctg tcg cag cgg tgc ccg ccc aac ggg 777
Asp Ala Ala Tyr Arg Ser Phe Leu Ser Gln Arg Cys Pro Pro Asn Gly
            230                 235                 240

ccg ccg gcg gcg ctc aac gac ctg gac ccg acg acg ccg gac acc ttc 825
Pro Pro Ala Ala Leu Asn Asp Leu Asp Pro Thr Thr Pro Asp Thr Phe
        245                 250                 255

gac aac cac tac tac acc aac atc gag gtc aac cgc ggc ttc ctc cag 873
Asp Asn His Tyr Tyr Thr Asn Ile Glu Val Asn Arg Gly Phe Leu Gln

260                 265                 270

tcg gac cag gag ctc aag tcg gcg ccg gag gcg acc ggg acg acg gcg     921
Ser Asp Gln Glu Leu Lys Ser Ala Pro Glu Ala Thr Gly Thr Thr Ala
275                 280                 285                 290

ccc atc gtc gac cgc ttc gcc acc agc cag gcc gcc ttc ttc cgc agc     969
Pro Ile Val Asp Arg Phe Ala Thr Ser Gln Ala Ala Phe Phe Arg Ser
                295                 300                 305

ttc gcg cag tcc atg atc aac atg ggc aac ctc tcc cct gtc act gac     1017
Phe Ala Gln Ser Met Ile Asn Met Gly Asn Leu Ser Pro Val Thr Asp
            310                 315                 320

cct tcc ctg ggt gag gtc cgg acc aac tgc aga agg gtc aat taa         1062
Pro Ser Leu Gly Glu Val Arg Thr Asn Cys Arg Arg Val Asn
        325                 330                 335

ttatcacttc atgtatgacg ttggttagtt taaaattgaa gtaaccaacg tcatcatgtc 1122

aaacataatt aattacgtat gtgtcgatta ttattgttgt tattattagc aaaatgagag 1182

agatttattt gtgcatagtt aattaagcgc gcttgcattt gtgagaaata ttagtagtcc 1242

agcttaatta atcaaggaga agaggatgga aaaaagaatg tacatgtgtt caatttggat 1302

gtgtgatcaa ggatctatta tgatcacgcg catccagctt aataattggc caattggttg 1362

accaatgtaa ttgtacgtac catgattgtg tttgttccaa attaaaggtg caataaacgg 1422

tttacatgtt t                                                      1433

<210> 40
<211> 336
<212> PRT
<213> Oryza sativa
<223> s11222

<400> 40
Met Ala Ala Ser Ser Ser Ser Ser Ser Ser Leu Ala Val Val Val Val
1               5                   10                  15

Ala Ala Ala Val Ala Leu Val Ala Gly Gly Gly Ala Ala Val Ala Gln
            20                  25                  30

Leu Cys Glu Glu Tyr Tyr Asp Cys Thr Cys Pro Asp Ala Tyr Asp Ile
        35                  40                  45

Val Arg Arg Val Leu Ile Asp Ala His Arg Ser Asp Ala Arg Ile Phe
    50                  55                  60

Ala Ser Leu Ile Arg Leu His Phe His Asp Cys Phe Val Gln Gly Cys
65                  70                  75                  80

Asp Ala Ser Leu Leu Leu Asp Ser Val Pro Gly Met Pro Ser Glu Lys
                85                  90                  95

Thr Ser Pro Pro Asn Asn Asn Ser Ala Arg Gly Phe Pro Val Val Asp
            100                 105                 110

Asp Val Lys Ala Ala Leu Glu Asp Ala Cys Pro Gly Val Val Ser Cys
        115                 120                 125

Ala Asp Ile Leu Ala Leu Ala Ala Glu Ile Ser Val Glu Leu Ser Gly

130                 135                 140

Gly Pro Gly Trp Gly Val Leu Leu Gly Arg Leu Asp Gly Lys Thr Ser
145                 150                 155                 160

Asp Phe Asn Gly Ser Leu Asn Leu Pro Ala Pro Thr Asp Asn Leu Thr
                165                 170                 175

Val Leu Arg Gln Lys Phe Ala Ala Leu Asn Leu Asn Asp Val Asp Leu
            180                 185                 190

Val Ala Leu Ser Gly Gly His Thr Phe Gly Arg Val Gln Cys Gln Phe
        195                 200                 205

Val Thr Asp Arg Leu Tyr Asn Phe Ser Asn Thr Gly Arg Pro Asp Pro
    210                 215                 220

Thr Met Asp Ala Ala Tyr Arg Ser Phe Leu Ser Gln Arg Cys Pro Pro
225                 230                 235                 240

Asn Gly Pro Pro Ala Ala Leu Asn Asp Leu Asp Pro Thr Thr Pro Asp
                245                 250                 255

Thr Phe Asp Asn His Tyr Tyr Thr Asn Ile Glu Val Asn Arg Gly Phe
            260                 265                 270

Leu Gln Ser Asp Gln Glu Leu Lys Ser Ala Pro Glu Ala Thr Gly Thr
        275                 280                 285

Thr Ala Pro Ile Val Asp Arg Phe Ala Thr Ser Gln Ala Ala Phe Phe
    290                 295                 300

Arg Ser Phe Ala Gln Ser Met Ile Asn Met Gly Asn Leu Ser Pro Val
305                 310                 315                 320

Thr Asp Pro Ser Leu Gly Glu Val Arg Thr Asn Cys Arg Arg Val Asn
                325                 330                 335

<210> 41
<211> 1445
<212> DNA
<213> Oryza sativa

<220>
<221> CDS
<222> (74)..(1105)

<220>
<223> S14082

<400> 41
ctactatatg caagttaagt tagtgcactg aaggagagcc tgagatacgt cgtcgtacac 60

acttgattaa tta atg gcg tgg agg aac agc ggg cgg gtg aag gtg atg     109
              Met Ala Trp Arg Asn Ser Gly Arg Val Lys Val Met
              1               5                   10

aac agg agg agc agc agg atg gca gtc atg gca gcc gcc gtg ctg gcc     157
Asn Arg Arg Ser Ser Arg Met Ala Val Met Ala Ala Ala Val Leu Ala
        15                  20                  25

gtg tgc agc ttt gcg gcg gtg acc atg gcc cag ctg gag atg gac ttc     205

Val Cys Ser Phe Ala Ala Val Thr Met Ala Gln Leu Glu Met Asp Phe
    30                  35                  40

tac agc aag acg tgc ccg aac gtc gag gag atc gtc cgg cgg gag atg 253
Tyr Ser Lys Thr Cys Pro Asn Val Glu Glu Ile Val Arg Arg Glu Met
45                  50                  55                  60

gag gag atc ctc cgg gtg gcg ccg acg ctc gcc ggc ccg ctc ctc cgc 301
Glu Glu Ile Leu Arg Val Ala Pro Thr Leu Ala Gly Pro Leu Leu Arg
                65                  70                  75

ctc cat ttc cac gac tgc ttc gtc agg ggg tgc gac gcg tcg gtg ctg 349
Leu His Phe His Asp Cys Phe Val Arg Gly Cys Asp Ala Ser Val Leu
            80                  85                  90

att gac tcg acg gcc ggc aac gtg gcg gag aag gac gcc aag ccc aac 397
Ile Asp Ser Thr Ala Gly Asn Val Ala Glu Lys Asp Ala Lys Pro Asn
        95                  100                 105

ctc act ctc cgc ggc ttc ggg gcg gtg cag cgg gtc aag gac aag ctc 445
Leu Thr Leu Arg Gly Phe Gly Ala Val Gln Arg Val Lys Asp Lys Leu
    110                 115                 120

aac gcc gcc tgc ccg gcc acc gtc tcc tgc gcc gac gtc ctc gcc ctc 493
Asn Ala Ala Cys Pro Ala Thr Val Ser Cys Ala Asp Val Leu Ala Leu
125                 130                 135                 140

atg gcc cgt gac gcc gtc gtc ctc gcc aac ggg ccc tcc tgg ccc gtc 541
Met Ala Arg Asp Ala Val Val Leu Ala Asn Gly Pro Ser Trp Pro Val
                145                 150                 155

tcg ctc ggc cgc cgc gac ggc cgc ctc tcc atc gcc aac gac acc aac 589
Ser Leu Gly Arg Arg Asp Gly Arg Leu Ser Ile Ala Asn Asp Thr Asn
            160                 165                 170

cag ctg ccg ccc ccc acc gcc aac ttc acc cag ctc tcc cag atg ttc 637
Gln Leu Pro Pro Pro Thr Ala Asn Phe Thr Gln Leu Ser Gln Met Phe
        175                 180                 185

gcc gcc aaa ggc ctc gac gcc aag gac ctc gtc gtc ctc tcc ggc ggc 685
Ala Ala Lys Gly Leu Asp Ala Lys Asp Leu Val Val Leu Ser Gly Gly
    190                 195                 200

cac acg ctc ggc acg gcg cac tgc gcg ctc ttc tcc gac cgc ctc tac 733
His Thr Leu Gly Thr Ala His Cys Ala Leu Phe Ser Asp Arg Leu Tyr
205                 210                 215                 220

aac ttc acc ggc ctg gtg aac gac ggc gac gtg gac ccg gcg ctg gac 781
Asn Phe Thr Gly Leu Val Asn Asp Gly Asp Val Asp Pro Ala Leu Asp
                225                 230                 235

gcg gcg tac atg gcg aag ctc aag gcc aag tgc cgg agc ctg agc gac 829
Ala Ala Tyr Met Ala Lys Leu Lys Ala Lys Cys Arg Ser Leu Ser Asp
            240                 245                 250

aac acc acc ctg tcg gag atg gac ccc ggc agc ttc ctc acc ttc gac 877
Asn Thr Thr Leu Ser Glu Met Asp Pro Gly Ser Phe Leu Thr Phe Asp
        255                 260                 265

gcc agc tac tac cgg ctg gtg gcc aag cgc cgc ggc atc ttc cac tcc 925
Ala Ser Tyr Tyr Arg Leu Val Ala Lys Arg Arg Gly Ile Phe His Ser
    270                 275                 280

gac tcc gcg ctg ctc acc gat ccc gtc acc agg gcc tac gtc gag cgc 973

Asp Ser Ala Leu Leu Thr Asp Pro Val Thr Arg Ala Tyr Val Glu Arg
285                 290                 295                 300

cag gcc acc ggc cac ttc gcc gac gac ttc ttc cgc gac ttc gcc gac    1021
Gln Ala Thr Gly His Phe Ala Asp Asp Phe Phe Arg Asp Phe Ala Asp
                305                 310                 315

tcc atg gtg aag atg agc acc att gac gtg ctc acc ggg gcg cag ggc    1069
Ser Met Val Lys Met Ser Thr Ile Asp Val Leu Thr Gly Ala Gln Gly
            320                 325                 330

gag atc agg aac aag tgc tac gcc atc aac ata taa taaagtaact         1115
Glu Ile Arg Asn Lys Cys Tyr Ala Ile Asn Ile
        335                 340

gcatctgcat gcatgcgcac taatcaaagg ttcgatttat ttggtttctt ccgtcaattt 1175

tttttaattg gtttcagttt atttgcaaaa aagatcgtgt taatttggtt tgggtacagt 1235

acagatgaac gaatccatca ctgcgtacta tgtatatgtg tgtaactgat gtactatgta 1295

cgtagattgc atgccaatgg gtttcttgtt ttgctacttt ttaatttgtt ttttggggtg 1355

cgtggcatct atgttcaact cttgtgaatt cattcatgat atatgtaata agttcaaacg 1415

agacaataac taaaatatac tctctttcac                                  1445

<210> 42
<211> 343
<212> PRT
<213> Oryza sativa

<220>
<223> S14082

<400> 42
Met Ala Trp Arg Asn Ser Gly Arg Val Lys Val Met Asn Arg Arg Ser
1               5                   10                  15

Ser Arg Met Ala Val Met Ala Ala Ala Val Leu Ala Val Cys Ser Phe
            20                  25                  30

Ala Ala Val Thr Met Ala Gln Leu Glu Met Asp Phe Tyr Ser Lys Thr
        35                  40                  45

Cys Pro Asn Val Glu Glu Ile Val Arg Arg Glu Met Glu Glu Ile Leu
    50                  55                  60

Arg Val Ala Pro Thr Leu Ala Gly Pro Leu Leu Arg Leu His Phe His
65                  70                  75                  80

Asp Cys Phe Val Arg Gly Cys Asp Ala Ser Val Leu Ile Asp Ser Thr
                85                  90                  95

Ala Gly Asn Val Ala Glu Lys Asp Ala Lys Pro Asn Leu Thr Leu Arg
            100                 105                 110

Gly Phe Gly Ala Val Gln Arg Val Lys Asp Lys Leu Asn Ala Ala Cys
        115                 120                 125

Pro Ala Thr Val Ser Cys Ala Asp Val Leu Ala Leu Met Ala Arg Asp
    130                 135                 140

Ala Val Val Leu Ala Asn Gly Pro Ser Trp Pro Val Ser Leu Gly Arg
145                 150                 155                 160

Arg Asp Gly Arg Leu Ser Ile Ala Asn Asp Thr Asn Gln Leu Pro Pro
                165                 170                 175

Pro Thr Ala Asn Phe Thr Gln Leu Ser Gln Met Phe Ala Ala Lys Gly
            180                 185                 190

Leu Asp Ala Lys Asp Leu Val Val Leu Ser Gly Gly His Thr Leu Gly
        195                 200                 205

Thr Ala His Cys Ala Leu Phe Ser Asp Arg Leu Tyr Asn Phe Thr Gly
    210                 215                 220

Leu Val Asn Asp Gly Asp Val Asp Pro Ala Leu Asp Ala Ala Tyr Met
225                 230                 235                 240

Ala Lys Leu Lys Ala Lys Cys Arg Ser Leu Ser Asp Asn Thr Thr Leu
                245                 250                 255

Ser Glu Met Asp Pro Gly Ser Phe Leu Thr Phe Asp Ala Ser Tyr Tyr
            260                 265                 270

Arg Leu Val Ala Lys Arg Arg Gly Ile Phe His Ser Asp Ser Ala Leu
        275                 280                 285

Leu Thr Asp Pro Val Thr Arg Ala Tyr Val Glu Arg Gln Ala Thr Gly
    290                 295                 300

His Phe Ala Asp Asp Phe Phe Arg Asp Phe Ala Asp Ser Met Val Lys
305                 310                 315                 320

Met Ser Thr Ile Asp Val Leu Thr Gly Ala Gln Gly Glu Ile Arg Asn
                325                 330                 335

Lys Cys Tyr Ala Ile Asn Ile
            340


<210> 43
<211> 20
<212> DNA
<213> Oryza sativa

<220>
<223> C52903FP1

<400> 43
tgtgcccgtc gaacgcgtcg   20

<210> 44
<211> 18
<212> DNA
<213> Oryza sativa

<220>
<223> C62847FP1

<400> 44
tgcccgctca gctacagc     18

<210> 45
<211> 20

<212> DNA
<213> Oryza sativa

<220>
<223> prxRPAFP1

<400> 45
aacctccaga gcctctgtgc   20

<210> 46
<211> 20
<212> DNA
<213> Oryza sativa

<220>
<223> prxRPARP1

<400> 46
aaggcacata cattcagttc   20

<210> 47
<211> 18
<212> DNA
<213> Oryza sativa

<220>
<223> R0317F1

<400> 47
tcaagacgtt cgacctgg     18

<210> 48
<211> 18
<212> DNA
<213> Oryza sativa

<220>
<223> R1420FP1

<400> 48
ttcacctctg acgcggcg     18

<210> 49
<211> 20
<212> DNA
<213> Oryza sativa

<220>
<223> R2184FP1

<400> 49
cgacaacaag tactacttcg   20

<210> 50
<211> 18
<212> DNA
<213> Oryza sativa

<220>
<223> R2391FP2

<400> 50
gcctctacaa cgagacgg     18

<210> 51
<211> 17
<212> DNA
<213> Oryza sativa

<220>
<223> R2576F1

<400> 51
tcaaggccaa ctgccca     17

<210> 52
<211> 20
<212> DNA
<213> Oryza sativa

<220>
<223> S10927FP1

<400> 52
cgacctcgcc gcgctgtccg    20

<210> 53
<211> 20
<212> DNA
<213> Oryza sativa

<220>
<223> S11222FP1

<400> 53
gacgacggcg cccatcgtcg    20

<210> 54
<211> 20
<212> DNA
<213> Oryza sativa

<220>
<223> S13316FP1

<400> 54
gcgacaacac gacgctggcg    20

<210> 55
<211> 20
<212> DNA
<213> Oryza sativa

<220>
<223> S14082FP1

<400> 55
tcttccactc cgactccgcg    20

<210> 56
<211> 20
<212> DNA
<213> Oryza sativa

<220>
<223> S14493FP1

<400> 56
cggcggcgac accaacctgg    20

<210> 57
<211> 18
<212> DNA
<213> Oryza sativa

<220>
<223> S4325F1

<400> 57
atgttcagcg ccaagggc      18

<210> 58
<211> 20
<212> DNA
<213> Oryza sativa

<220>
<223> prxRPNFP1

<400> 58
cctcgtctcc agctccggcg    20

<210> 59
<211> 20
<212> DNA
<213> Oryza sativa

<220>
<223> prxRPNRP1

<400> 59
ttaaaccata tggcagttgc    20

### SUMMARY PARAGRAPH

The present invention is defined in the claims and the accompanying description.

For convenience other aspects of the present invention are presented herein by way of numbered paragraphs (para.s).
1. A set of peroxidase genes useful for evaluation of a characteristic of plants, comprising:
   (A1) a subset of root-expression constitutive genes including at least one type of gene selected from the gene group consisting of:
      (1) DNA having a sequence of SEQ ID NO: 1, a homolog thereof, or a fragment thereof;
      (2) DNA having a sequence of SEQ ID NO: 3, a homolog thereof, or a fragment thereof;
      (3) DNA having a sequence of SEQ ID NO: 5, a homolog thereof, or a fragment thereof;
      (4) DNA having a sequence of SEQ ID NO: 7, a homolog thereof, or a fragment thereof;
      (5) DNA having a sequence of SEQ ID NO: 9, a homolog thereof, or a fragment thereof;
      (6) DNA having a sequence of SEQ ID NO: 11, a homolog thereof, or a fragment thereof;
      (7) DNA having a sequence of SEQ ID NO: 13, a homolog thereof, or a fragment thereof;
      (8) DNA having a sequence of SEQ ID NO: 15, a homolog thereof, or a fragment thereof;
      (9) DNA having a sequence of SEQ ID NO: 17, a homolog thereof, or a fragment thereof;
      (10) DNA having a sequence of SEQ ID NO: 19, a homolog thereof, or a fragment thereof; and
      (11) DNA having a sequence of SEQ ID NO: 21, a homolog thereof, or a fragment thereof;
   (A2) a subset of aerial-expression constitutive genes including at least one type of gene selected from the gene group consisting of:
      (12) DNA having a sequence of SEQ ID NO: 23, a homolog thereof, or a fragment thereof; and
      (13) DNA having a sequence of SEQ ID NO: 25, a homolog thereof, or a fragment thereof;
   (B1) a subset of root-expression stress-inducible genes including at least one type of gene selected from the gene group consisting of:
      (14) DNA having a sequence of SEQ ID NO: 27, a homolog thereof, or a fragment thereof;
      (15) DNA having a sequence of SEQ ID NO: 29, a homolog thereof, or a fragment thereof;
      (16) DNA having a sequence of SEQ ID NO: 31, a homolog thereof, or a fragment thereof;
      (17) DNA having a sequence of SEQ ID NO: 33, a homolog thereof, or a fragment thereof; and
      (18) DNA having a sequence of SEQ ID NO: 35, a homolog thereof, or a fragment thereof;
   (B2) a subset of aerial-expression stress-inducible genes including at least one type of gene selected from the gene group consisting of:
      (19) DNA having a sequence of SEQ ID NO: 37, a homolog thereof, or a fragment thereof; and
      (20) DNA having a sequence of SEQ ID NO: 39, a homolog thereof, or a fragment thereof;
   (C) a gene below:
      (21) DNA having a sequence of SEQ ID NO: 41, a homolog thereof, or a fragment thereof.
2. A peroxidase gene, wherein the peroxidase gene is any of:
   (a) peroxidase DNA having a sequence of position 50 to 1021 in SEQ ID NO: 3;
   (b) peroxidase DNA having a sequence of position 108 to 1109 in SEQ ID NO: 5;
   (c) peroxidase DNA having a sequence of position 66 to 1046 in SEQ ID NO: 7;
   (d) peroxidase DNA having a sequence of position 71 to 1078 in SEQ ID NO: 9;
   (e) peroxidase DNA having a sequence of position 134 to 1108 in SEQ ID NO: 11;
   (f) peroxidase DNA having a sequence of position 75 to 1058 in SEQ ID NO: 13;
   (g) peroxidase DNA having a sequence of position 136 to 1147 in SEQ ID NO: 15;
   (i) peroxidase DNA having a sequence of position 29 to 997 in SEQ ID NO: 17;
   (j) peroxidase DNA having a sequence of position 14 to 997 in SEQ ID NO: 19;
   (k) peroxidase DNA having a sequence of position 110 to 1090 in SEQ ID NO: 21;
   (l) peroxidase DNA having a sequence of position 53 to 1033 in SEQ ID NO: 23;
   (m) peroxidase DNA having a sequence of position 20 to 982 in SEQ ID NO: 25;
   (n) peroxidase DNA having a sequence of position 81 to 1025 in SEQ ID NO: 29;
   (o) peroxidase DNA having a sequence of position 44 to 1084 in SEQ ID NO: 31;
   (p) peroxidase DNA having a sequence of position 68 to 1114 in SEQ ID NO: 33;
   (q) peroxidase DNA having a sequence of position 31 to 1101 in SEQ ID NO: 35;
   (r) peroxidase DNA having a sequence of position 34 to 1089 in SEQ ID NO: 37;
   (s) peroxidase DNA having a sequence of position 52 to 1062 in SEQ ID NO: 39; and
   (t) a gene having a sequence which hybridizes to any one sequence of (a) to (s) under stringent conditions and encoding a peroxidase having the same expression specificity as that of a peroxidase encoded by said one sequence.
3. A peroxidase gene promoter, wherein the promoter is present upstream of a coding region of a peroxidase gene having a sequence selected from the group consisting of SEQ ID NOs: 1, 3, 5, 7, 9, 11, 13, 15, 17, 19, 21, 23, 25, 27, 29, 31, 33, 35, 37 and 39, or a peroxidase gene which hybridizes to said peroxidase gene under stringent conditions and has the same specific expression activity as that of said peroxidase gene.
4. A peroxidase gene promoter, wherein the promoter is present on an upstream side of a coding region of a peroxidase gene having a sequence selected from the group consisting of SEQ ID NOs: 1, 3, 5, 7, 9, 11, 13, 15, 17, 19, 21, 27, 29, 31, 33 and 35, or a peroxidase gene which hybridizes to said peroxidase gene under stringent conditions and has root-specific expression activity.
5. A peroxidase gene promoter, wherein the promoter is present on an upstream side of a coding region of a peroxidase gene having a sequence selected from the group consisting of SEQ ID NOs: 15, 17, 19 and 21, or a peroxidase gene which hybridizes to said peroxidase gene under stringent conditions and has expression activity in root and aerial parts.
6. A peroxidase gene promoter, wherein the promoter is present on an upstream side of a coding region of a peroxidase gene having a sequence selected from the group consisting of SEQ ID NOs: 23, 25, 37 and 39, or a peroxidase gene which hybridizes to said peroxidase gene under stringent conditions and has aerial-specific expression activity.
7. A peroxidase gene promoter, wherein the promoter is present on an upstream side of a coding region of a peroxidase gene having a sequence selected from the group consisting of SEQ ID NOs: 1, 3, 5, 7, 9, 13, 15, 17, 21, 23 and 25, or a peroxidase gene which hybridizes to said peroxidase gene under stringent conditions and has constitutive expression activity.
8. A peroxidase gene promoter, wherein the promoter is present on an upstream side of a coding region of a peroxidase gene having a sequence selected from the group consisting of SEQ ID NOs: 11 and 19, or a peroxidase gene which hybridizes to said peroxidase gene under stringent conditions and has stress-reducible expression activity.
9. A peroxidase gene promoter, wherein the promoter is present on an upstream side of a coding region of a peroxidase gene having a sequence selected from the group consisting of SEQ ID NOs: 27, 29, 31, 33, 35, 37 and 39, or a peroxidase gene which hybridizes to said peroxidase gene under stringent conditions and has stress-inducible expression activity.
10. A method for producing an expression cassette, comprising the steps of:
   (1) providing:
      (a) a peroxidase gene containing a sequence selected from the group consisting of SEQ ID NO: 1, 3, 5, 7, 9, 11, 13, 15, 17, 19, 21, 23, 25, 27, 29, 31, 33, 35, 37 and 39; and
      (b) a peroxidase gene which hybridizes to said peroxidase gene under stringent conditions and has the same specific expression activity as that of said peroxidase gene;
   (2) specifying a region having a promoter activity on an upstream side of a coding region of peroxidase gene (a) or (b); and
   (3) operatively linking the specified region having the promoter activity to a heterologous gene.
11. A method for analyzing a characteristic of a plant using a set of peroxidase genes according to para. 1, comprising the steps of:
   extracting RNA from a sample;
   binding the RNA to a membrane;
   labeling the set of peroxidase genes according to para. 1;
   incubating the membrane along with the set of the labeled peroxidase genes; and
   detecting signals derived from the labeled peroxidase genes.
12. A method for analyzing a characteristic of a plant using a peroxidase gene according to para. 2, comprising the steps of:
   extracting RNA from a sample;
   binding the RNA to a membrane;
   labeling the peroxidase gene according to para. 2;
   incubating the membrane along with the labeled peroxidase gene; and
   detecting signals derived from the labeled peroxidase gene.
13. A method according to para. 12, wherein the characteristic is response to rice blast fungus.
14. A method according to para. 12 or 13, wherein the gene is at least one gene selected from the group consisting of SEQ ID NOs: 29, 31, 33 and 37.
15. A method according to any one of para-s 11 to 14, wherein the sample is derived from a plant of the family rice.
16. A method for analyzing a characteristic of a plant using a sequence derived from a promoter according to any one of para.s 3 to 9, comprising the steps of:
   extracting RNA from a sample;
   binding the RNA to a membrane;
   labeling an oligonucleotide having the sequence derived from a promoter according to any one of para.s 3 to 9;
   incubating the membrane along with the labeled oligonucleotide; and
   detecting a signal derived from the labeled oligonucleotide.
17. A method for analyzing gene expression using a DNA microarray, comprising the steps of:
   (a) immobilizing a set of peroxidase genes according to para. 1 on the DNA microarray;
   (b) preparing at least two samples from a plant;
   (c) labeling the samples;
   (d) mixing and hybridizing the labeled samples to the DNA microarray; and
   (e) washing the hybridized DNA microarray and detecting a signal derived from the label.
18. A method for analyzing gene expression using a DNA microarray, comprising the steps of:
   (a) immobilizing a peroxidase gene according to para. 2 on the DNA microarray;
   (b) preparing at least two samples from a plant;
   (c) labeling the samples;
   (d) mixing and hybridizing the labeled samples to the DNA microarray; and
   (e) washing the hybridized DNA microarray and detecting a signal derived from the label.
19. A method for analyzing gene expression using a DNA microarray, comprising the steps of:
   (a) immobilizing an oligonucleotide having the sequence derived from the promoter according to any one of para.s 3 to 9 on the DNA microarray;
   (b) preparing at least two samples from a plant;
   (c) labeling the samples;
   (d) mixing and hybridizing the labeled samples to the DNA microarray; and
   (e) washing the hybridized DNA microarray and detecting a signal derived from the label.
20. A method according to any one of part. 17 to 19, further comprising the step of:
   (f) correcting the detected signal.
21. A method according to any one of para.s 17 to 20, further comprising the step of:
   (g) analyzing the detected signal or the corrected signal by an analysis software.

## Claims

1. A set of peroxidase genes useful for evaluation of a characteristic of plants, comprising at least one gene selected from the subsets B 1 and B2, wherein
(B1) is a subset of root-expression stress-inducible genes including at least one type of gene having the DNA sequence of SEQ ID NO: 29,
(B2) is a subset of aerial-expression stress-inducible genes including at least one type of gene having the DNA sequence of SEQ ID NO: 37.

2. A peroxidase gene, wherein the peroxidase gene has the DNA sequence of positions 81 to 1025 in SEQ ID NO: 29.
